# EUROPEAN PATENT SPECIFICATION

(11) **EP 3 769 088 B1**
(45) Date of publication and mention of the grant of the patent: **21.10.2026**
(21) Application number: 19718913.7
(22) Date of filing: 22.03.2019
(51) Int. Cl.: G01N 33/68

(54) **BIOMARKER COMBINATION FOR IDENTIFICATION OF "AT-RISK" SUBJECTS FOR ACUTE KIDNEY INJURY (AKI)**
BIOMARKER-KOMBINATION ZUR IDENTIFIZIERUNG VON "GEFÄHRDETEN" PROBANDEN FÜR AKUTE NIERENSCHÄDIGUNG
COMBINAISON DE BIOMARQUEURS POUR L'IDENTIFICATION DE SUJETS "À RISQUE" D'INSUFFISANCE RÉNALE AIGÜE

(30) Priority: 22.03.2018 GB 201804648
(43) Date of publication of application: 27.01.2021
(73) Proprietor: Randox Laboratories Ltd, Crumlin, Antrim BT29 4QY (GB); Belfast Health And Social Care Trust, Belfast BT9 7AB (GB)
(72) Inventor: KURTH, Mary Jo, Crumlin, Antrim BT29 4QY (GB); LAMONT, John, Crumlin, Antrim BT29 4QY (GB); FITZGERALD, Peter, Crumlin, Antrim BT29 4QY (GB); RUDDOCK, Mark, Crumlin, Antrim BT29 4QY (GB); MCBRIDE, William, Belfast, Antrim BT9 7AB (GB)
(74) Representative: Murgitroyd & Company
(86) International application number: PCT/GB2019/050835
(87) International publication number: WO 2019/180463

(56) References cited:
- WO-A1-2009/130505
- WO-A1-2010/054389
- WO-A1-2013/186564
- WO-A1-2016/041069
- HAYASHI H. ET AL.: "Efficacy of urinary midkine as a biomarker in patients with acute kidney injury", CLIN. EXP. NEPHROL., vol. 21, no. 4, 16 August 2016 (2016-08-16), pages 597 - 607, XP036373858
- OEZKUR M. ET AL.: "Preoperative serum h-FABP concentration is associated with postoperative incidence of acute kidney injury in patients undergoing cardiac surgery", BMC CARDIOVASC. DIS., vol. 14, no. 1, 117, 12 September 2014 (2014-09-12), pages 1 - 6, XP021196758
- AN J.N. ET AL.: "Circulating tumour necrosis factor receptors 1 and 2 predict contrast-induced nephropathy and progressive renal dysfunction: A prospective cohort study", NEPHROLOGY, vol. 20, no. 8, August 2015 (2015-08-01), pages 552 - 559, XP055529748
- VAIDYA V.S. ET AL.: "Urinary biomarkers for sensitive and specific detection of acute kidney injury in humans", CLIN. TRANSLATION. SCI., vol. 1, no. 3, December 2008 (2008-12-01), pages 200 - 208, XP009165229

## Description

The present invention relates to a method for determining predisposition of a subject to developing renal dysfunction, and to a kit for use in making such a determination. Acute Kidney Injury (AKI), is a recognised complication of cardiac surgery (CS) with cardiopulmonary bypass (CPB) and is characterised by an abrupt deterioration of estimated glomular filtration rate (eGFR). AKI-CS is associated with increased hospital stay, morbidity and mortality. Identification and diagnosis of AKI-CS is based on changes in serum creatinine concentration. However, this is problematic as increases in serum creatinine may be delayed by 24-72 hours post insult. Early prediction and identification of 'at-risk' patients would allow implementation of suitable preoperative, intraoperative and postoperative renal protection strategies. The benefits of early Renal Replacement Therapy (RRT), compared to late RRT, are associated with improved patient survival and decreased length of ICU stay (RRT <3 days' post-surgery).

Pre and intra-operative renal protection strategies, such as administration of erythropoietin or minimising the duration of CPB may be provided to those subjects identified pre-surgery to be at risk of AKI. Post-surgery detection of those subjects at risk from AKI, suitably within 24 hours from surgery, would allow early post-operative interventions such as Renal Replacement Therapy.

Further, subjects suffering physical trauma, which can include fractures, often develop acute renal dysfunction (AKI). Other causes of a similar acute renal dysfunction include prolonged hypotensive states (e.g. associated with mucosal gut ischaemia and endotoxin translocation from gut to circulation), sepsis and septic shock syndromes.

A robust post physical trauma test, suitably within 24 hours of the trauma, would allow preventative measures to be taken in the intensive care unit with subjects considered to be 'at risk' of AKI

WO 2010/054389 A1 relates to assays that detect one or more markers selected from a group including midkine, heart-type fatty acid binding protein (H-FABP), IFN-γ, IL12p40 for evaluating renal status, namely "acute kidney injury" or "AKl." Said markers may be measured in combination with further markers including MIP2-a, NGAL, MCP-1, sTNFR-I, sTNFR-II. The sample may be urine, serum or plasma. The method may be used to determine the treatment of a patient in need. Cardiac surgery patients are also mentioned.

Hayashi H. et al., Efficacy of urinary midkine as a biomarker in patients with acute kidney injury. Clin. Exp. Nephrol., 2016, 21(4) : 597-607 demonstrates the efficacy of urinary midkine (MK) as a biomarker in patients with acute kidney injury.

WO 2016/041069 A1 discloses a method for determining the risk of AKI in a mammal following cardiac surgery that comprises determining the sample value of IL-6 or hFABP concentration or combinations thereof, including combinations of hFABP, IL-6 and IL-6, IL-10.

### Summary of the invention

Whilst several markers have been identified for identification of subjects 'at risk' of developing Acute Kidney Injury (AKI) improved detection of such subjects would be beneficial.

The inventors have determined that measurement of specific combinations of biomarkers present in a subject pre and post trauma can provide for improved detection of subjects 'at risk' of developing AKI.

Accordingly, a first aspect of the present invention provides a method to determine a predisposition of a subject to developing AKI, the method comprising the step of:
determining the level of at least one marker selected from Midkine (MK) or H-FABP present in a blood or urine sample taken from the subject prior to cardiac surgery or a fracture trauma;
wherein when the level of the Midkine or H-FABP is higher than a normal level of Midkine or H-FABP in a blood or urine sample from a control it is indicative the subject has a greater than normal predisposition for developing AKI following cardiac surgery or a fracture trauma;
and further comprising, determining the level of at least a second marker selected from at least one of TNFRI and TNFRII wherein when the detected level of TNFRI and/ or TNFRII is higher than a normal level of TNFRI and/ or TNFRII respectively in a control it is indicative the subject has a greater than normal predisposition for developing AKI following cardiac surgery or fracture trauma; and, further wherein the determining step comprises determining the level of at least three markers selected from: TNFRI, H-FABP and Midkine; or TNFRI, TNFRII and Midkine.

According to a second aspect of the present invention there is provided a method to determine a predisposition of a subject to developing AKI, the method comprising the step of:
determining the level of at least one marker selected from Midkine (MK) or H-FABP present in a blood or urine sample taken from the subject within 48 hours following surgery or a fracture trauma;
wherein when the level of the Midkine or H-FABP is higher than a normal level of Midkine or H-FABP in a blood or urine sample from a control it is indicative the subject has a greater than normal predisposition for developing AKI following cardiac surgery or a fracture trauma;
and further comprising, determining the level of at least a second marker selected from at least one of TNFRI and TNFRII wherein when the detected level of TNFRI and/ or TNFRII is higher than a normal level of TNFRI and/ or TNFRII respectively in a control it is indicative the subject has a greater than normal predisposition for developing AKI following cardiac surgery or fracture trauma; and, further wherein the determining step comprises determining the level of at least three markers selected from: TNFRI, H-FABP and Midkine; or TNFRI, TNFRII and Midkine.

The invention is defined by the appended claims.

As used herein, marker and biomarker are used interchangeably.

As will be appreciated, the level(s) of biomarker(s) from a subject being tested may be compared with the level(s) of biomarker(s) in a sample from a subject with an AKI disease state and when the level(s) of the biomarker(s) in the sample from the subject being tested is not differential to the level(s) of biomarker(s) in the sample from the subject with an AKI disease state, it is indicative that the subject being tested has AKI.

Alternatively, as will be appreciated, the level(s) of biomarker(s) from a subject being tested may be compared with the level(s) of biomarker(s) in a sample from a subject with a non AKI disease state and when the level(s) of the biomarker(s) in the sample from the subject being tested is not differential to the level(s) of biomarker(s) in the sample from the subject with a non AKI disease state, it is indicative that the subject being tested does not have AKI.

"Biomarker" means a biological compound, that is differentially present (i.e. increased or decreased) in a biological sample from a subject or a group of subjects having a first phenotype (e.g. having a disease) as compared to a biological sample from a subject or group of subjects having a second phenotype (e.g. not having the disease). A biomarker may be differentially present at any level, but suitably differentially present at a level that is statistically significant (i.e, a p-value less than 0.05 and/or a q-value of less than 0.10 as determined using either Welch's T-test or Wilcoxon's rank-sum Test). Suitably the biomarker may be present at a level that has an AUC (area under the curve of a receiving operating characteristic curve) of 0.7 or greater, suitably 0.75 or greater, suitably 0.8 or greater. The "level" of one or more biomarkers means the absolute or relative amount or concentration of the biomarker in the sample.

"Sample" or "biological sample" means biological material isolated from a subject. The biological sample may contain any biological material suitable for detecting the desired biomarkers, and may comprise cellular and/or non-cellular material from the subject. The sample can be isolated from any suitable biological fluid such as, for example, blood, blood plasma, blood serum, or urine.

A "reference level or normal level" of a biomarker means a level of the biomarker that is indicative of a non AKI state, phenotype, or predisposition to developing an AKI disease state or phenotype. A "level" of a biomarker may be an absolute or relative amount or concentration of the biomarker, a presence or absence of the biomarker, a range of amount or concentration of the biomarker, a minimum and/or maximum amount or concentration of the biomarker, a mean amount or concentration of the biomarker, and/or a median amount or concentration of the biomarker; and, in addition, "reference levels" of combinations of biomarkers may also be ratios of absolute or relative amounts or concentrations of two or more biomarkers with respect to each other. Appropriate normal reference levels of biomarkers for AKI may be determined by measuring levels of desired biomarkers in one or more appropriate subjects, and such reference levels may be tailored to specific populations of subjects (e.g., a reference level may be age-matched or gender-matched so that comparisons may be made between biomarker levels in samples from subjects of a certain age or gender and reference levels for a AKI disease state, phenotype, or lack thereof in a certain age or gender group).

When the methods of the invention are used to aid in the diagnosis of AKI, the results of the method may be used along with other methods (or the results thereof) useful in the clinical determination of whether a subject has AKI.

Any suitable method may be used to analyze the biological sample in order to determine the level(s) of the one or more biomarkers in the sample. Suitable methods include chromatography (e.g., HPLC, gas chromatography, liquid chromatography), mass spectrometry (e.g., MS, MS-MS), enzyme-linked immunosorbent assay (ELISA), antibody linkage, other immunochemical techniques, and combinations thereof. Further, the level(s) of the one or more biomarkers may be measured indirectly, for example, by using an assay that measures the level of a compound (or compounds) that correlates with the level of the biomarker(s) that are desired to be measured.

It will be appreciated that the markers as discussed herein may be detected using any suitable assays or devices, for example ELISA assay, BIOCHIP technology or the like. Suitably, TNFRI and II biomarkers (Soluble Tumour Necrosis Factor Receptors I and II) may be detected using a Cytokine Array IV (Randox).

Suitably, determining levels of combinations of the biomarkers may allow greater sensitivity and specificity in diagnosing AKI and aiding in the diagnosis of AKI. For example, ratios of the levels of certain biomarkers (and non-biomarker compounds, for example metabolites) in biological samples may allow greater sensitivity and specificity in diagnosing AKI and aiding in the diagnosis of AKI. After the level(s) of the one or more biomarkers in the sample are determined, the level(s) can be compared to AKI-positive and/or AKI-negative reference levels to diagnose or aid in diagnosing whether the subject has AKI. Levels of the one or more biomarkers in a sample matching the AKl-positive reference levels (e.g., levels that are the same as the reference levels, substantially the same as the reference levels, above and/or below the minimum and/or maximum of the reference levels, and/or within the range of the reference levels) are indicative of a diagnosis of AKI in the subject. Levels of the one or more biomarkers in a sample matching the AKI-negative reference levels (e.g., levels that are the same as the reference levels, substantially the same as the reference levels, above and/or below the minimum and/or maximum of the reference levels, and/or within the range of the reference levels) are indicative of a diagnosis of no AKI in the subject. In addition, levels of the one or more biomarkers that are differentially present (especially at a level that is statistically significant) in the sample as compared to AKI-negative reference levels are indicative of a diagnosis of AKI in the subject. Levels of the one or more biomarkers that are differentially present (especially at a level that is statistically significant) in the sample as compared AKI-positive reference levels are indicative of a diagnosis of no AKI in the subject.

The level(s) of the one or more biomarkers may be compared to AKI-positive and/or AKl-negative reference levels using various techniques, including a simple comparison (e.g., a manual comparison) of the level(s) of the one or more biomarkers in the biological sample to AKl-positive and/or AKI-negative reference levels. The level(s) of the one or more biomarkers in the biological sample may also be compared to AKI-positive and/or AKI-negative reference levels using one or more statistical analyses (e.g., t-test, Welch's T-test, Wilcoxon's rank sum test, Random Forest, T-score, Z-score) or using a mathematical model (e.g., algorithm, statistical model, mixed effects model).

Suitably Midkine (MK or NEGF-2 - a 13 kDa heparin-binding embryonic growth factor may be detected using an ELISA assay as available from Cellmid (Australia). Midkine is a known marker in relation to cancer (see for example: Muramatsu, T., Midkine and pleiotrophin: two related proteins involved in development, survival, inflammation and tumorigenesis. J Biochem, 2002. 132(3): p.359-71;. Ikematsu, S., et al., Serum midkine levels are increased in patients with various types of carcinomas. Br J Cancer, 2000. 83(6): p. 701-6; Ibusuki, M., et al., Midkine in plasma as a novel breast cancer marker. Cancer Sci,2009. 100(9): p. 1735-9; Ikematsu, S., et al., Correlation of elevated level of blood midkine with poor prognostic factors of human neuroblastomas. Br J Cancer, 2003. 88(10): p. 1522-6.)

The inventors have determined that detecting the markers TNFRI, TNFRII and Midkine in a pre-operative serum sample from a subject in a model for AKI, an area under the curve of a receiving operating characteristic curve for a sample pre-surgery from a subject to undergo cardiac surgery with cardiopulmonary bypass is of about 0.75. Further, the inventors have determined that using the markers TNFRI, TNFRII and Midkine in a post-operative serum sample from a subject, an area under the curve of a receiving operating characteristic curve for a sample post-surgery from a subject who has undergone cardiac surgery with cardiopulmonary bypass is of about 0.80.

Suitably the inventors have determined that using the markers TNFRI and TNFRII in a pre-operative serum sample from a subject, an area under the curve of a receiving operating characteristic curve for a sample pre-surgery from a subject to undergo cardiac surgery with cardiopulmonary bypass is of about 0.73.

Suitably the inventors have determined that using the markers TNFRI and Midkine in a pre-operative serum sample from a subject, an area under the curve of a receiving operating characteristic curve for a sample pre-surgery from a subject to undergo cardiac surgery with cardiopulmonary bypass is of about 0.74.

Suitably the inventors have determined that using the markers TNFRII and Midkine in a pre-operative serum sample from a subject, an area under the curve of a receiving operating characteristic curve for a sample pre-surgery from a subject to undergo cardiac surgery with cardiopulmonary bypass is of about 0.75.

Further, the inventors have determined that using the markers TNFRI, TNFRII and Midkine in a post-operative serum sample from a subject, an area under the curve of a receiving operating characteristic curve for a sample post-surgery from a subject who has undergone cardiac surgery with cardiopulmonary bypass is of about 0.80.

Suitably the inventors have determined that using the markers TNFRI and TNFRII in a post-operative serum sample from a subject, an area under the curve of a receiving operating characteristic curve for a sample post-surgery from a subject who has undergone cardiac surgery with cardiopulmonary bypass is of about 0.77.

Suitably the inventors have determined that using the markers TNFRI and Midkine in a post-operative serum sample from a subject, an area under the curve of a receiving operating characteristic curve for a sample post-surgery from a subject who has undergone cardiac surgery with cardiopulmonary bypass is of about 0.76.

Suitably the inventors have determined that using the markers TNFRII and Midkine in a post-operative serum sample from a subject, an area under the curve of a receiving operating characteristic curve for a sample post-surgery from a subject who has undergone cardiac surgery with cardiopulmonary bypass is of about 0.80.

Suitably the inventors have determined that using the markers TNFRI and H-FABP in a pre-operative serum sample from a subject, an area under the curve of a receiving operating characteristic curve for a sample pre-surgery from a subject to undergo a fracture trauma is of about 0.74.

Suitably the inventors have determined that using the markers TNFRI and H-FABP in a post-operative serum sample from a subject, an area under the curve of a receiving operating characteristic curve for a sample post-surgery from a subject who has undergone fracture trauma is of about 0.87.

Suitably the inventors have determined that using the markers TNFRI and H-FABP and Midkine in a pre-operative serum sample from a subject, an area under the curve of a receiving operating characteristic curve for a sample pre-surgery from a subject to undergo a fracture trauma is of about 0.76.

Suitably the inventors have determined that using the markers TNFRI and H-FABP and Midkine in a post-operative serum sample from a subject, an area under the curve of a receiving operating characteristic curve for a sample post-surgery from a subject who has undergone fracture trauma is of about 0.88.

After the level(s) of the one or more biomarker(s) is determined, the level(s) may be compared to AKI disease or non AKI disease reference level(s) or reference curves of the one or more biomarker(s) to determine a rating for each of the one or more biomarker(s) in the sample. The rating(s) may be aggregated using any algorithm to create a score, for example, an AKI score, for the subject. The algorithm may take into account any factors relating to AKI including the number of biomarkers, the correlation of the biomarkers to AKI, etc. Suitably, a mathematical model or formula containing one or more biomarkers as variables may be established using regression analysis, e.g., multiple linear regressions. By way of non-limiting example, the developed formulas may include the following: $A + B \left({Biomarker}_{1}\right) + C \left({Biomarker}_{2}\right) + D \left({Biomarker}_{3}\right) + E \left({Biomarker}_{4}\right) = PatientScore$ 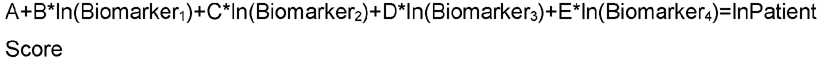 A+B*In(Biomarker1)+C*In(Biomarker2)+D*In(Biomarker3)+E*In(Biomarker4)=InPatient Score wherein A, B, C, D, E can be constant numbers; Biomarker₁, Biomarker₂, Biomarker₃, Biomarker₄ are the measured values of a respective Biomarker and PatientScore is the measure of AKI presence or absence or severity.

Suitably a pre-operative patient score can be provided which is indicative the subject has a greater than normal predisposition for developing AKI following cardiac surgery with cardiopulmonary bypass.

Suitably a cardiac patient score may be provided by, for example, $7 \left({log}_{10} TNFRII+1\right) + 2 \left({log}_{10}TNFRI+1\right) + 1 / 3 \left({log}_{10}MK+1\right)$

In an embodiment when a patient score is greater than or equal to 2.6 it is indicative the subject is at risk of AKI. In an embodiment when a patient score is greater than or equal to 2.7 and less than 3.4 it is indicative the subject is at risk of AKI. In an embodiment when a patient score is greater than or equal to 2.75 and less than 3.3 it is indicative the subject is at risk of AKI. In an embodiment when a patient score is greater than or equal to 2.9 and less than 3.1 it is indicative the subject is at risk of AKI.

Suitably, in relation to fracture repair, the subjects undergo two insults. The initial insult and the surgery to repair the fracture. The time delay between the injury occurring and the surgery, results in subjects becoming conditioned i.e. the inflammatory process has started before surgery. As a result, the subjects may develop AKI before surgery. The classification of AKI was based on the RIFLE system. Patients were assumed to have a baseline GFR of at least 60ml/min/1.73m² therefore, a value of less than 45ml/min/1.73 m² was used to define a patient as AKI positive.

Suitably a pre-fracture repair patient score may be provided by, for example, $1.579 \left({log}_{10}\left(H-FABP+1\right)\right) + 8.555 \left({log}_{10}TNFRI+1\right)$

In an embodiment when a patient score is greater than or equal to 4 it is indicative the subject is at risk of AKI. In an embodiment when a patient score is greater than or equal to 4.1 and less than 5 it is indicative the subject is at risk of AKI. In an embodiment when a patient score is greater than or equal to 4.2 and less than 4.5 it is indicative the subject is at risk of AKI. In an embodiment when a patient score is greater than or equal to 4.3 and less than 4.5 it is indicative the subject is at risk of AKI.

Suitably, samples for analysis are taken prior to surgery and within 24 hours following surgery. Subjects with a MDRD <45ml/min/1.73m² at any time were defined as AKI positive.

In embodiments of the second aspect of the present invention a sample may be taken from the subject within 24 hours following surgery, physical trauma, hypotension, sepsis and/or septic shock syndrome, in particular cardiac surgery or a fracture trauma wherein when the level of TNFRI, TNFRII and Midkine is higher than a normal level of TNFRI, TNFRII and Midkine in a sample it is indicative the subject has a greater than normal predisposition for developing AKI following cardiac surgery with cardiopulmonary bypass.

The inventors have determined that using the markers TNFRI, TNFRII and Midkine in a post-operative sample from a subject, a patient score can be provided which is indicative the subject has a greater than normal predisposition for developing AKI following cardiac surgery with cardiopulmonary bypass.

Suitably a post operative cardiac patient score may be provided by, for example, $7.3 \left({log}_{10} TNFRII\right) + 2 \left({log}_{10}TNFRI\right) + 6 / 5 \left({log}_{10}MK\right)$

In an embodiment when a patient score is greater than or equal to 6.8 it is indicative the subject is at risk of AKI. In an embodiment when a patient score is greater than or equal to 6.9 and less than 7.6 it is indicative the subject is at risk of AKI. In an embodiment when a patient score is greater than or equal to 6.9 and less than 7.5 it is indicative the subject is at risk of AKI. In an embodiment when a patient score is greater than or equal to 7.0 and less than 7.4 it is indicative the subject is at risk of AKI.

The inventors have determined that using the markers TNFRI and H-FABP in a post-operative sample from a subject, a patient score can be provided which is indicative the subject has a greater than normal predisposition for developing AKI following cardiac surgery with cardiopulmonary bypass.

Suitably a post-fracture patient score may be provided by, for example, $4.81 \left({log}_{10}\left(H-FABP+1\right)\right) + 9.7 \left({log}_{10}TNFRI+1\right)$

In an embodiment when a patient score is greater than or equal to 10.5 it is indicative the subject is at risk of AKI. In an embodiment when a patient score is greater than or equal to 10.7 and less than 14 it is indicative the subject is at risk of AKI. In an embodiment when a patient score is greater than or equal to 10.8 and less than 14 it is indicative the subject is at risk of AKI. In an embodiment when a patient score is greater than or equal to 11 and less than 14 it is indicative the subject is at risk of AKI.

In an embodiment the patient score is determined from a sample obtained from a subject within 24 hours of a surgery. In an embodiment the patient score is determined from a sample obtained from a subject within 48 hours of a surgery. In an embodiment the patient score is determined from a sample obtained from a subject within 72 hours of a surgery. In an embodiment the patient score is determined from a sample obtained from a subject within 120 hours of a surgery. Suitably a sample from a subject may be obtained within 24 to 48 hours post surgery, suitably within 48 hours to 72 hours post surgery, suitably within 72 to 120 hours post surgery.

In embodiments the sample for use in the first or second aspect can be from blood. Suitably the sample may be plasma or serum. In embodiments the sample can be urine.

Suitably, the method may allow determination of a predisposition of renal dysfunction wherein the renal dysfunction is acute renal dysfunction. Suitably, the renal dysfunction may present 5-days or more following surgery, physical trauma, hypotension, sepsis and/or septic shock syndrome, in particular cardiac surgery or a fracture trauma

Suitably, renal dysfunction may be defined as a 25% or more decrease from normal glomerular filtration rate (eGFR less than 75%).

The method of the present invention predicts the likelihood of the subject developing post-event (i.e. post surgery, physical trauma, hypotension, sepsis and/or septic shock syndrome, in particular cardiac surgery or a fracture trauma) renal dysfunction, should those events occur. The method is therefore a prognostic method. Subjects may be determined to have a greater than normal chance of developing renal dysfunction when they present with a patient score derived from a combination of TNFRI, TNFRII and MK in a serum sample greater than a normal patient score derived from such a combination.

A 'normal or control level' (non AKI) patient score is the level presented by a control group of individuals who did not develop AKI.

The renal dysfunction to be prognosed may be early renal dysfunction, late renal dysfunction, or general renal dysfunction. Early renal dysfunction occurs within two days of the event that induces the renal dysfunction. Late renal dysfunction occurs 5 days or later after such an event. Determining whether or not an individual has renal dysfunction is a clinical question well within the abilities of the skilled person. However, in the interests of clarity, renal dysfunction is characterised by a reduction in the capacity to excrete metabolic products which accumulate systemically and are detectable clinicopathologically by renal function tests (in progressed states, renal dysfunction may be acute kidney failure, uremia or chronic renal damage). For example, renal dysfunction may be defined as a 25% or more decrease from normal glomerular filtration rate. Normal glomerular filtration rate is the pre-event rate. Glomerular filtration rate may be established in accordance with the MDRD study group formula. Such acute forms of renal dysfunction can be distinguished from autoimmune mediated chronic renal dysfunction, a condition that is clinically apparent over a prolonged period of time in parallel with the coexisting autoimmune condition (i.e. there is no requirement for a biological marker to predict the development of renal dysfunction occurring a few days later because the renal dysfunction is already well established).

The sample taken from the subject may be any sample capable of being analysed for the level of anti-inflammatory cytokine therein. For example, the sample may be a urine sample, a blood sample, for example a serum or a plasma sample. A serum sample is particularly preferred.

The samples analysed according to the first aspect of the invention may be obtained from the subject 48, 24 or 12 hours before the event (i.e. cardiac surgery). The sample will optimally be obtained 24 hours before the event.

In embodiment of the first or second aspect the step of determining the level of at least one marker selected from Midkine or H-FABP can be undertaken on serum from the subject.

In embodiments TNFRI and / or TNFRII can be detected in a urine sample and Midkine and / or H-FABP can be detected in serum. Suitably a ratio of TNFRI and / or TNFRII and Midkine and / or H-FABP may be determined.

In embodiments the determining step can comprise determining the level of at least three markers selected from TNFRI, TNFRII and Midkine.

In embodiments the determining step can comprise determining the level of at least three markers selected from TNFRI, Midkine and H-FABP.

Suitably the methods may include detection of an additional marker selected from at least one of IL-1a, IL-5, IL-6, IL-8, IL-10, IL-15, VEGF, INF-gamma, TNF-alpha, MCP, MIP1-alpha, NGAL, IL12P40, IP10 or IL1Ra. Suitably, at least one of IL-1a, IL-5, IL-6, IL-8, IL-10, IL-15, VEGF, INF-gamma, TNF-alpha, MCP, MIP1-alpha and NGAL may be detected from plasma from the subject. Suitably, IL12P40 may be detected from serum or urine of a subject. Suitably, IP10 or IL1Ra may be detected in serum or urine. Suitably NGAL may be detected in urine.

Suitably when considering a subject who will undergo cardiac surgery the following markers may be utilized in a diagnostic model IL-6, IL-1a, VEGF, INF-gamma, TNF-alpha, MCP, MIP1-alpha and NGAL.

Suitably when considering a subject who will or has undergone cardiac surgery a post surgery model may include, TNF-alpha, MCP, MIP1-alpha and NGAL.

The individual markers may be measured in samples obtained at the same time, or they may be determined from samples obtained at different times (e.g., an earlier or later time). The individual markers may also be measured in the same or different body fluid samples. For example, one marker may be measured in a serum or plasma sample and another marker may be measured in a urine sample. In addition to absolute levels, ratios of markers may be useful in identifying subjects at risk of AKI within the scope of the current invention. For example, the ratio of one marker measured in serum to another marker measured in urine, at the same or different time points, may be informative to the prognosis of AKI following cardiac or orthopedic surgery. Suitable ratios would include, for example, H-FABP to TNFRI, Midkine to TNFRI, H-FABP to Midkine, H-FABP to TNFRII, Midkine to TNFRII and TNFRI to TNFRII.

The present disclosure describes a kit for use in the method of the first or second aspect, wherein the kit comprises:-
- one or more reagents to detect at least one of TNFI, TNFII, H-FABP and Midkine or a combination thereof
- instructions for determining whether the at least one of TNFRI, TNFRII, H-FABP and Midkine is higher than a normal level as observed in a subject according to the first or second aspect.

The kit may further comprise one or more reagents for the detection of one or more anti-inflammatory mediator, for example an anti-inflammatory cytokine, and instructions for using the one or more reagents for detecting the one or more anti - inflammatory mediator.

The kit may further comprise instructions for using the detection of the one or more anti-inflammatory cytokines in order to arrive at a prognosis for renal dysfunction. The instructions may be in accordance with the steps for prognosis of renal dysfunction provided in the first or second aspect. The kit may further comprise instructions for using the detecting of the one or more anti-inflammatory mediator in order to arrive at a prognosis for renal dysfunction.

The present disclosure describes a method of treating renal dysfunction induced by cardiac surgery or a fracture trauma, wherein the method includes the steps of: (i) prognosing renal dysfunction according to any of the methods of the first aspect or second aspect and (ii) when the subject is identified to be at increased risk of developing renal dysfunction, applying therapeutic measures to treat or obviate the impending renal dysfunction.

The advantage of such a method over current therapeutic interventions is that therapy may be administered at a stage when full renal failure may be prevented. The therapeutic measures applied in step (ii) may be: maintaining a supra-normal blood pressure; ensuring adequate tissue oxygen delivery; administration of steroids; renal replacement therapy; dialysis; or any combination thereof, administration of erythropoietin, minimizing the duration of cardiopulmonary bypass, early renal replacement therapy. A further advantage of such a method would be to allow intensive care managers to identify early in the intensive care stay of the patient those individuals who are likely to spend longer in intensive care than would otherwise be anticipated providing earlier planning for staff deployment. The biomarker(s) and algorithm(s) discussed herein may assist a physician to decide a treatment path.

A physical trauma can be the impact on the body from external forces applied to the body, for example:- lesions caused by surgery or by blows or cuts to the body (such as might be experienced during a car crash), or; the impact on the blood as it interacts with the foreign surface of a heart-lung bypass machine. Renal dysfunction induced by physical trauma may be postoperative renal dysfunction. The post-operative renal dysfunction may be following cardiac, cardiovascular, cardiopulmonary or bone fracture surgery. Physical trauma does not include reperfusion injury.

Whether or not an individual has hypotension is a clinical question and therefore well within the skill of an ordinary person in the art. For the avoidance of doubt however hypotension in adults may be defined as a systolic blood pressure < 80 mmHg, or a mean arterial pressure (MAP) < 50 mmHg. The hypotension may be prolonged, for example for over 2 hours.

Whether or not an individual has sepsis is a clinical question and therefore well within the skill of an ordinary person in the art. For the avoidance of doubt however, sepsis may be considered present if infection is highly suspected or proven and two or more of the following systemic inflammatory response syndrome (SIRS) criteria are met:
1. Heart rate > 90 beats per minute (tachycardia);
2. Body temperature < 36°C (97°F) or > 38 °C (100°F) (hypothermia or fever);
3. Respiratory rate > 20 breaths per minute or, on blood gas, a PₐCO₂ less than 32 mm Hg (4.3 kPa) (tachypnea or hypocapnia due to hyperventilation); and
4. White blood cell count < 4,000 cells/mm³ or > 12,000 cells/mm³ (< 4 x 10⁹ or > 12 x 10⁹ cells/L), or greater than 10% band forms.

Whether or not an individual has septic shock is a clinical question and therefore well within the abilities of an ordinary person skilled in the art. For the avoidance of doubt however, septic shock may be defined by the presence of the following two criteria:
1. Evidence of infection, through a positive blood culture; and
2. Refractory hypotension - hypotension despite adequate fluid resuscitation and cardiac output. In adults it is defined as a systolic blood pressure < 90 mmHg, or a MAP < 60 mmHg, before institution of required resuscitative inotropic support, or a reduction of 40 mmHg in the systolic blood pressure from baseline. In children it is BP < 2 SD of the normal blood pressure.

Whether or not an individual has SIRS is a clinical question and therefore well within the abilities of an ordinary person skilled in the art. For the avoidance of doubt however SIRS may be diagnosed when two or more of the following are present:
1. Heart rate > 90 beats per minute
2. Body temperature < 36 or > 38°C
3. Tachypnea (high respiratory rate) > 20 breaths per minute or, on blood gas, a PₐCO₂ < 4.3 kPa (32 mm Hg)
4. White blood cell count < 4000 cells/mm³ or > 12000 cells/mm³ (< 4 x 10⁹ or > 12 x 10⁹ cells/L), or the presence of greater than 10% immature neutrophils.

Preferred features and embodiments of each aspect of the invention are as for each of the other aspects mutatis mutandis unless context demands otherwise.

As used herein, the articles "a" and "an" refer to one or to more than one (for example to at least one) of the grammatical object of the article.

"About" shall generally mean an acceptable degree of error for the quantity measured given the nature or precision of the measurements.

Throughout the specification, unless the context demands otherwise, the terms 'comprise' or 'include', or variations such as 'comprises' or 'comprising', 'includes' or 'including' will be understood to imply the includes of a stated integer or group of integers, but not the exclusion of any other integer or group of integers.

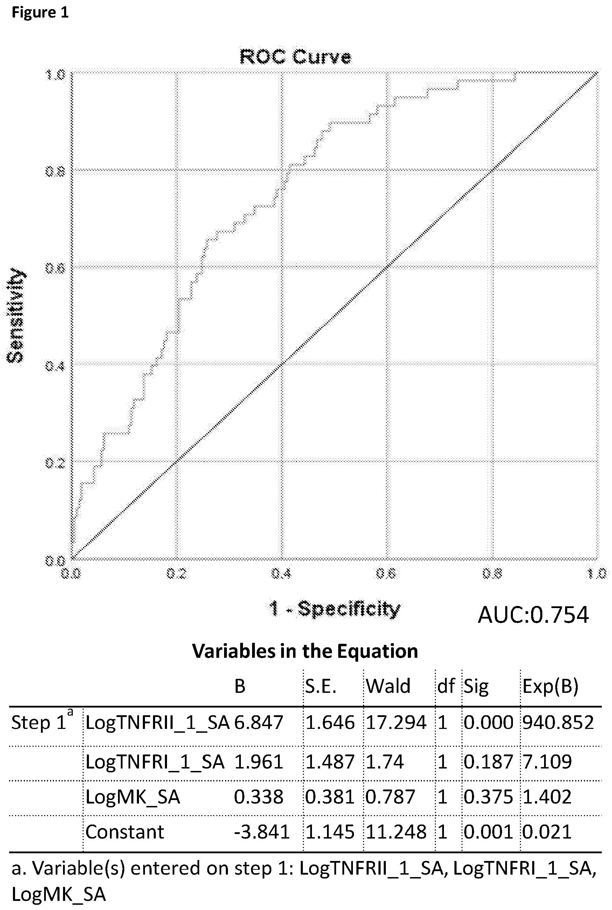

Embodiments of the present invention will now be described, by way of example, with reference to the accompanying figures, in which:-
Figure 1 shows pre-surgery ROC of TNFRI, TNFRII and Midkine model;

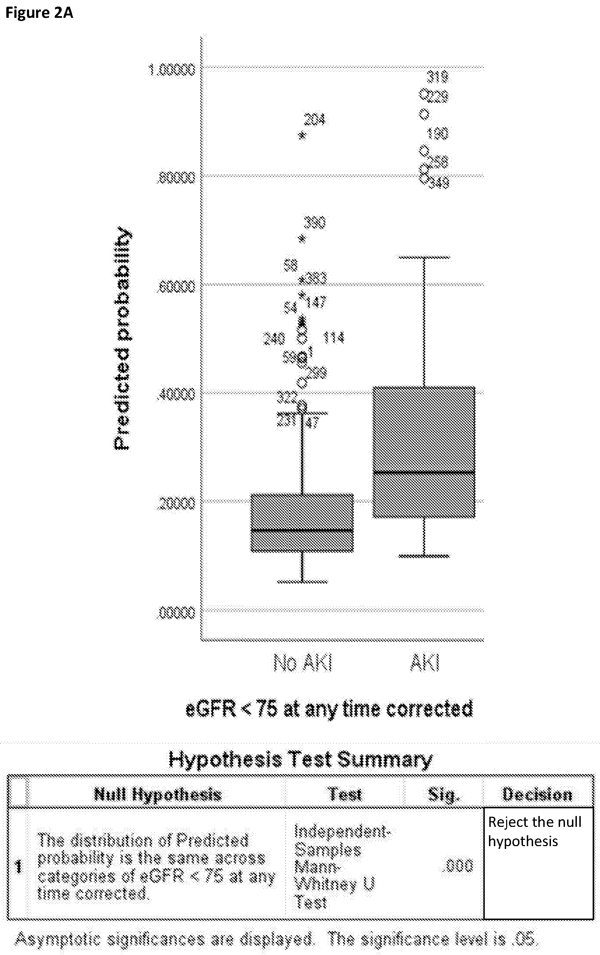

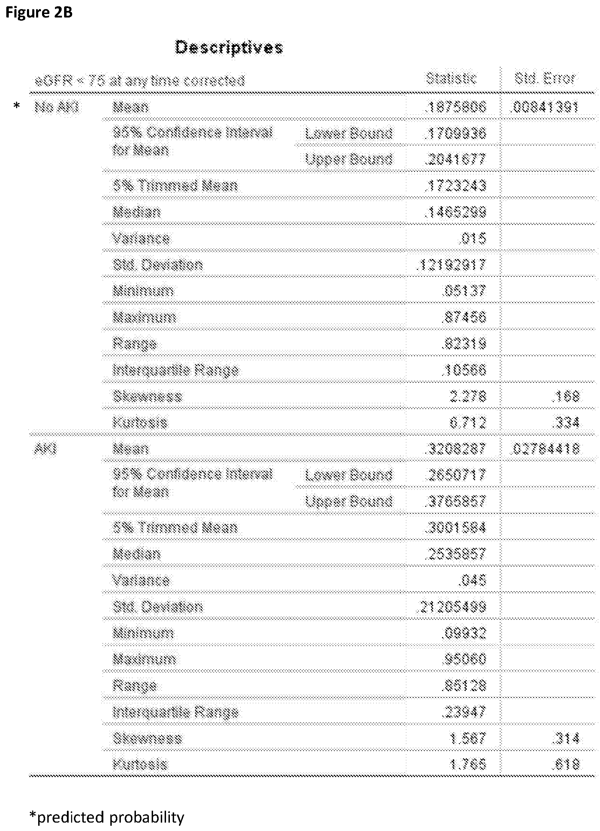

Figure 2 shows a pre-surgery TNFRI, TNFRII and Midkine model;

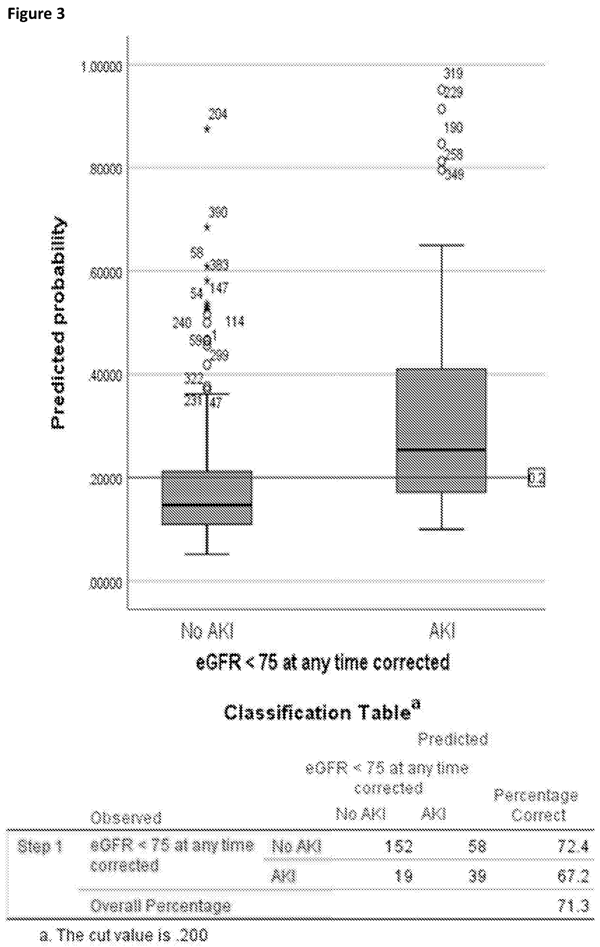

Figure 3 shows a pre-surgery TNFRI, TNFRII and Midkine model;

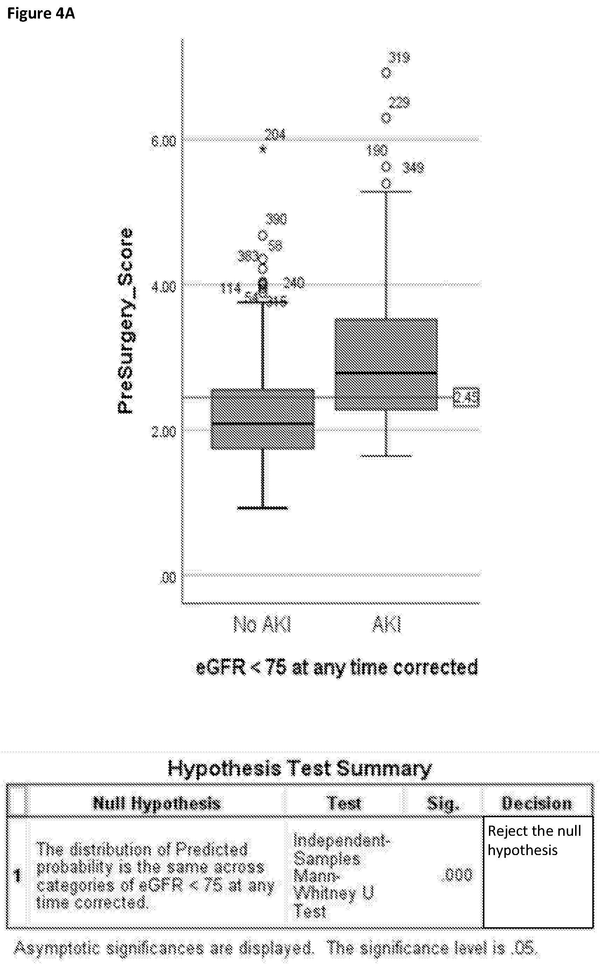

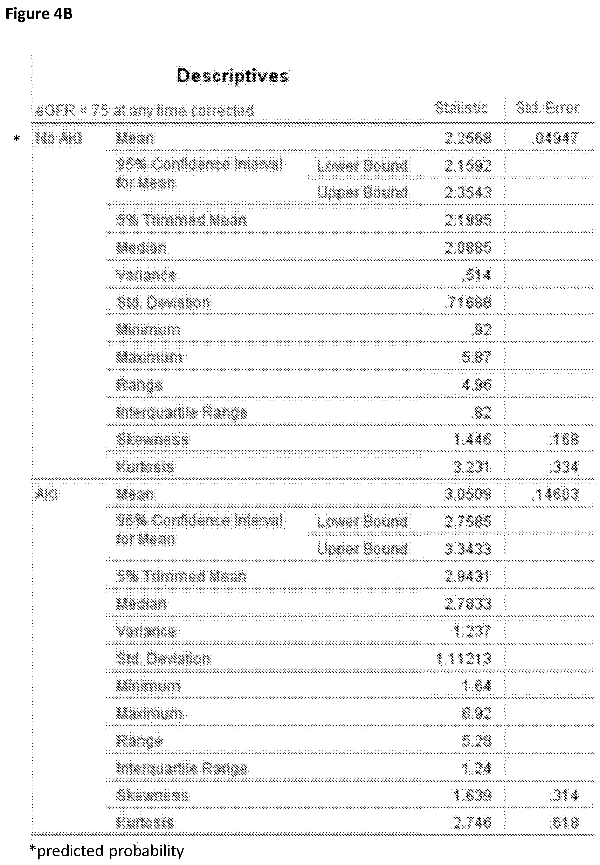

Figure 4 shows a patient scores using Serum Pre-surgery TNFRI, TNFRII and Midkine model;

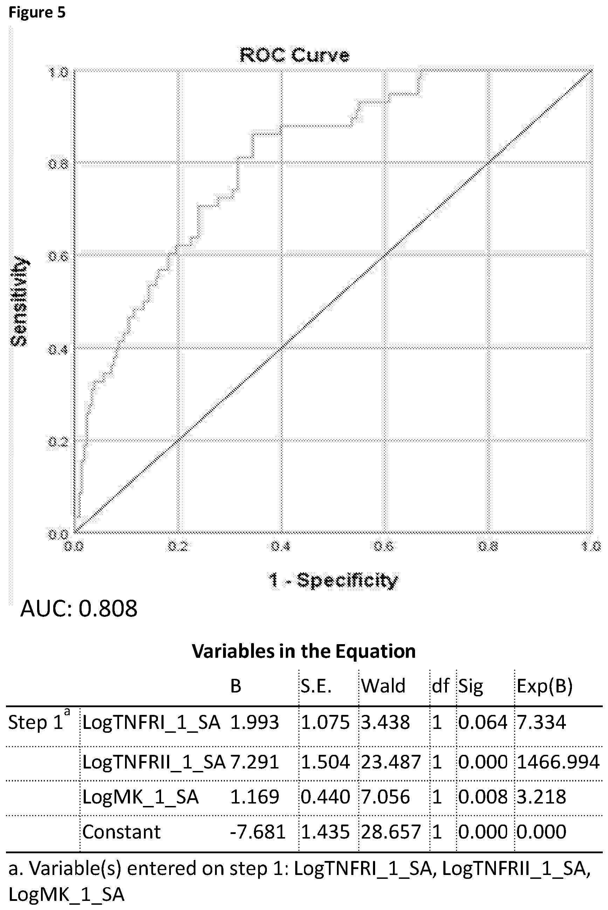

Figure 5 shows ROC for a serum post surgery TNFRI, TNFRII and Midkine model;

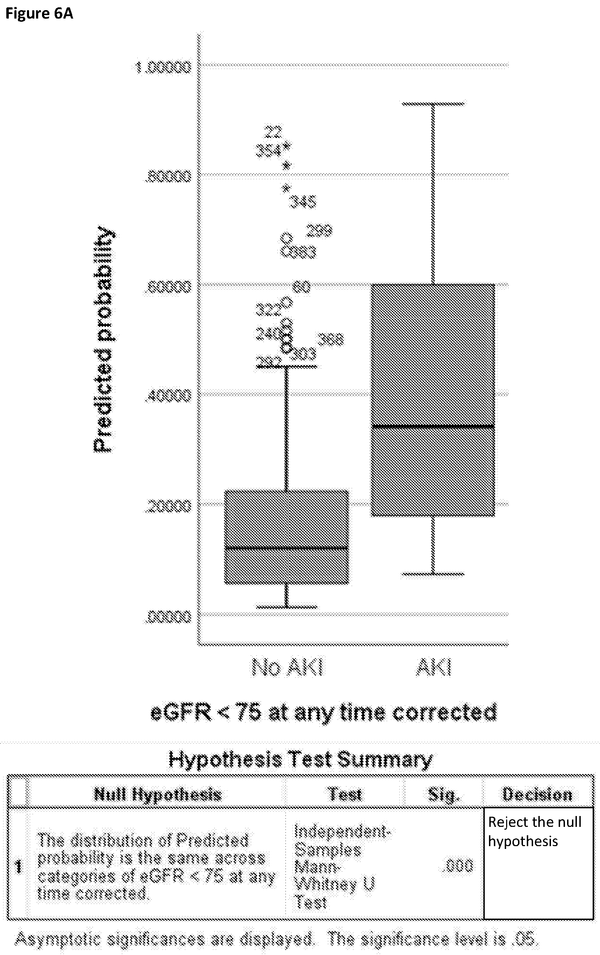

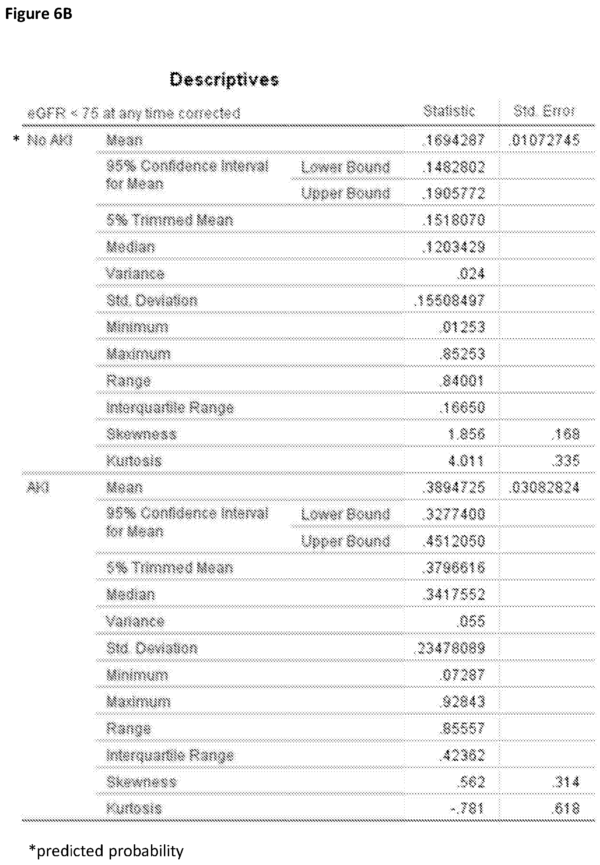

Figure 6 shows a serum post surgery TNFRI, TNFRII and Midkine model;

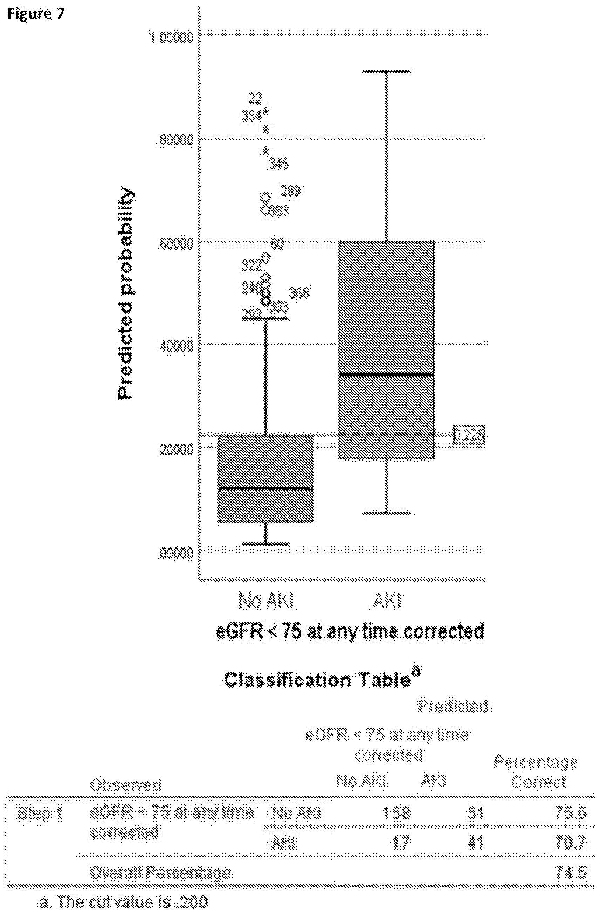

Figure 7 shows a serum post surgery TNFRI, TNFRII and Midkine model;

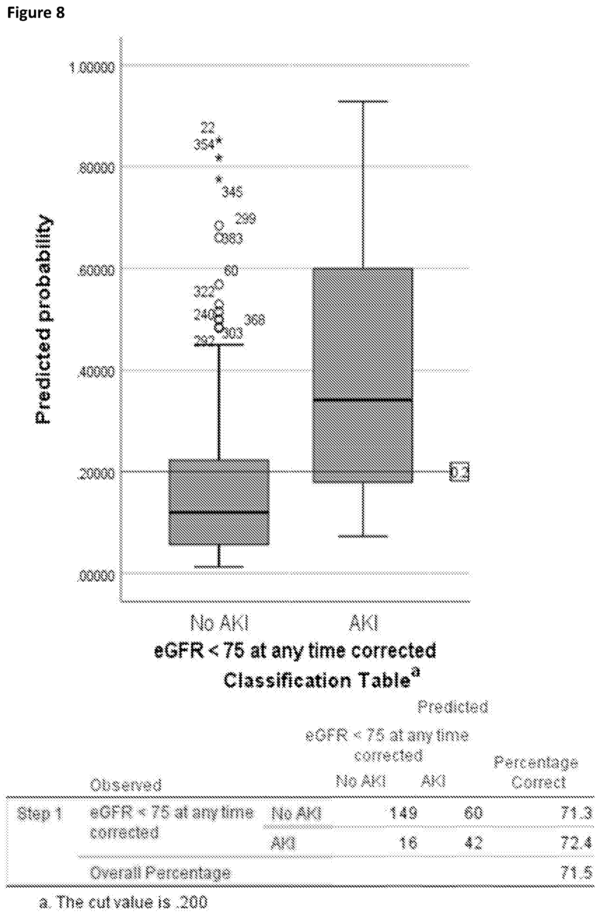

Figure 8 shows a serum post surgery TNFRI, TNFRII and Midkine model;

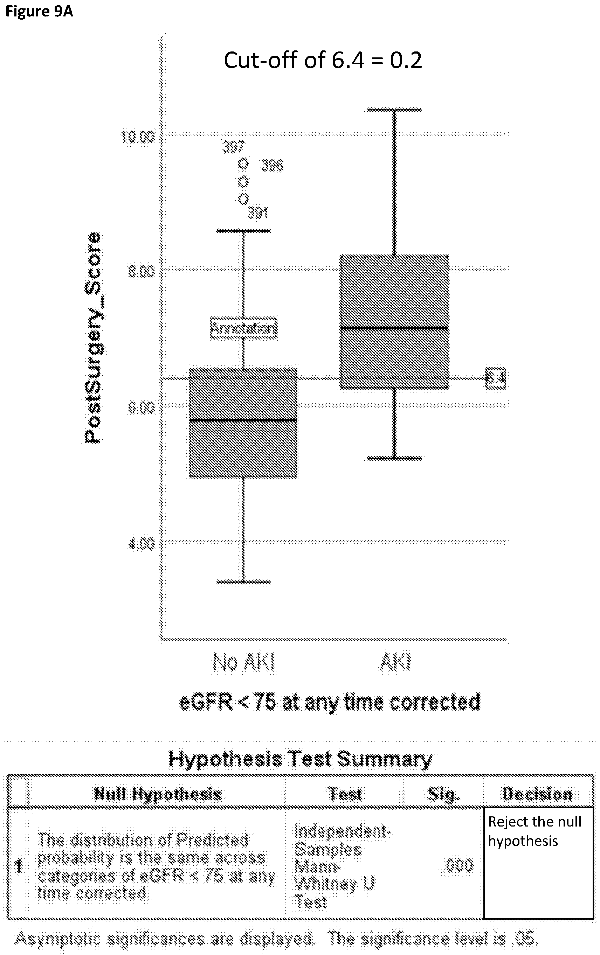

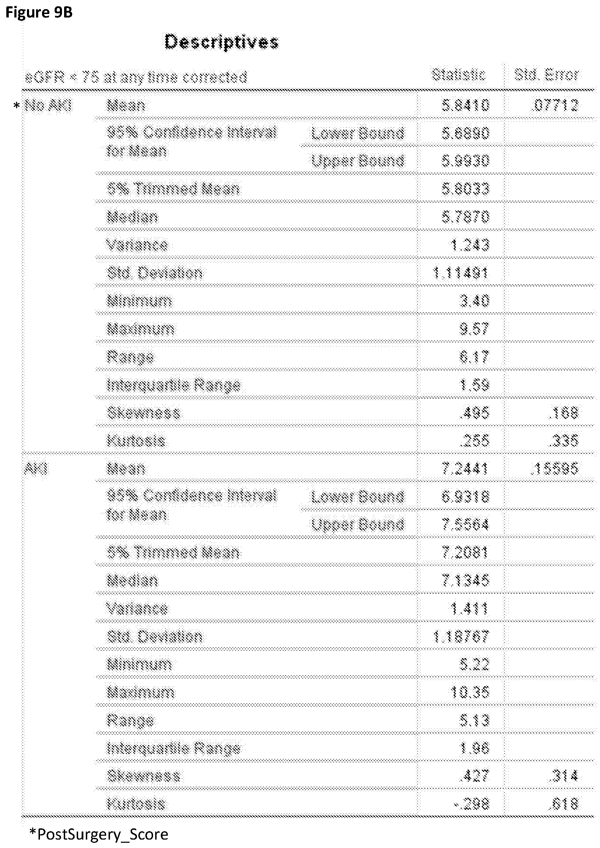

Figure 9 shows patient scores for a serum post surgery TNFRI, TNFRII and Midkine model;

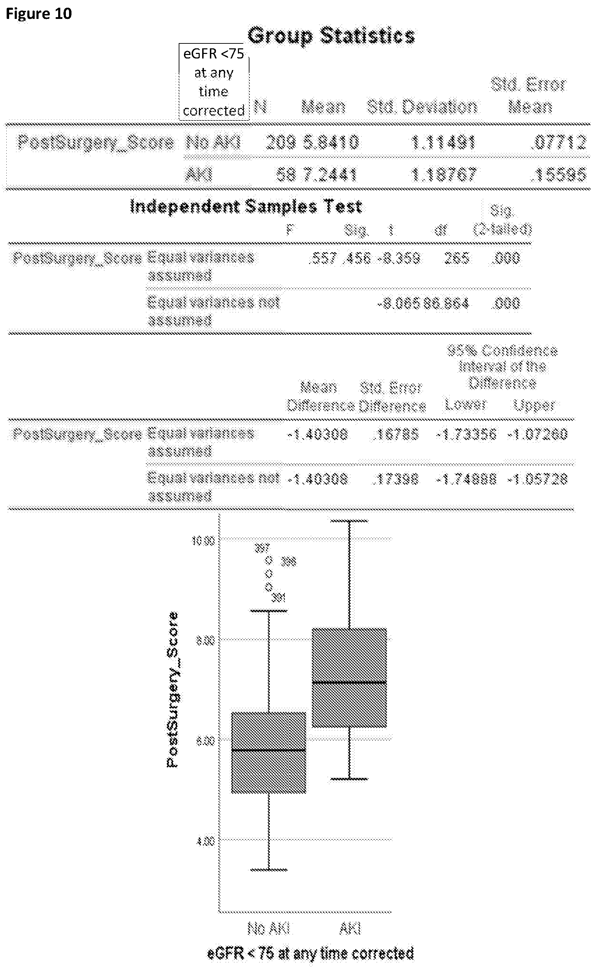

Figure 10 shows patient scores for a serum post surgery TNFRI, TNFRII and Midkine model;

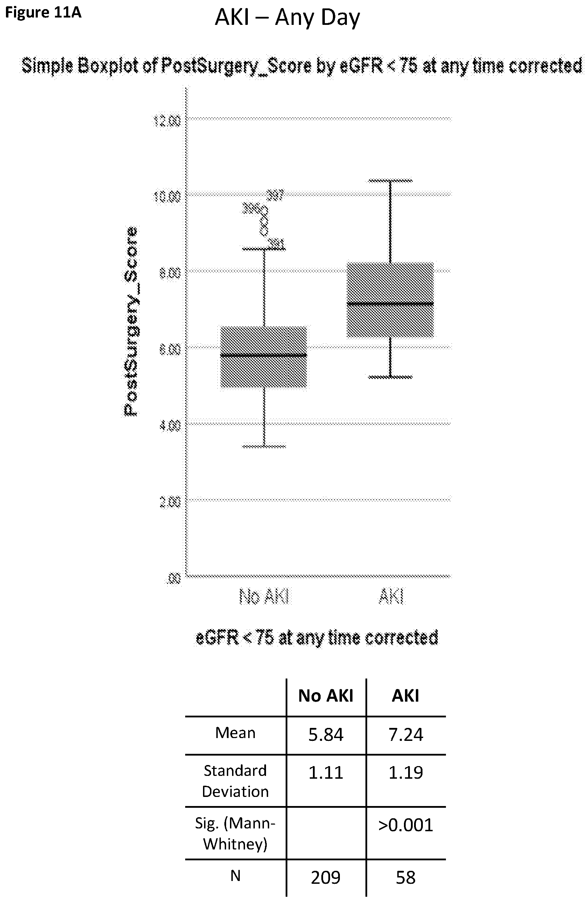

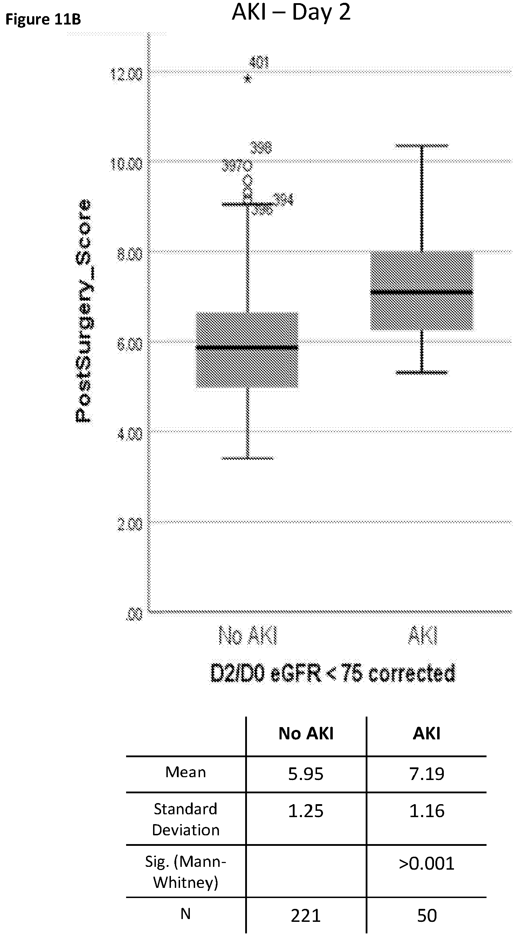

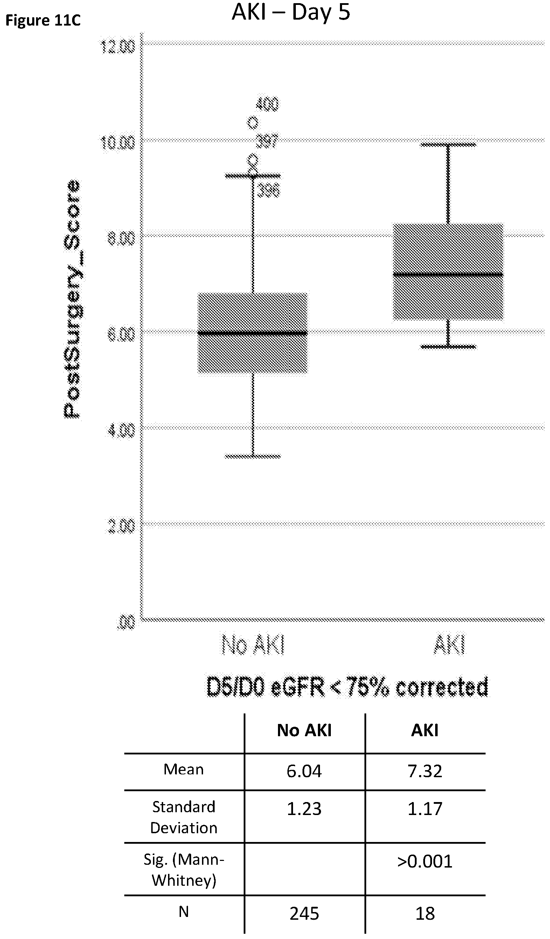

Figure 11 shows patient scores for a serum post surgery TNFRI, TNFRII and Midkine model;

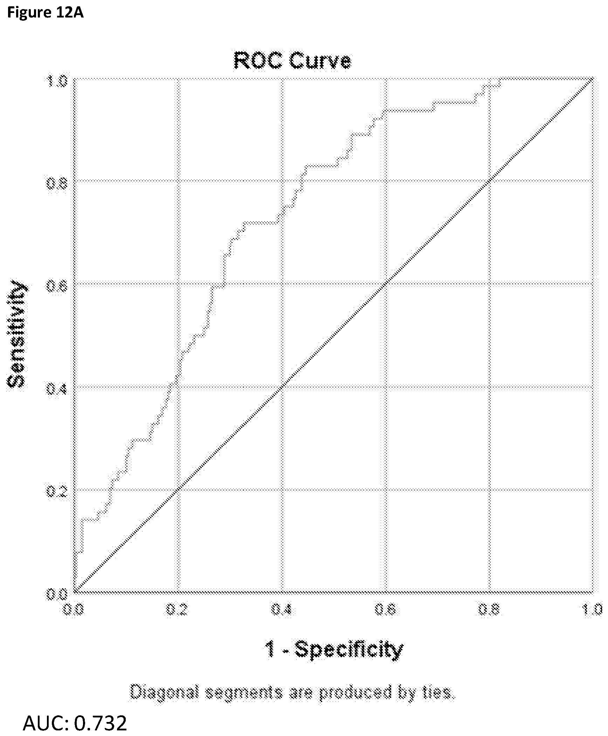

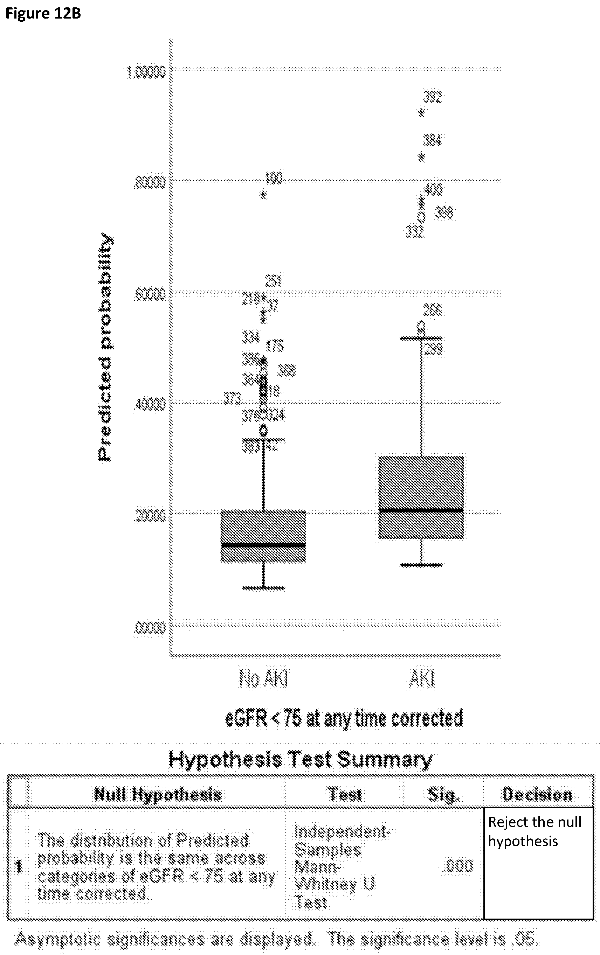

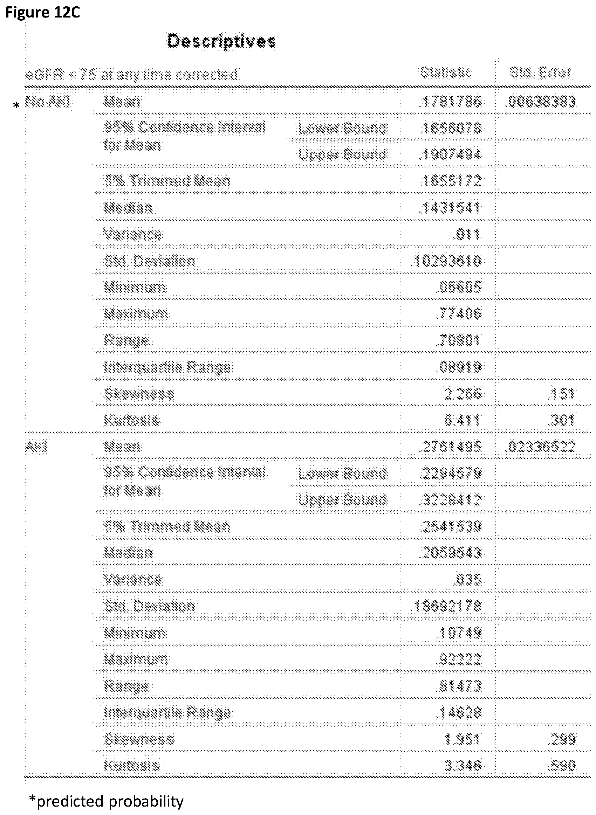

Figure 12 shows ROC for serum pre-surgery TNFRI and TNFRII;

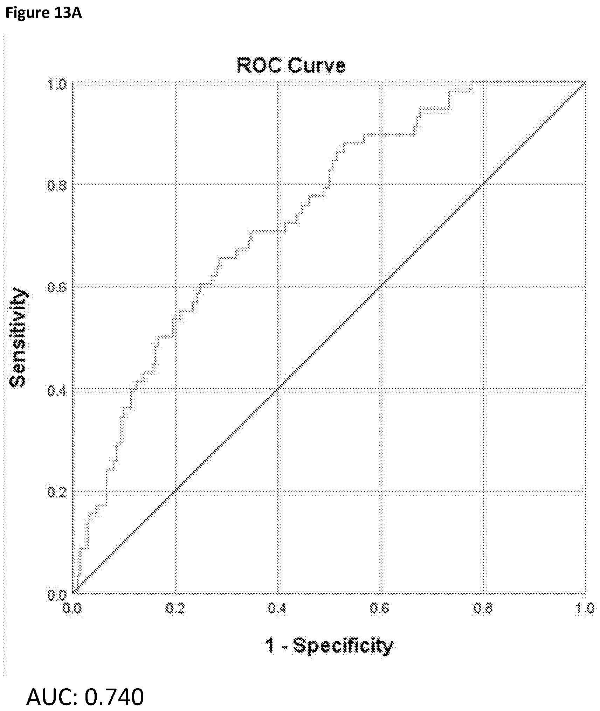

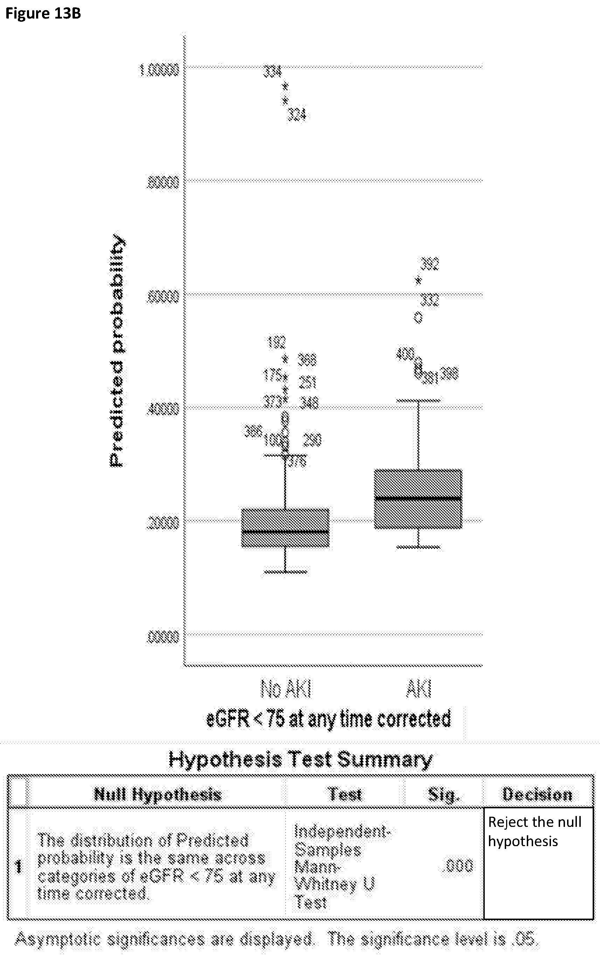

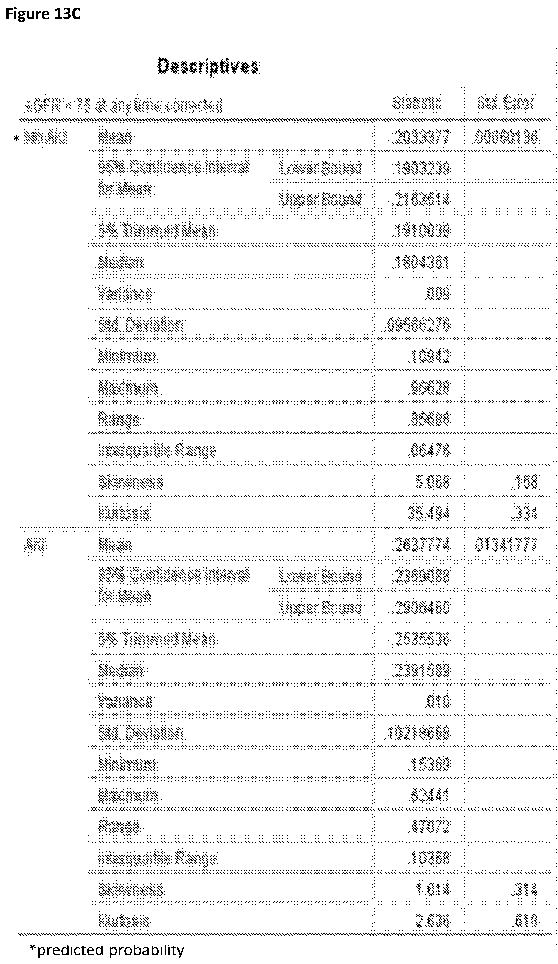

Figure 13 shows serum pre-surgery TNFRI and Midkine;

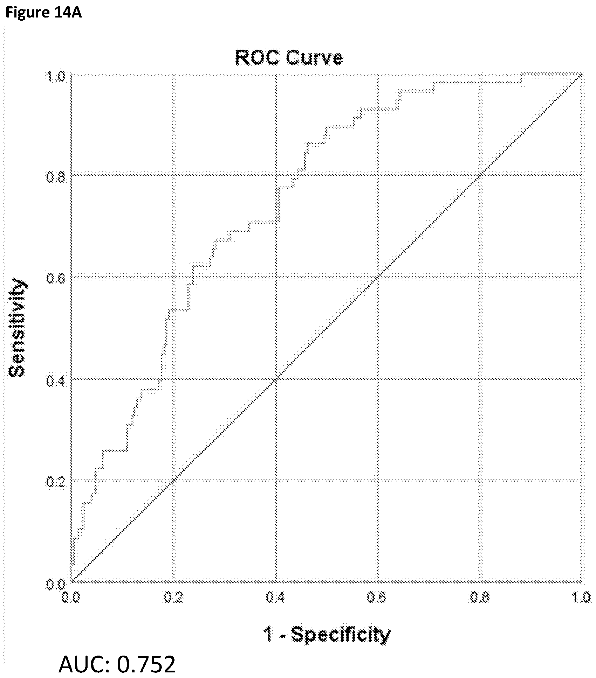

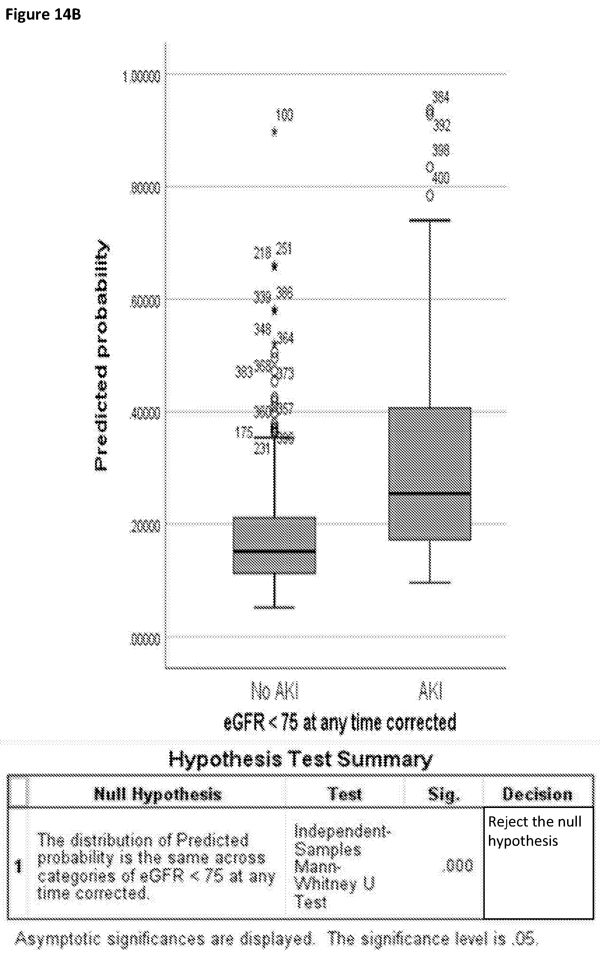

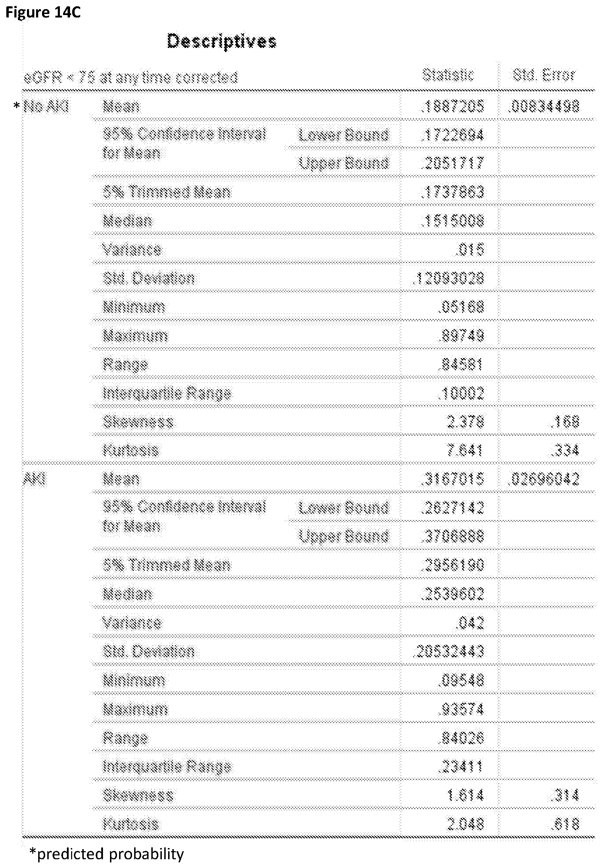

Figure 14 shows serum pre-surgery TNFRII and Midkine;

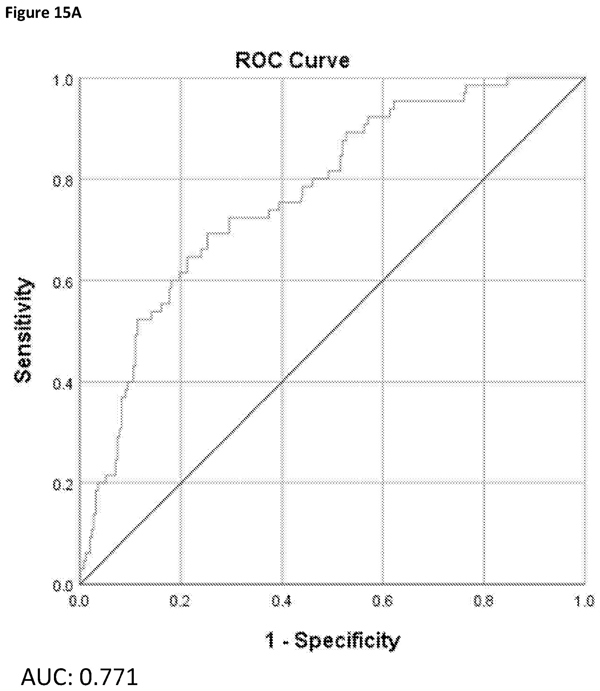

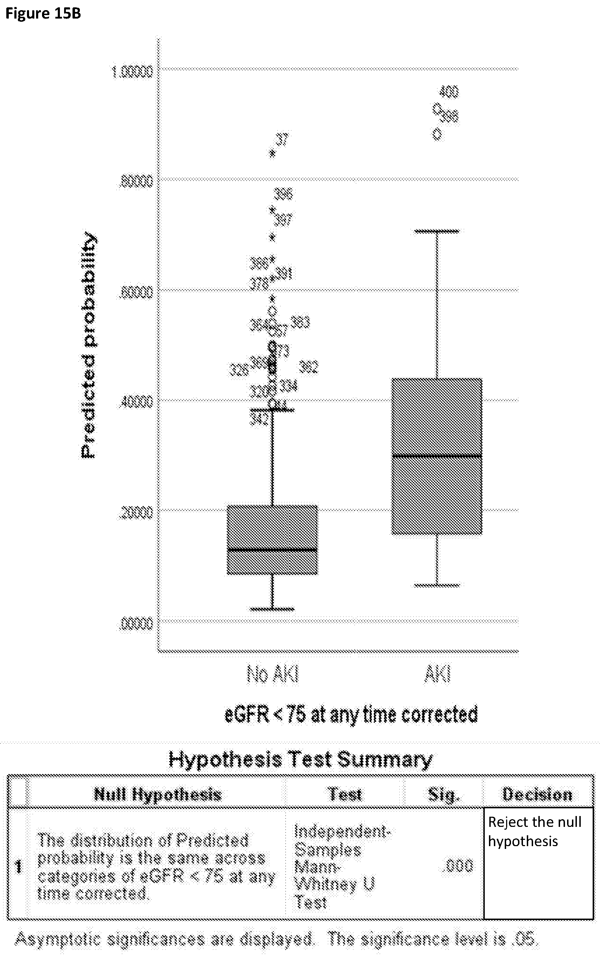

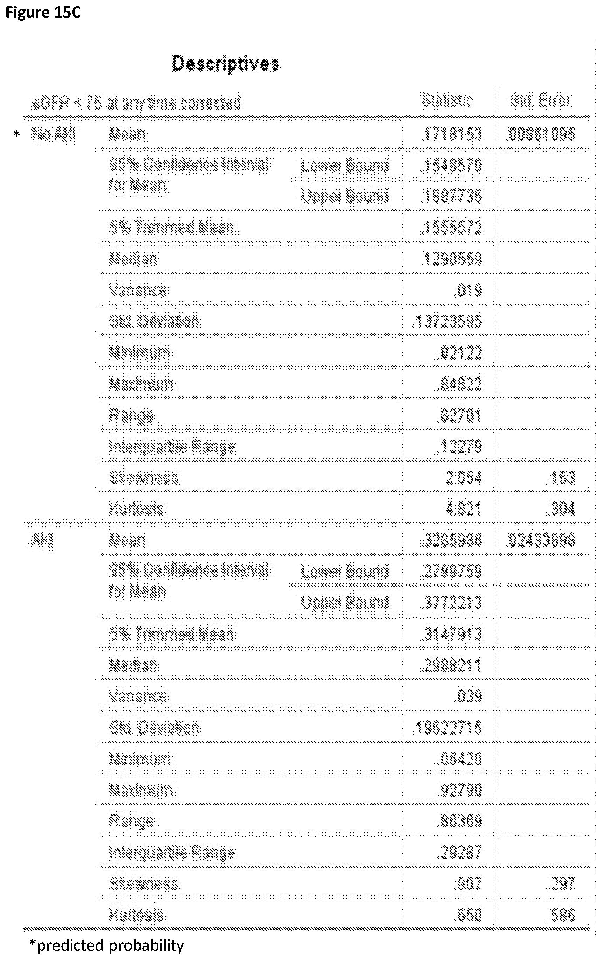

Figure 15 shows serum post surgery TNFRI and TNFRII;

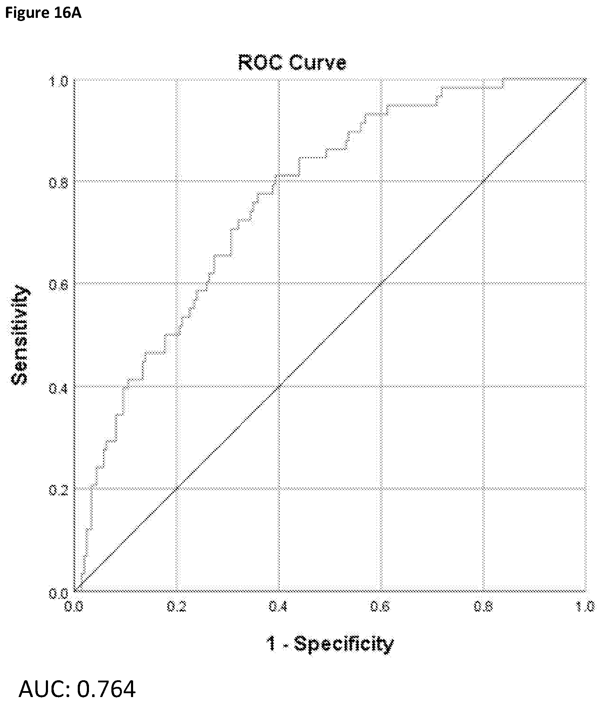

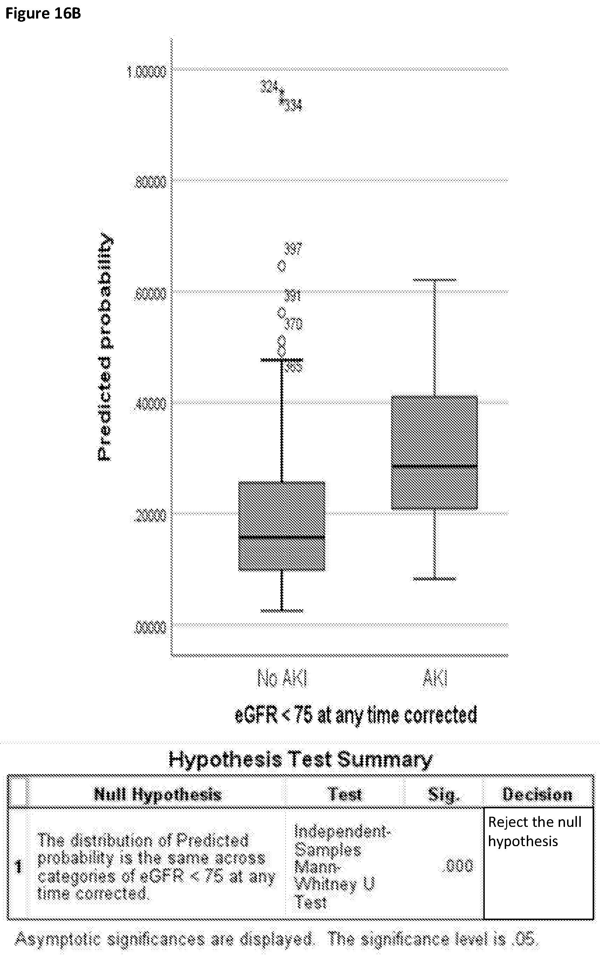

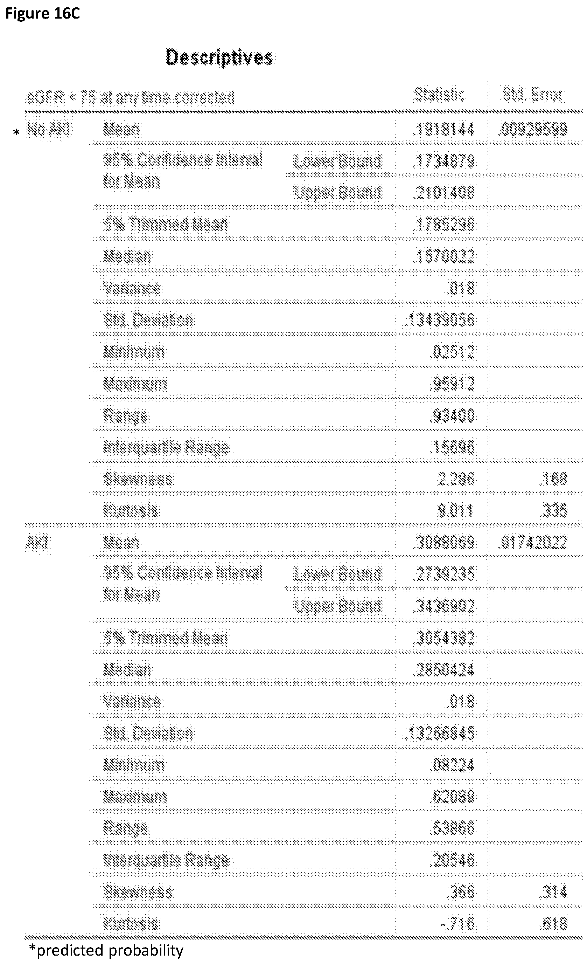

Figure 16 shows serum post-surgery TNFRI and Midkine;

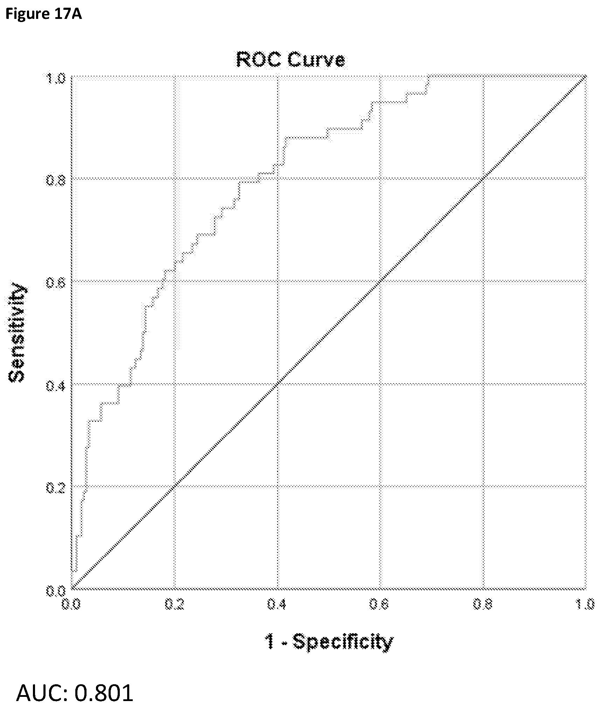

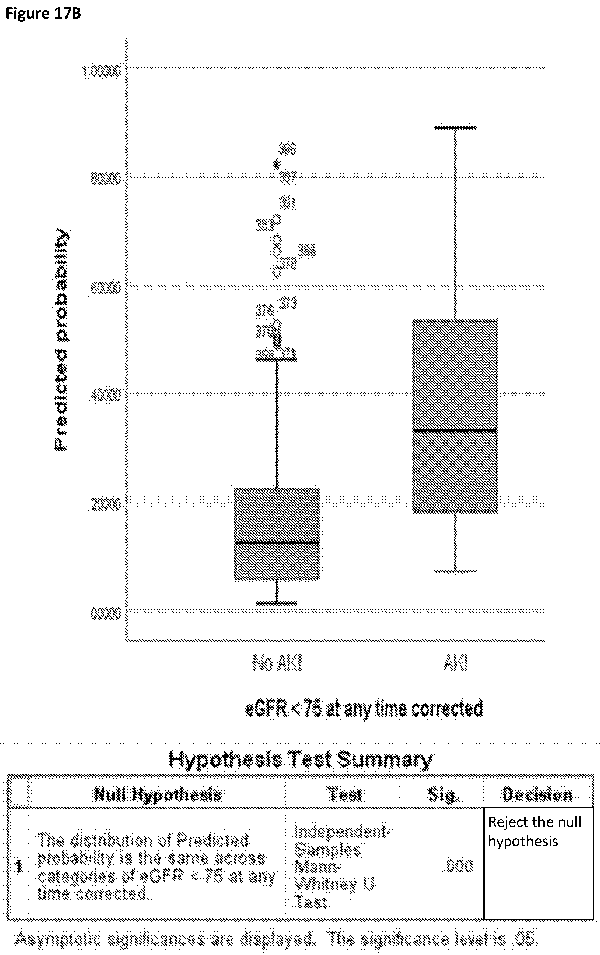

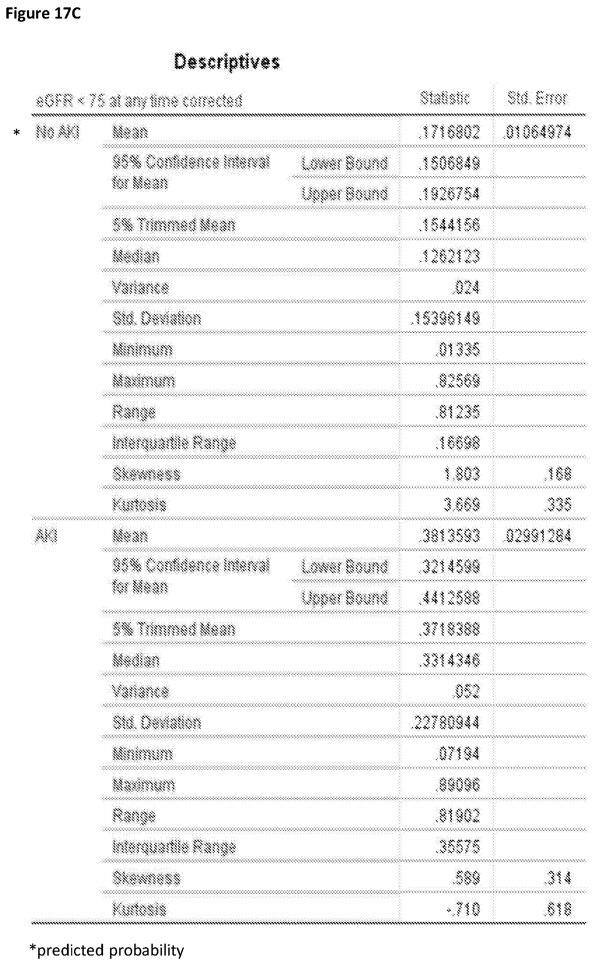

Figure 17 shows serum post-surgery TNFRII and Midkine;

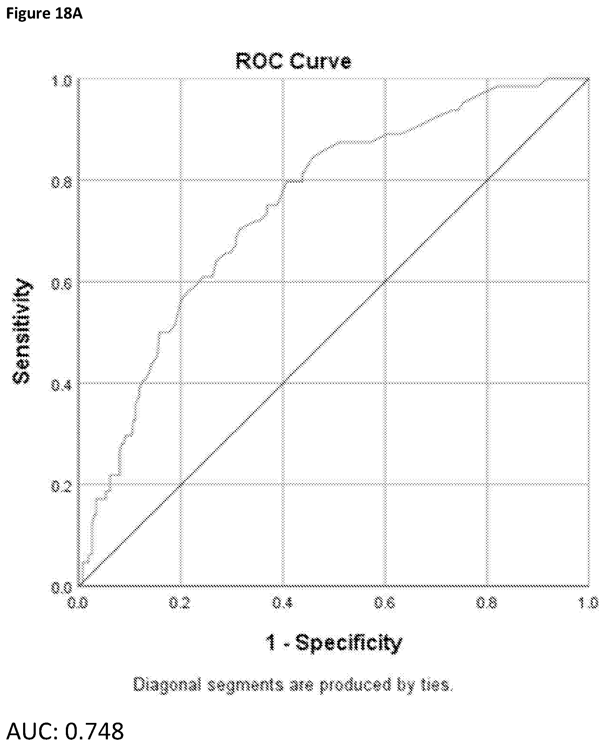

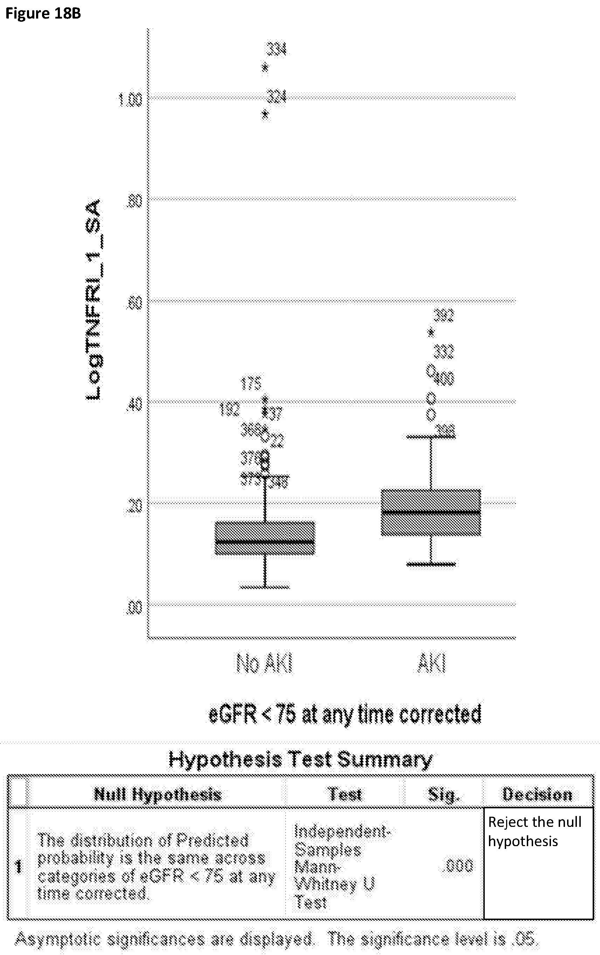

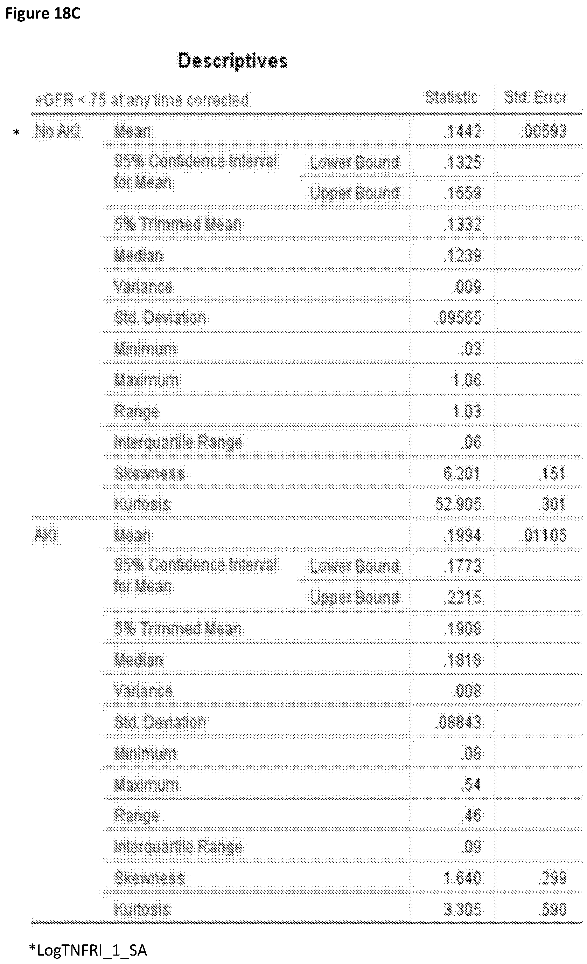

Figure 18 shows serum pre-surgery TNFRI;

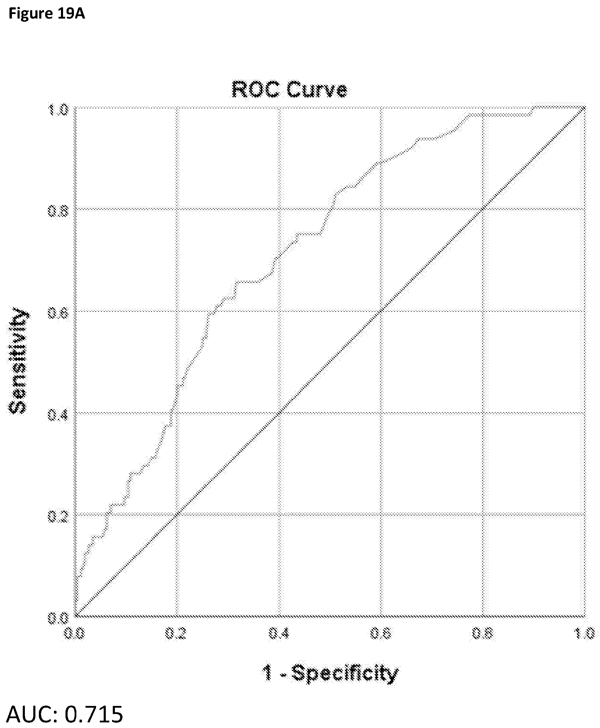

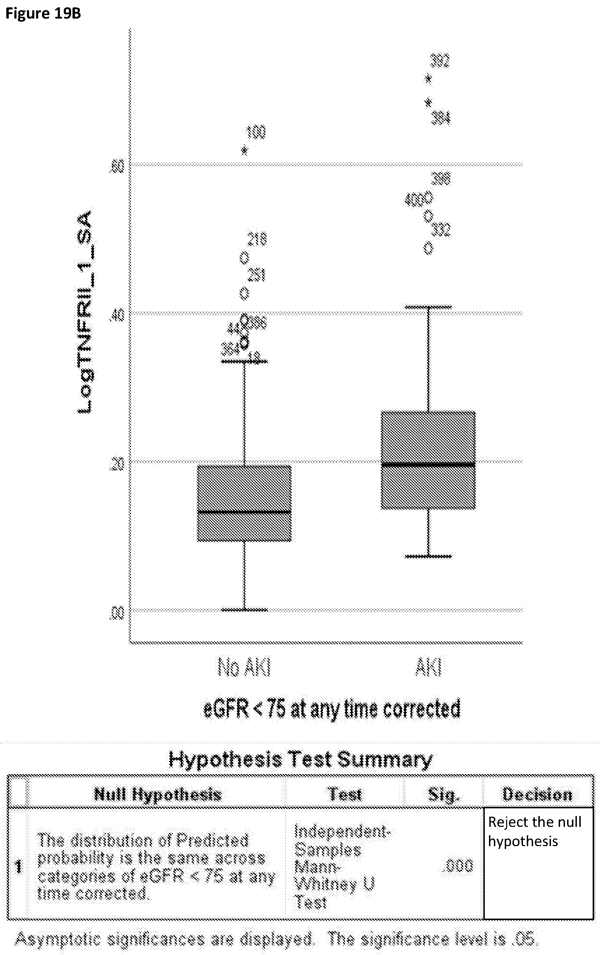

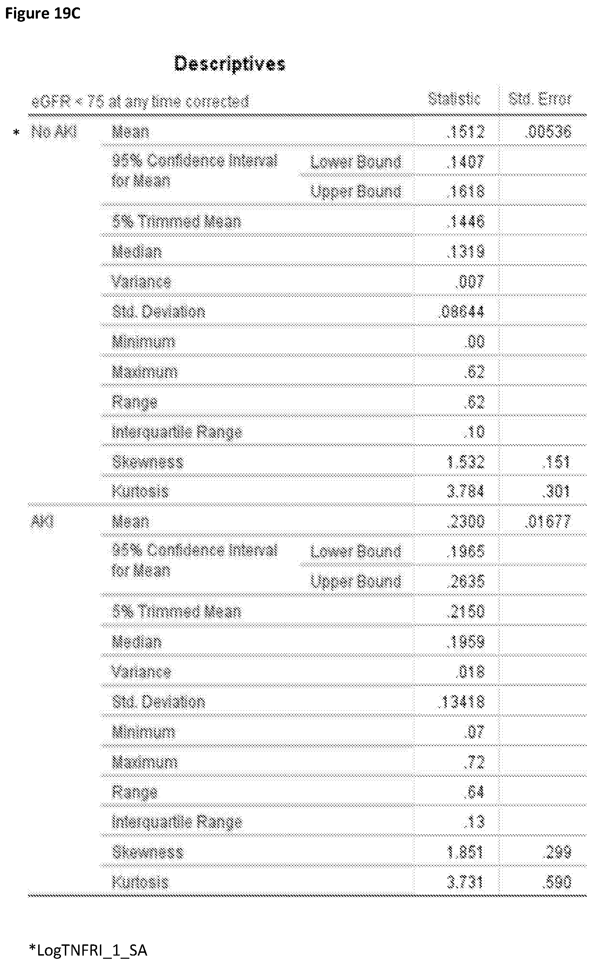

Figure 19 shows serum pre-surgery TNFRII;

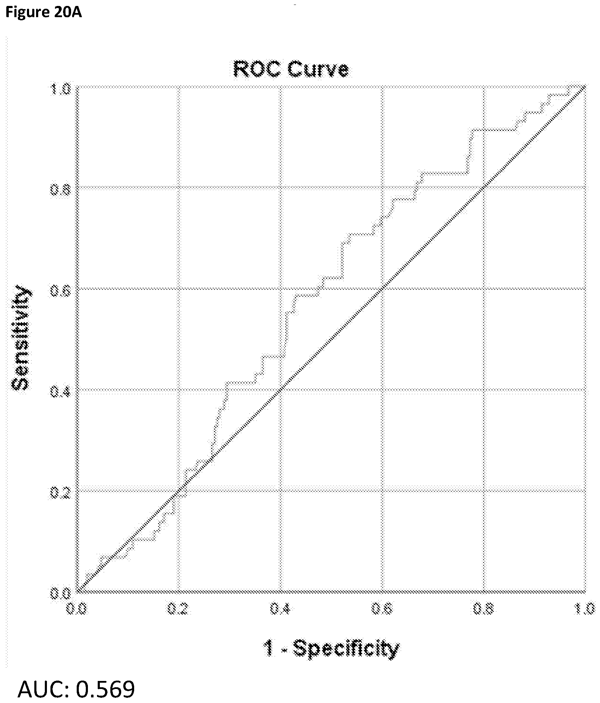

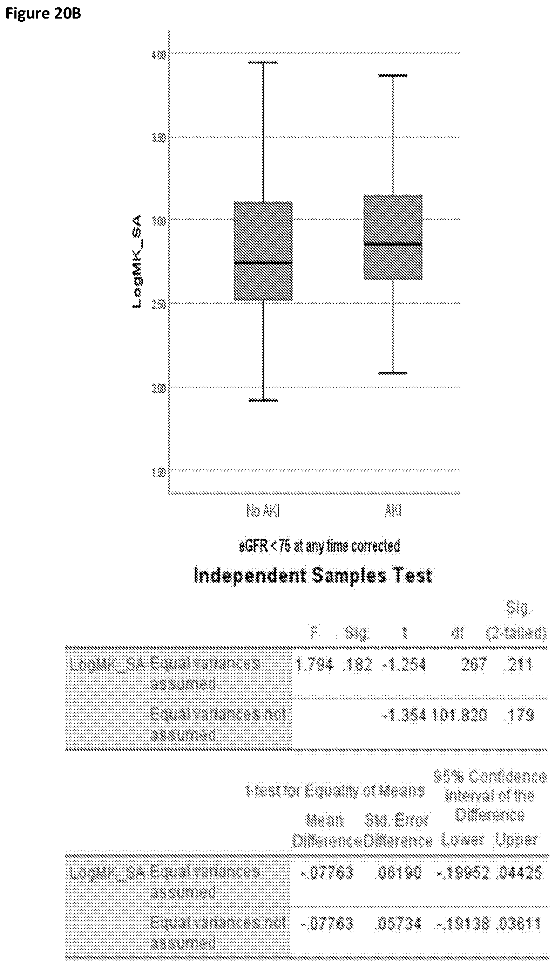

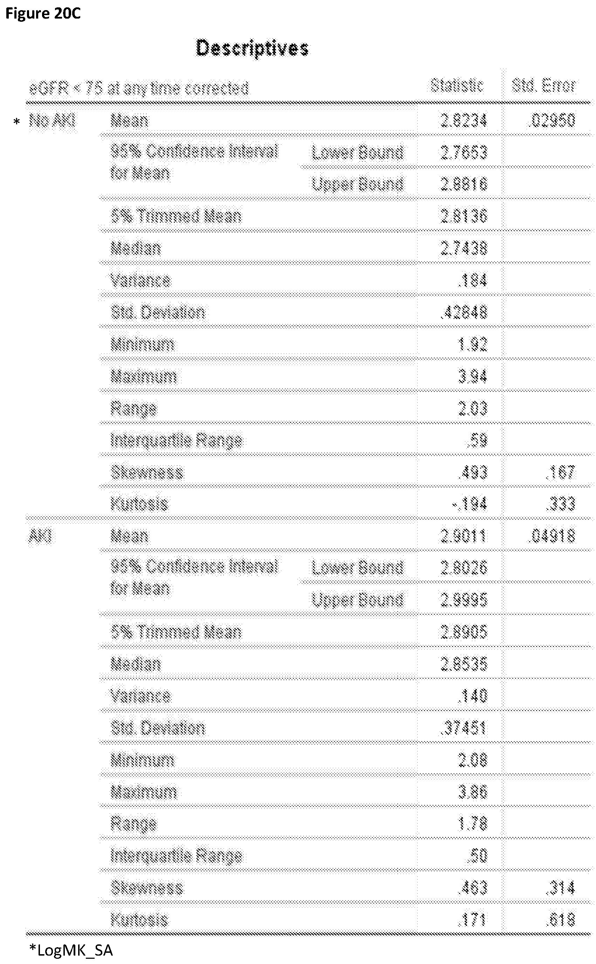

Figure 20 shows serum pre-surgery Midkine;

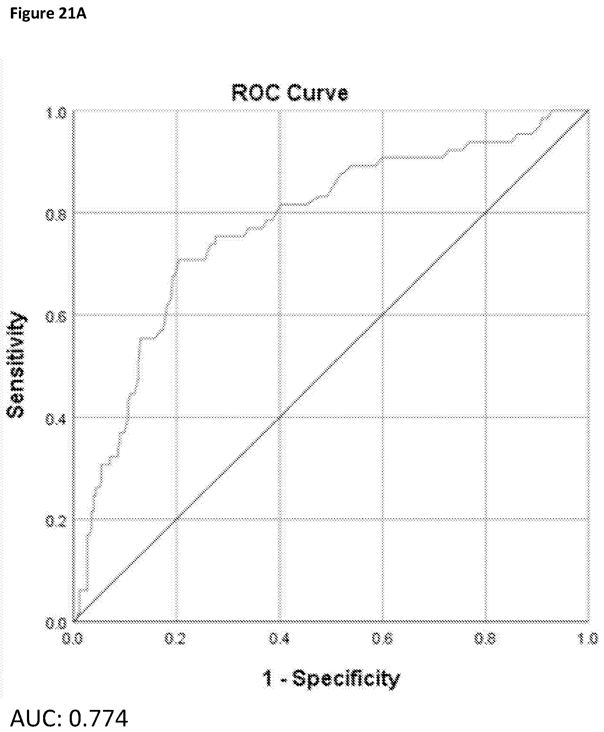

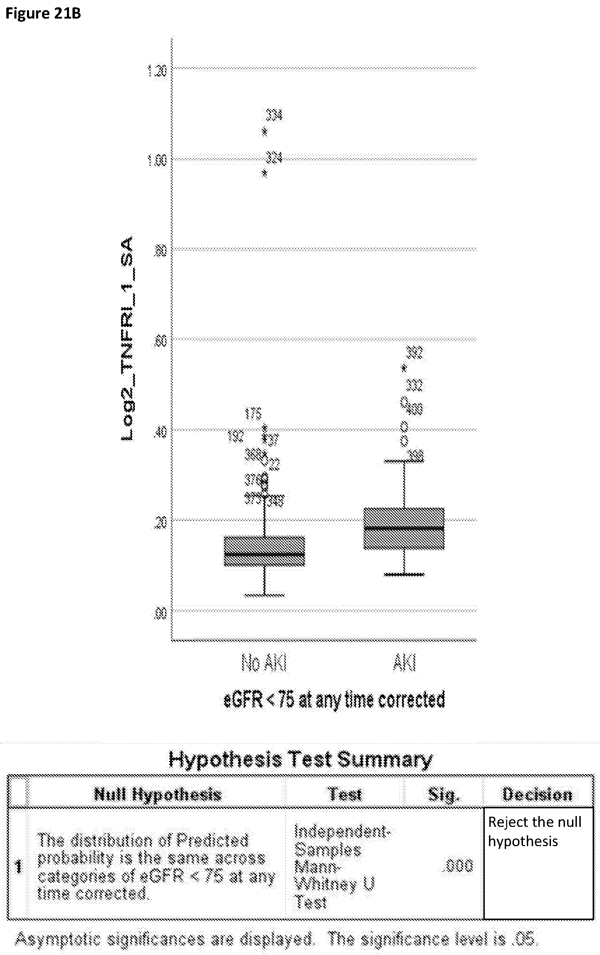

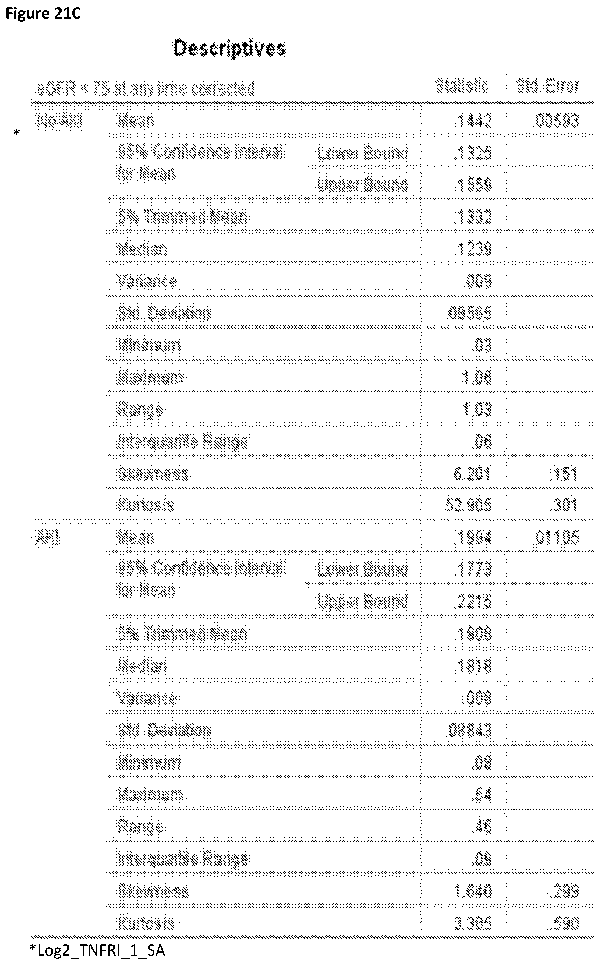

Figure 21 shows serum post-surgery TNFRI;

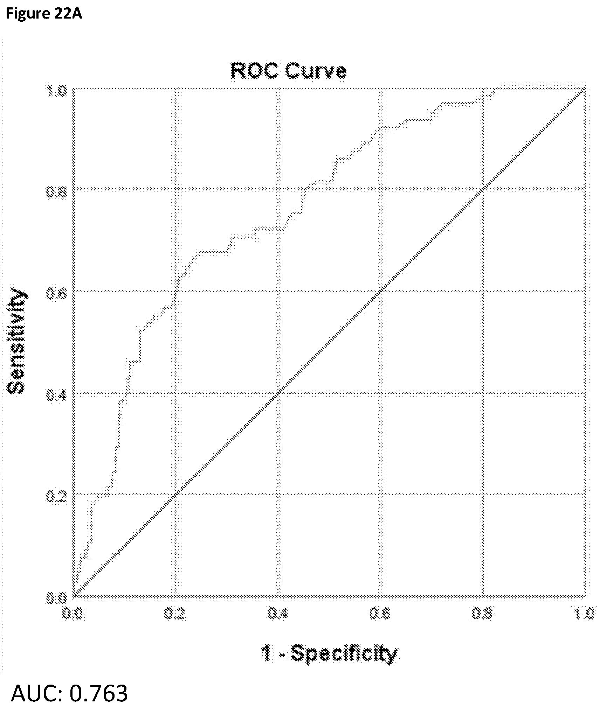

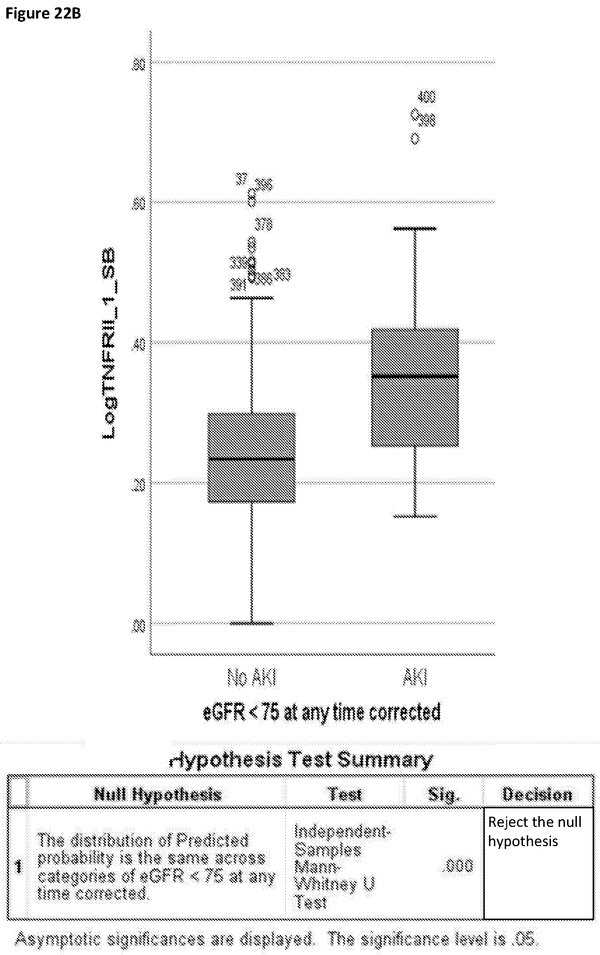

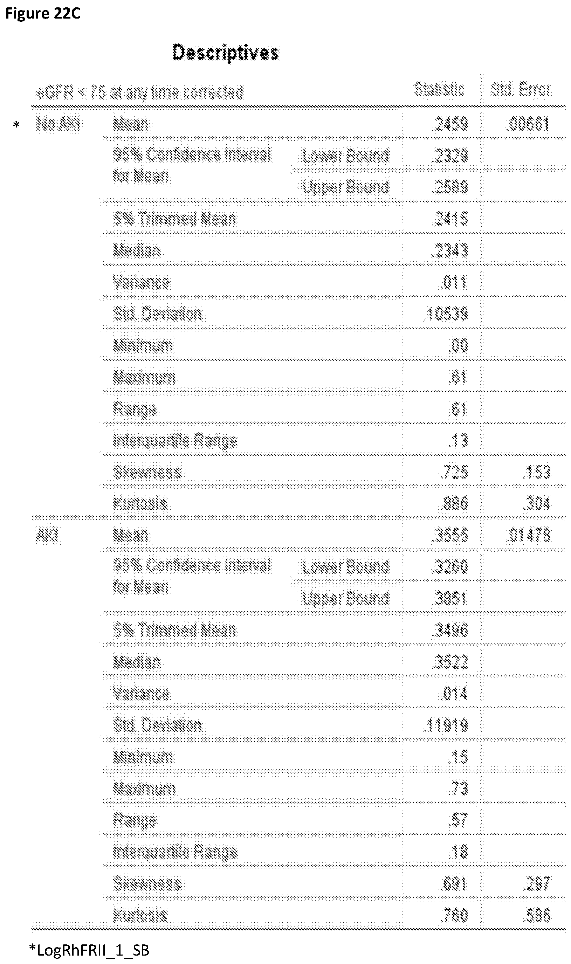

Figure 22 shows serum post-surgery TNFRII;

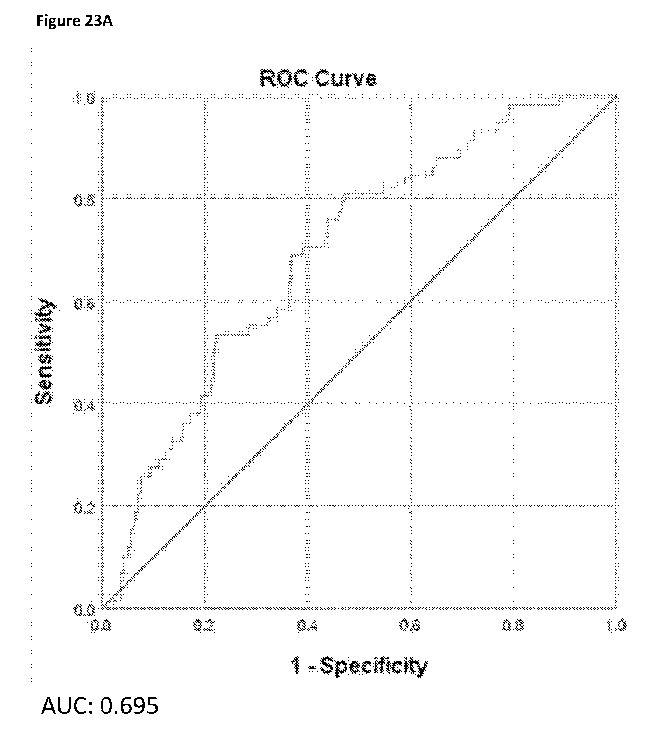

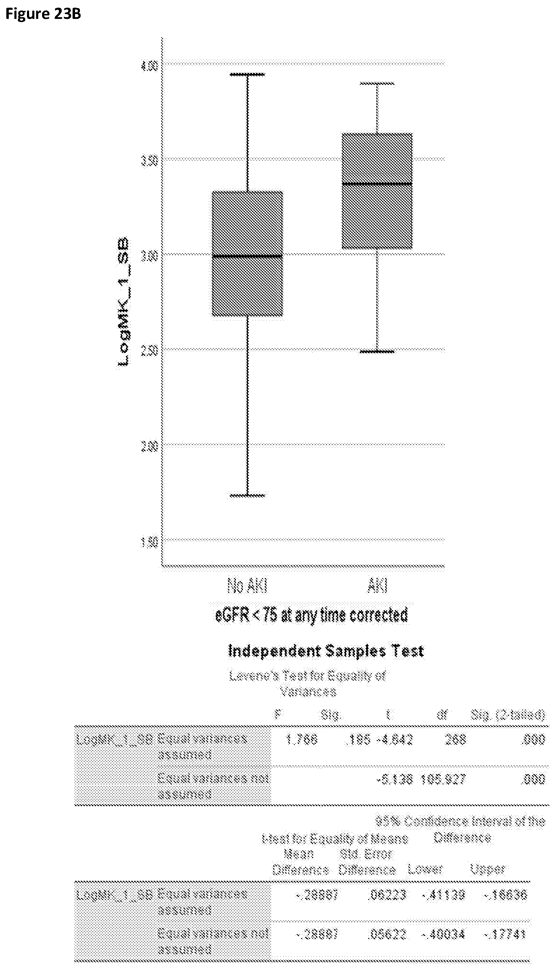

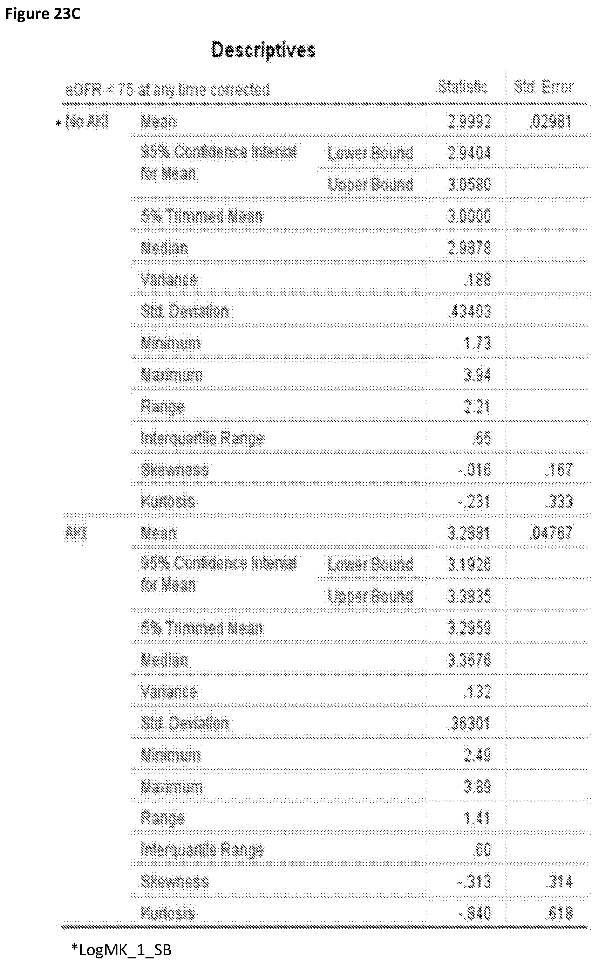

Figure 23 shows serum post-surgery Midkine;

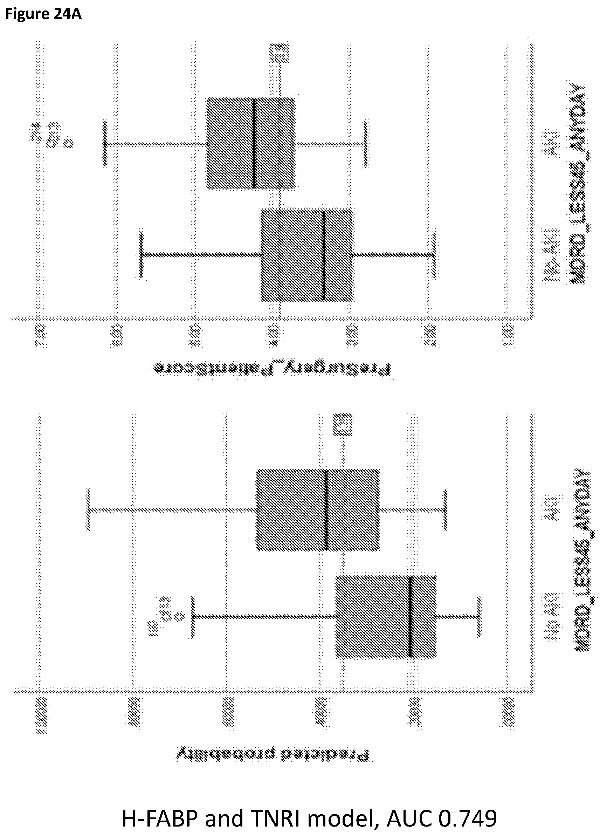

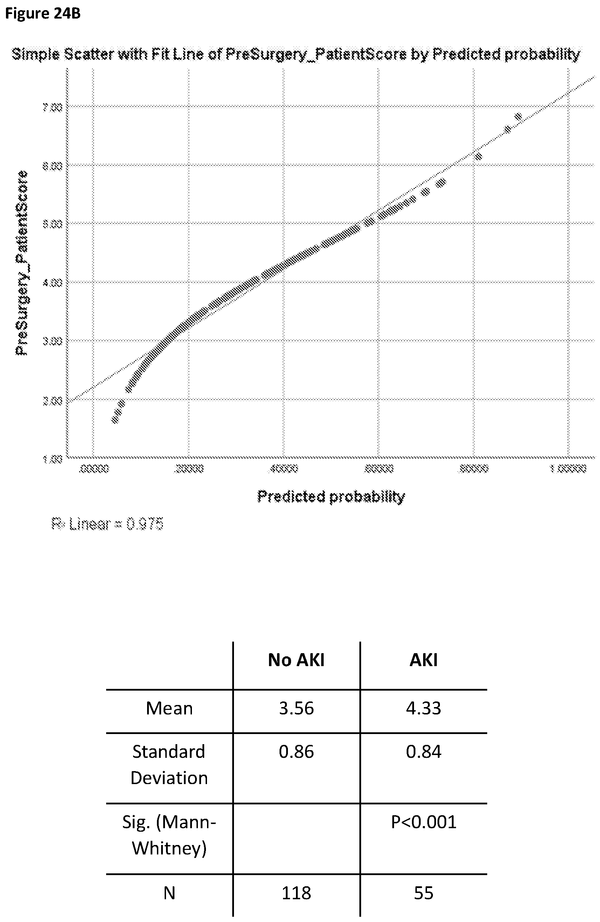

Figures 24 A and B show serum pre-fracture repair surgery;

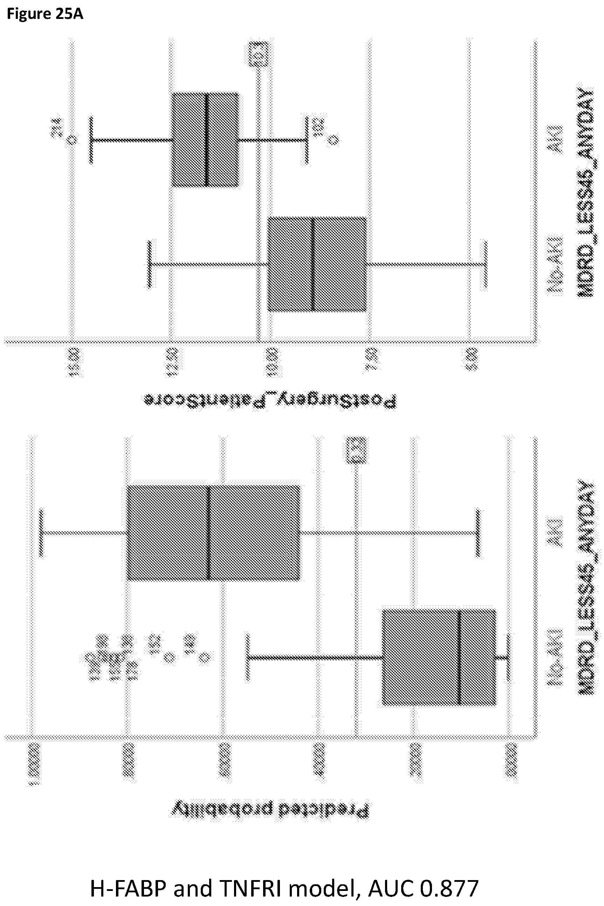

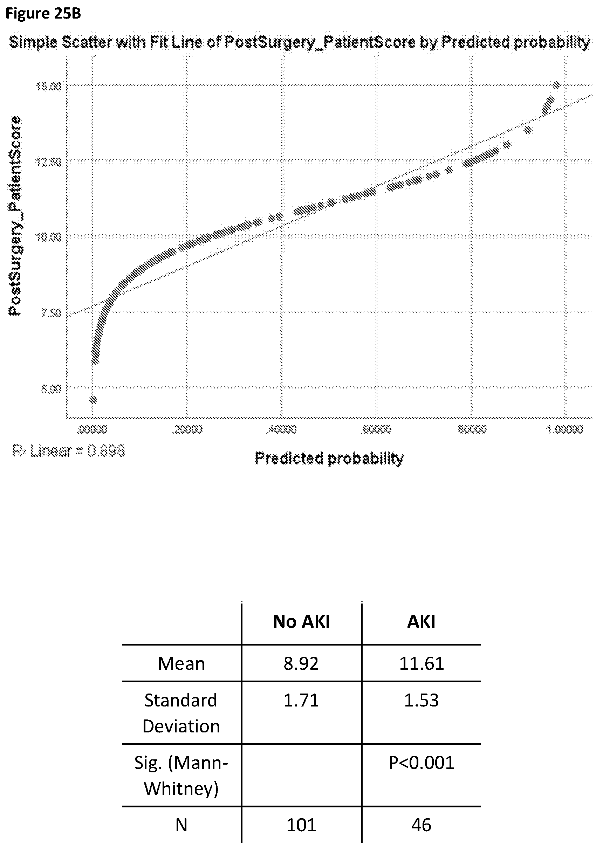

Figures 25 A and B show serum post fracture repair surgery;

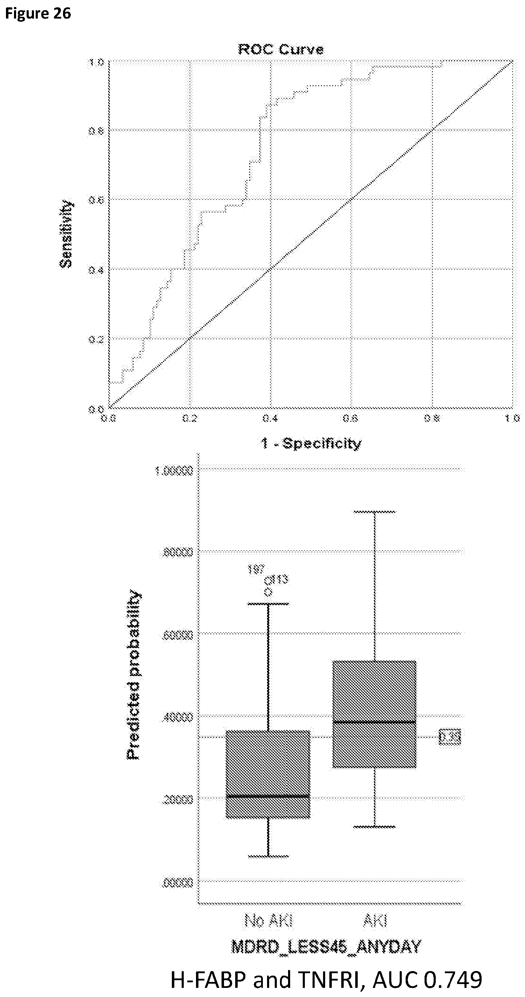

Figure 26 illustrates Serum pre-surgery predictive model H-FABP and TNFRI;

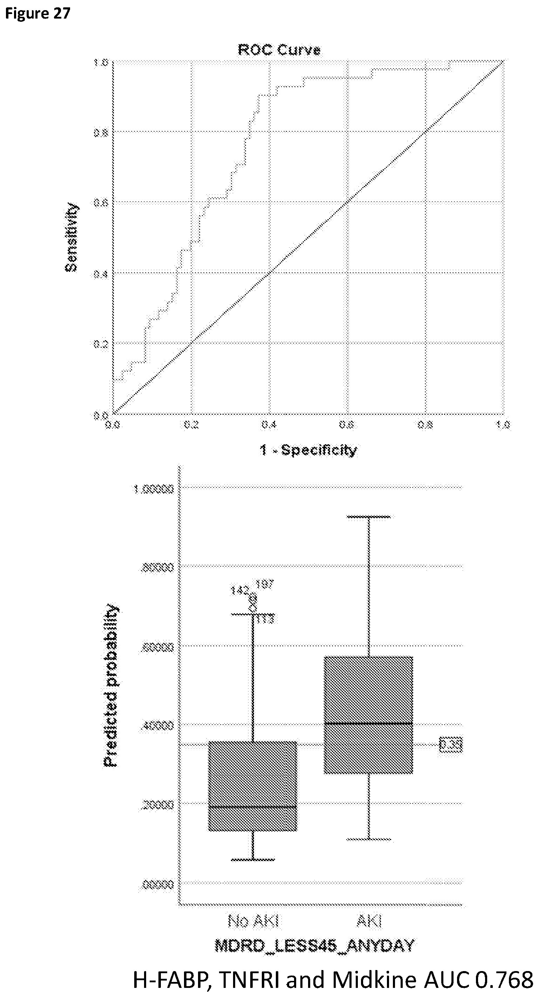

Figure 27 illustrates Serum pre-surgery predictive model H-FABP, TNFRI and Midkine;

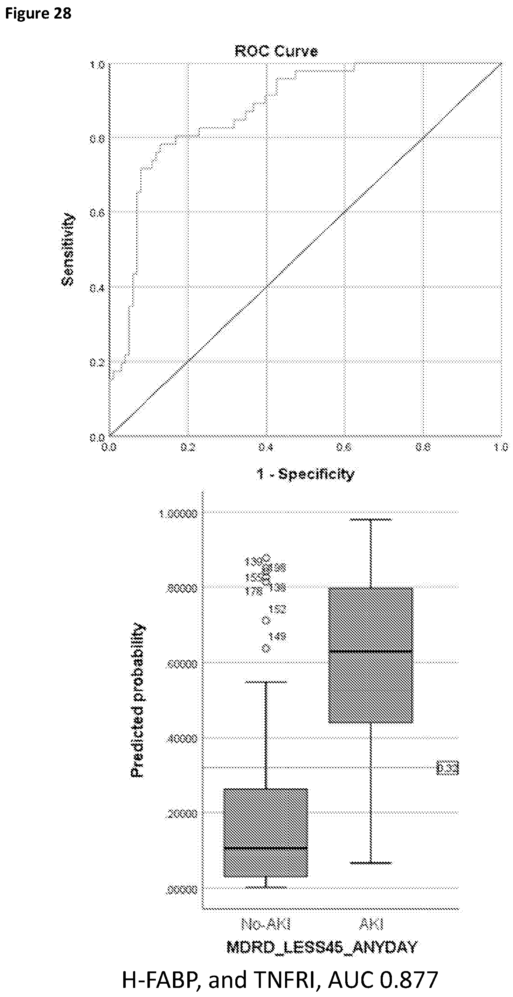

Figure 28 illustrates Serum post-surgery model H-FABP and TNFRI;

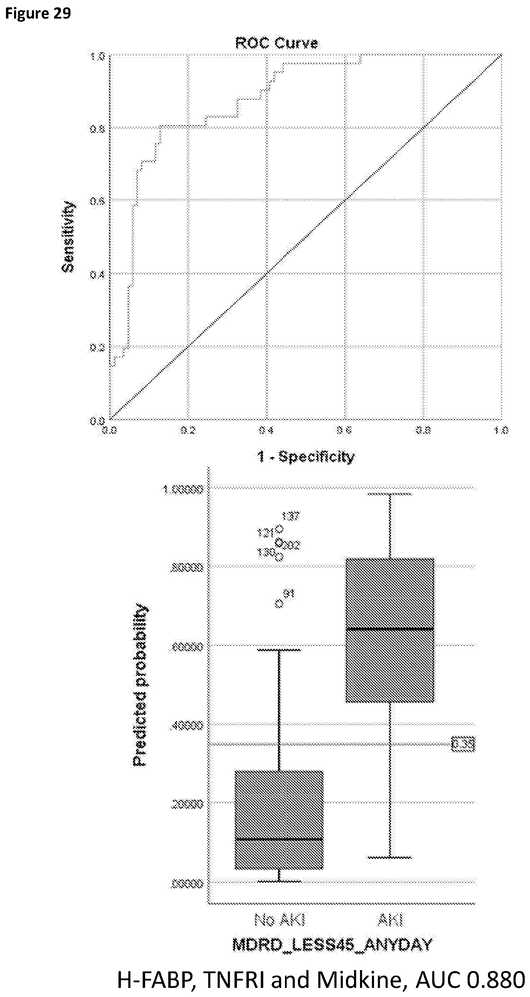

Figure 29 illustrates Serum post-surgery H-FABP, TNFRI and Midkine biomarker model;
Figure 30 illustrates biomarker results;
Figure 31 illustrates biomarker results;
Figure 32 illustrates biomarker results;
Figure 33 illustrates biomarker results;
Figure 34 illustrates biomarker results;
Figure 35 illustrates biomarker results;
Figure 36 illustrates biomarker results;
Figure 37 illustrates biomarker results;
Figure 38 illustrates biomarker results;
Figure 39 illustrates biomarker results;
Figure 40 illustrates biomarker results;
Figure 41 illustrates biomarker results;
Figure 42 illustrates biomarker results;
Figure 43 illustrates biomarker results;
Figure 44 illustrates biomarker results; and
Figure 45 illustrates biomarker results.

### Experimental Methods

Ethical approval was granted from the Research Ethics Committee and the Royal Research Office research governance committee.

Cardiac surgery patients were consecutively scheduled for elective and emergency cardiac surgery within the Cardiac Surgical Unit of the Royal Victoria Hospital Belfast while the orthopaedic trauma patients were consecutively scheduled for open reduction internal fixation of their fracture within the fracture unit of the Belfast Trust. Exclusion criteria for all patients was pre-operative or pre-trauma dialysis dependent renal failure or known significant renal disease prior to entrance into the study (known eGFR < 30).

### Sampling protocol

Urinalysis (Sample A) (20ml) was performed on admission (for fracture patients) or for cardiac patients following catheterisation on induction of anaesthesia and (Sample B) (20ml) on day 1 post-operatively.

Blood Sample A (20ml) was performed on admission for fracture patients together with their routine pre-operative blood sample work-up such that participation in this study did not involve additional venepuncture. Blood sample A for cardiac patients followed routine arterial line insertion pre-operatively. Day 1 post-operative Sample B (20ml) for fracture patients was taken at the time of routine analyses, hence not requiring further venepuncture. If, for a fracture patient, a blood sample was inadvertently missed during the time that routine bloods are being taken, it was not pursued to avoid additional discomfort of venepuncture beyond what was required for routine care, unless that patient, in intensive care or HDU had a routinely placed arterial catheter in situ. Unlike many fracture patients who do not have a routinely placed arterial line, all cardiac surgery patients have an arterial line inserted pre-operatively, remaining in situ for 48 hours such that obtaining the post-operative day 1 blood Sample B will be painless.

Blood and urine samples were immediately centrifuged in the clinical area and the

| | **Non-AKI patients** | **AKI patients** |
|---|---|---|
| **AKI Day 2** | 220 | 50 |
| **AKI Day 5** | 246 | 18 |
| **AKI Any Day** | 210 | 58 |

resulting supernatants stored in fridges. Each week these samples were transported to Randox Laboratories Ltd for storage and analysis.

### Example 1 - Cardiac Surgery

Acute Kidney Injury (AKI) was defined as a drop of baseline eGFR of >25%. A drop in eGFR was recorded on day 1, day 2 and day 5 following surgery. To increase the number of patients in the population, the analyses are based upon an 'AKI any-day' definition. Patients were included in this category if eGFR dropped to less than 75% of baseline, at any day following cardiac surgery. Based on this criteria, a population was described.

*Table 1: AKI is defined as a drop of eGFR >25% from baseline. The number of patients in each group is shown for each of the AKI timepoints.*

The levels of biomarkers were then determined from the AKI and non-subjects for for plasma, serum and urinary samples. Biomarkers were highlighted as significant at p< 0.05 between the AKI and non-AKI groups using a Mann-Whitney U test. The predictive ability of individual biomarkers, identified as significant by Mann-Whitney U test, was investigated by ROC analyses.

Results for plasma, serum and urinary biomarkers are shown in Tables 2, 3 and 4, respectively.

**Table 2: Plasma biomarkers identified as showing a significant difference between AKI and non-AKI.**

| **Marker** | **Pre/ Post surgery** | **Sig.** | **AUC** |
|---|---|---|---|
| **IL-6** | Pre | 0.001 | 0.630 |
| **IL-1a** | Pre | 0.040 | 0.628 |
| **VEGF** | Pre | 0.002 | 0.628 |
| **INFγ** | Pre | 0.030 | 0.550 |
| **TNFα** | Pre and Post | <0.001, <0.001 | 0.645 |
| **MCP** | Pre and Post | 0.010, 0.001 | 0.604 |
| **MIP1α** | Pre and Post | 0.001, <0.001 | 0.639 |
| **NGAL** | Pre and Post | 0.001, <0.001 | 0.632 |
| **IL-8** | Post | 0.005 | 0.614 |
| **IL-10** | Post | 0.009 | 0.604 |

**Table 3: Serum biomarkers identified as showing a significant difference between AKI and non-AKI.**

| **Marker** | **Pre/Post surgery** | **Sig.** | **AUC** |
|---|---|---|---|
| **IL12P40** | Pre and Post | <0.001, <0.001 | 0.682, 0.714 |
| **IL1RA** | Pre and Post | 0.005, <0.001 | 0.542, 0.687 |
| **IP10** | Pre and Post | 0.012, 0.046 | 0.601, 0.580 |
| **TNFRI** | Pre and Post | <0.001, <0.001 | 0.748, 0.748 |
| **TNFRII** | Pre and Post | <0.001, <0.001 | 0.715, 0.763 |
| **H-FABP** | Pre and Post | 0.001, 0.001 | 0.637, 0.637 |
| **Midkine** | Post | <0.001 | 0.695 |

**Table 4: Urine biomarkers identified as showing a significant difference between AKI and non-AKI.**

| **Marker** | **Pre/ Post Surgery** | **Sig.** | **AUC** |
|---|---|---|---|
| **TNFRI** | Pre and Post | 0.044, 0.020 | 0.581, 0.593 |
| **TNFRII** | Pre and Post | 0.018, 0.018 | 0.595, 0.595 |
| **IL12P40** | Pre and Post | 0.035, <0.001 | 0.580, 0.667 |
| **IL10** | Post | 0.001 | 0.637, 0.637 |
| **IL1Ra** | Post | 0.024 | 0.591 |
| **NGAL** | Post | <0.001 | 0.651 |

Biomarker combinations were then considered which provided the greatest ability to predict AKI from non-AKI subject groups.

### Example 2 - Fracture surgery

AKI could not be defined using the same definition as applied in the cardiac population, namely a drop of >25% in eGFR from baseline, as patients may already have AKI, as a result of their fracture injury, before surgery. The Classification of AKI in the fracture population was based on the RIFLE system. Patients were assumed to have a baseline GFR of at least 60ml/min/1.73m² therefore, a value of less than 45ml/min/1.73m² was used to define a patient as AKI positive.

The levels of biomarkers were then determined from the AKI and non-subjects for for plasma, serum and urinary samples. Biomarkers were highlighted as significant at p< 0.05 between the AKI and non-AKI groups using a Mann-Whitney U test. The predictive ability of individual biomarkers, identified as significant by Mann-Whitney U test, was investigated by ROC analyses.

Results for plasma, serum and urinary biomarkers are shown in Tables 5, 6 and 7, respectively.

*Table 5: Plasma biomarkers identified as showing a significant difference between AKI and non-AKI group.*

**Table 6: Serum biomarkers identified as showing a statistically significant difference between AKI and non-AKI groups.**

| **Marker** | **Pre/Post Surgery** | **Sig.** | **AUC** |
|---|---|---|---|
| IL12P40 | Pre and Post-surgery | 0.004, 0.021 | 0.637, 0.619 |
| IL1RA | Post-surgery | 0.003 | 0.653 |
| TNFRI | Pre and Post-surgery | <0.001 | 0.729, 0.795 |
| TNFRII | Pre and Post-surgery | 0.004, <0.001 | 0.634, 0.734 |
| IL2Ra | Post-surgery | 0.040 | 0.606 |
| H-FABP | Pre and Post-surgery | <0.001 | 0.712, 0.829 |
| Midkine | Pre and Post-surgery | 0.035, 0.001 | 0.615, 0.678 |

| **Marker** | **Pre/Post Surgery** | **Sig.** | **AUC** |
|---|---|---|---|
| IL-6 | Post-surgery | 0.001 | 0.661 |
| TNFα | Pre and Post-surgery | 0.02, 0.002 | 0.607, 0.653 |
| MCP | Pre and Post-surgery | 0.035, 0.003 | 0.597, 0.646 |
| IL-5 | Pre-surgery | 0.039 | 0.594 |
| IL-15 | Post-surgery | 0.001 | 0.523, 0.663 |
| MIP1a | Pre and Post-surgery | 0.009, 0.003 | 0.619, 0.644 |
| NGAL | Pre and Post-surgery | <0.001, <0.001 | 0.752, 0.812 |

**Table 7: Urinary biomarkers identified as significant between AKI and non-AKI groups.**

| **Marker** | **Pre/Post Surgery** | **Sig.** | **AUC** |
|---|---|---|---|
| TNFRI | Pre and Post-Surgery | 0.004, 0.001 | 0.638, 0.656 |
| NGAL | Post-surgery | 0.008 | 0.631 |

Biomarker combinations were then considered which providing the greatest ability to predict AKI from non-AKI subject groups.

Further, analysis of biomarkers was undertaken wherein ratios between biomarkers were considered. Ratios of urinary anti- and pro-inflammatory mediators were determined. Further ratios of anti- and pro-inflammatory mediators in the blood (in particular the serum) were considered. Additionally, ratios of anti- and pro-inflammatory mediators in the urine and blood were considered.

Based on this work it was considered that the ratio or balance of anti- and pro-inflammatory mediators in blood and urine will be lower in those subjects that develop renal dysfunction post operatively that those subjects retaining normal post-operative renal function.

Without wishing to be bound by theory, it is considered that in blood and urine of those subjects who develop renal dysfunction (AKI) there would either be no correlation or a negative correlation between anti- and pro-inflammatory mediators. Further it is considered that hypoperfusion in combination with a further insult of an imbalanced inflammatory response can be more injurious to the kidney than either insult occurring alone. Since hypoperfusion is an important contributing factor to perioperative AKI, suitably markers such as HFABP and VEGF can be measured and used to assess AKI risk.

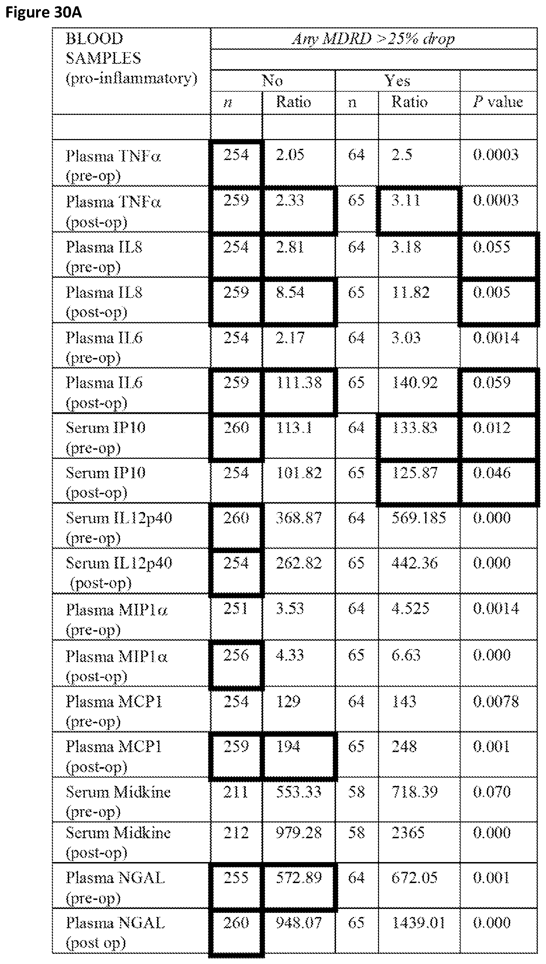

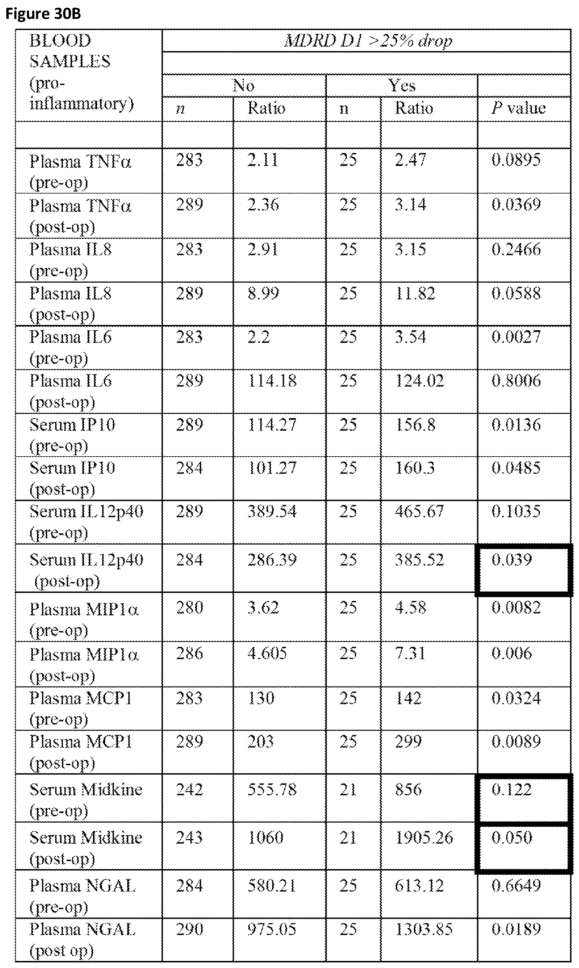

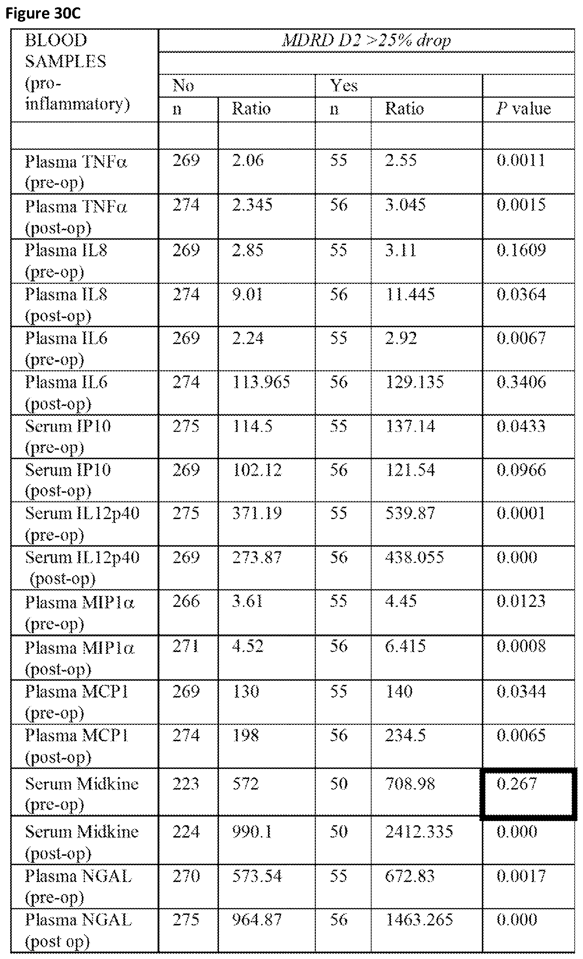

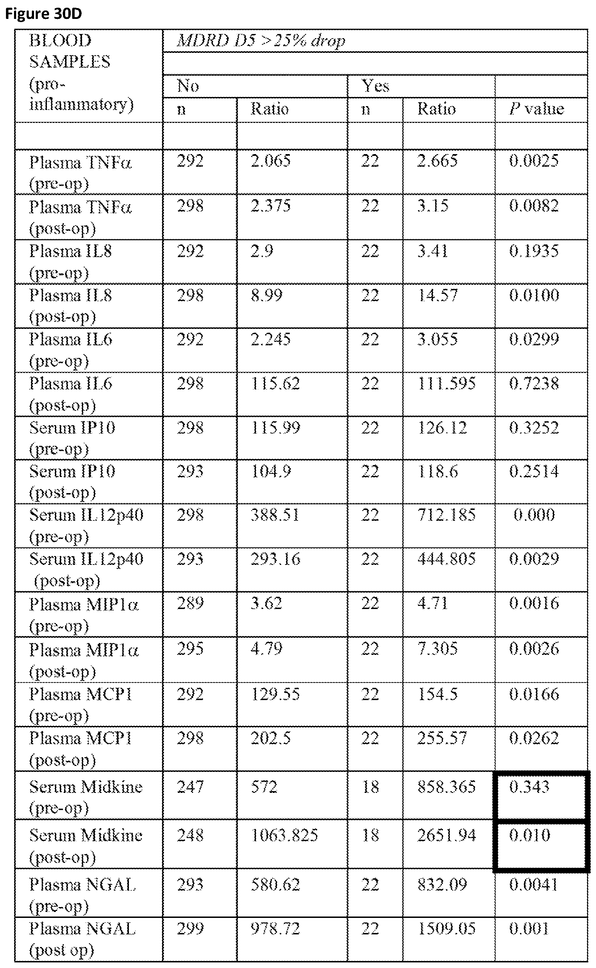

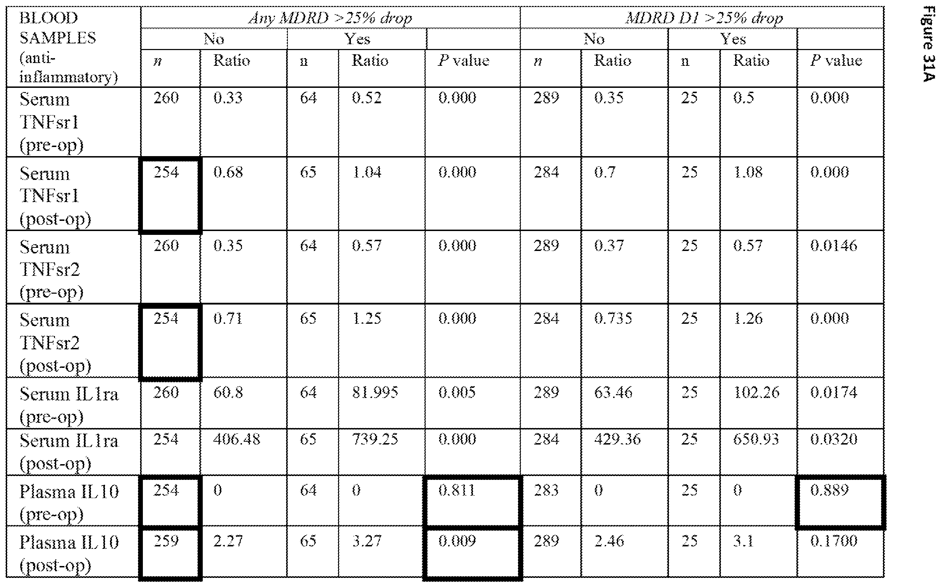

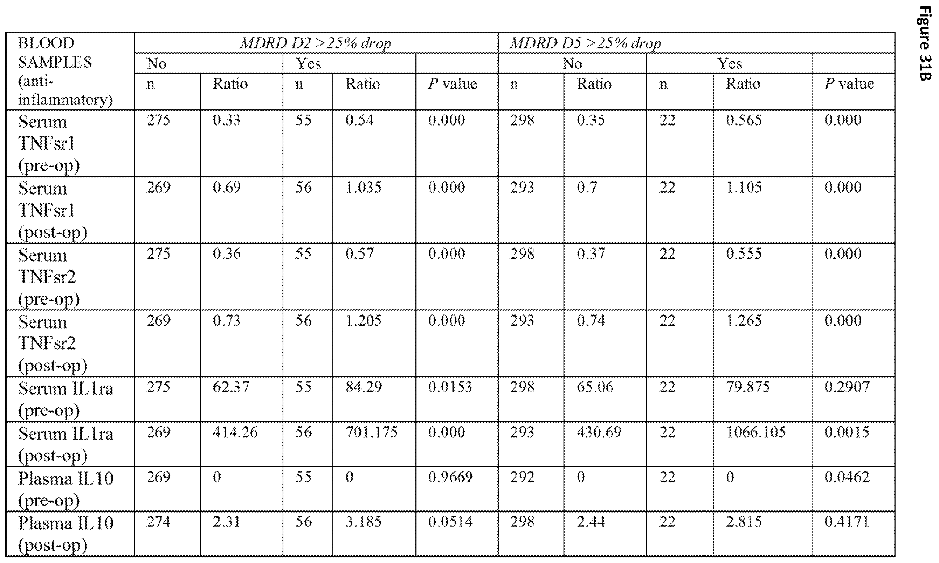

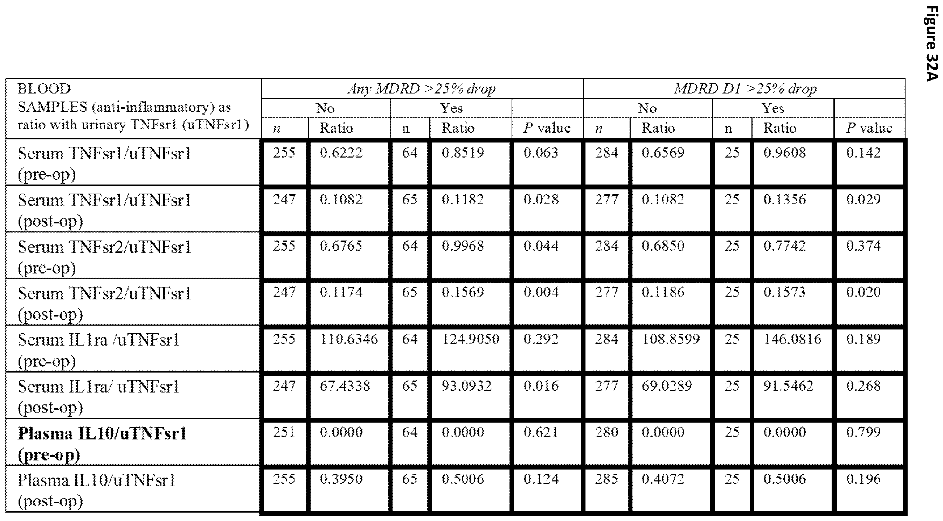

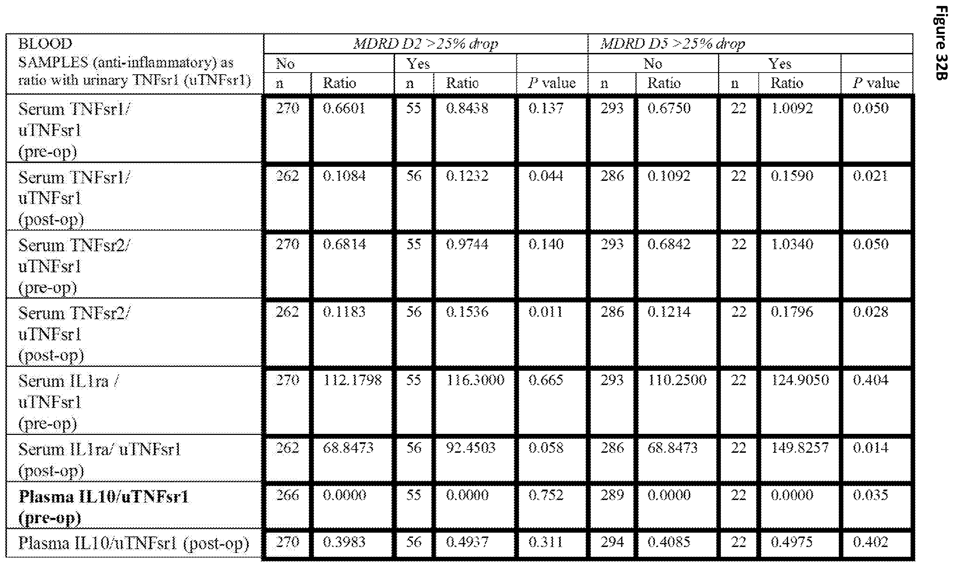

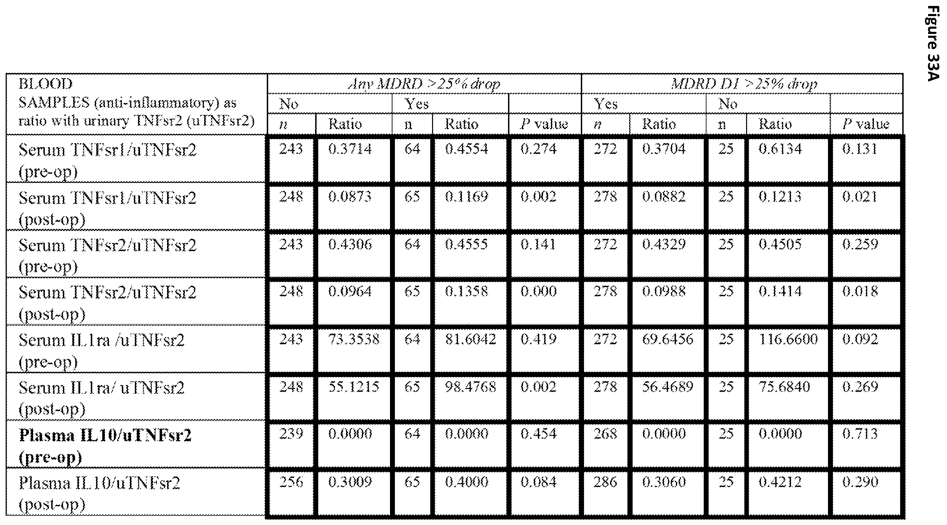

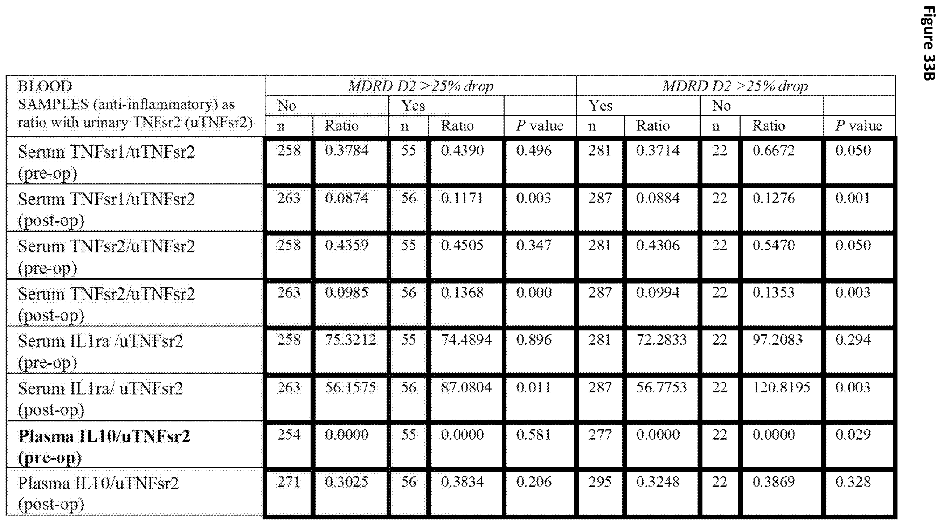

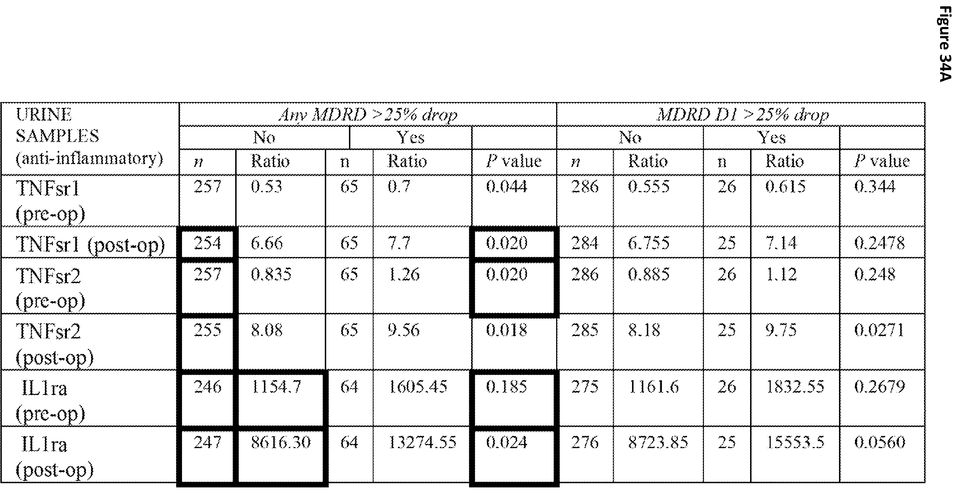

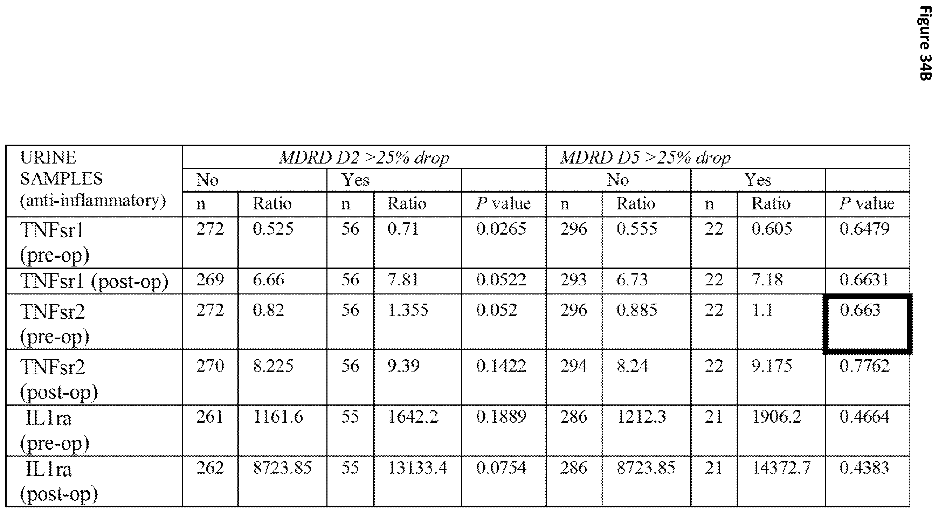

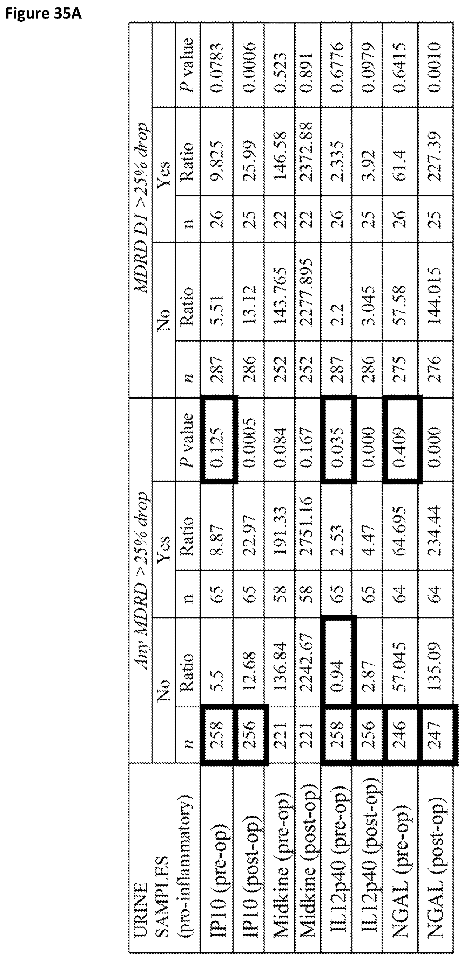

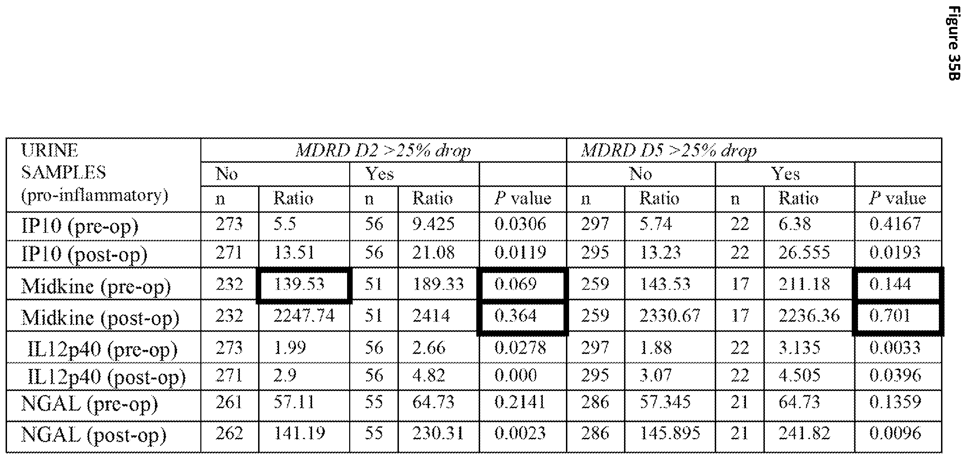

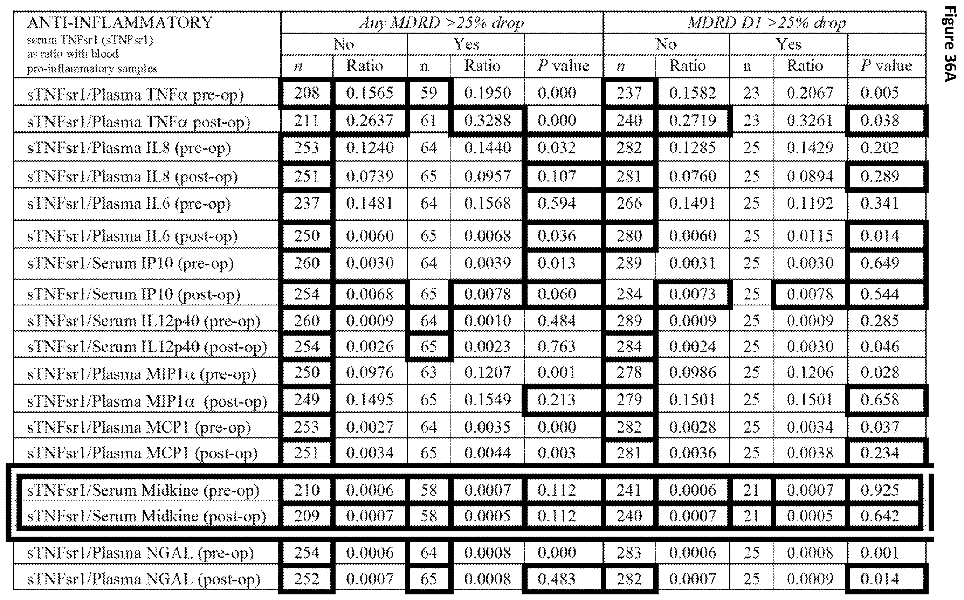

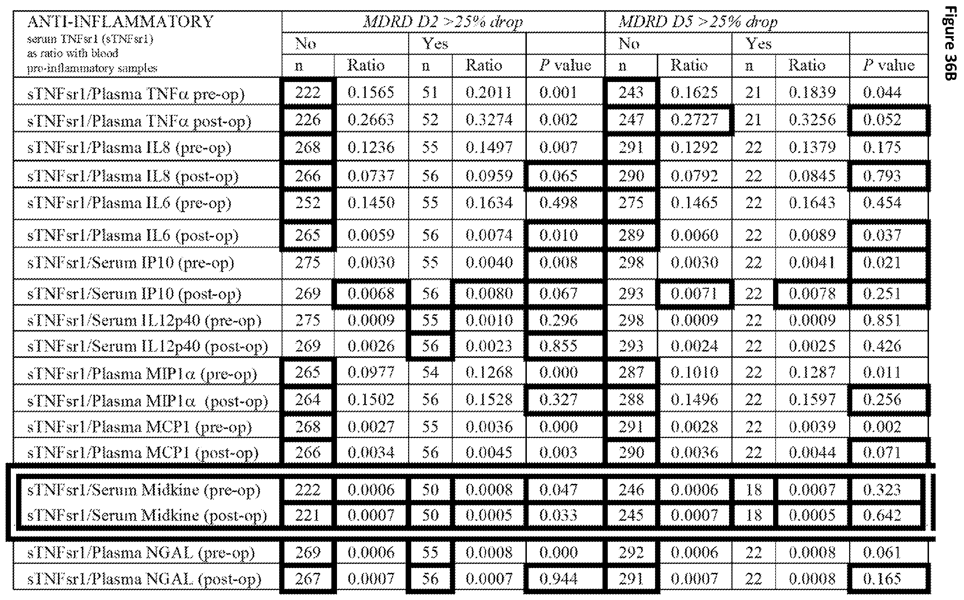

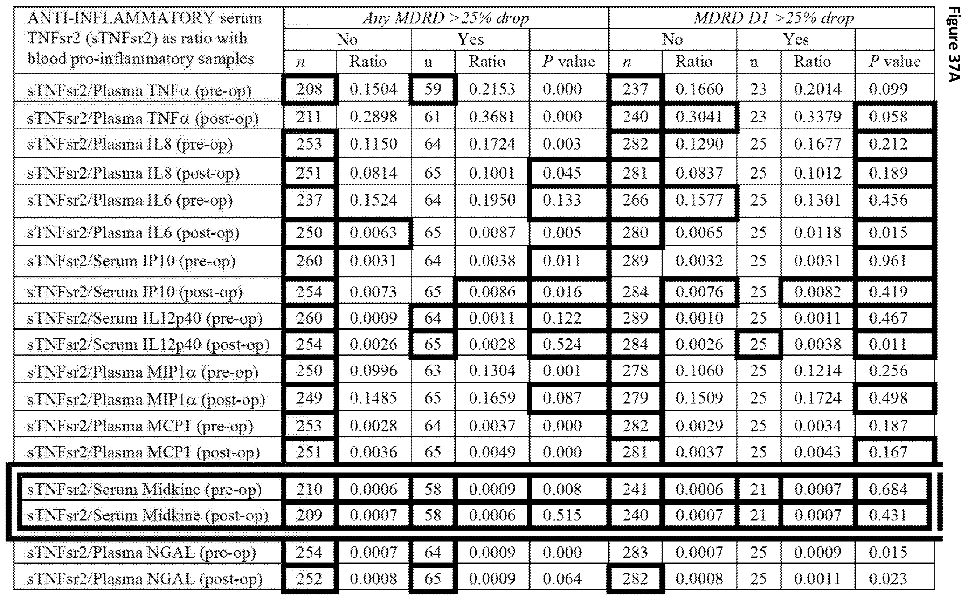

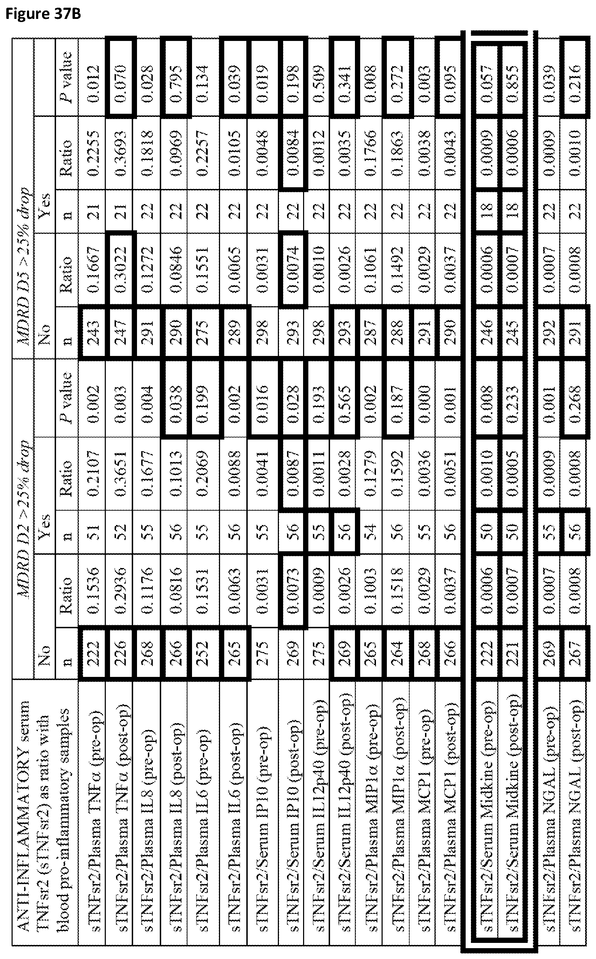

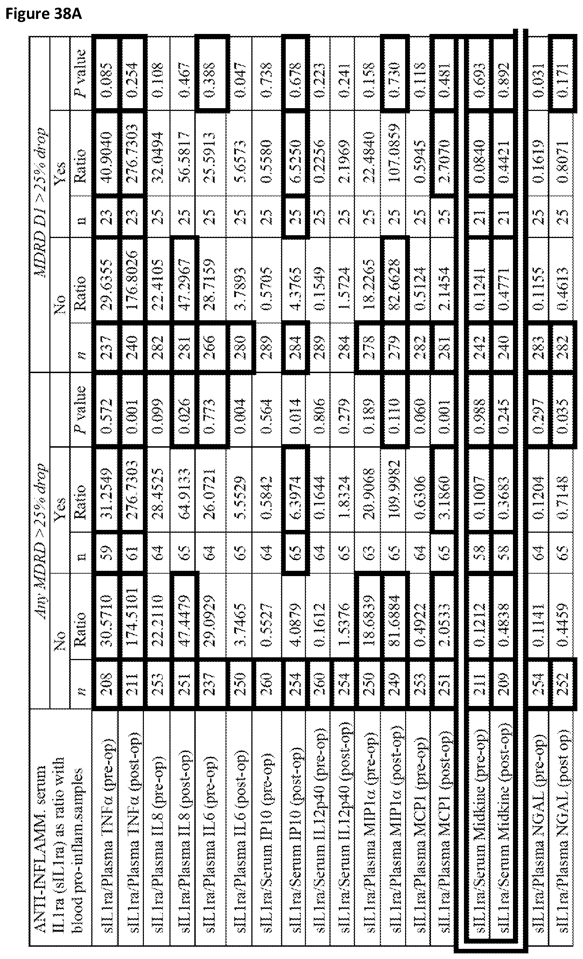

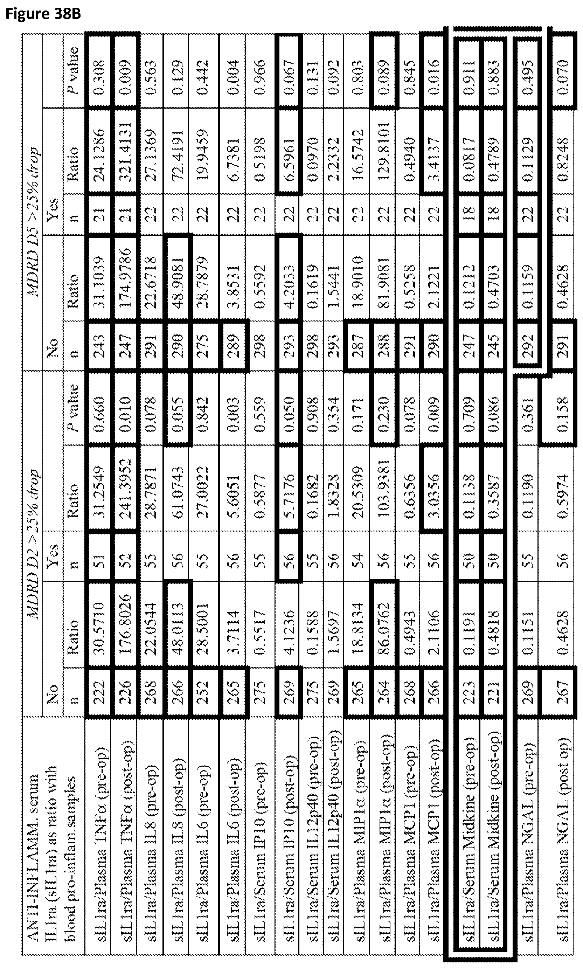

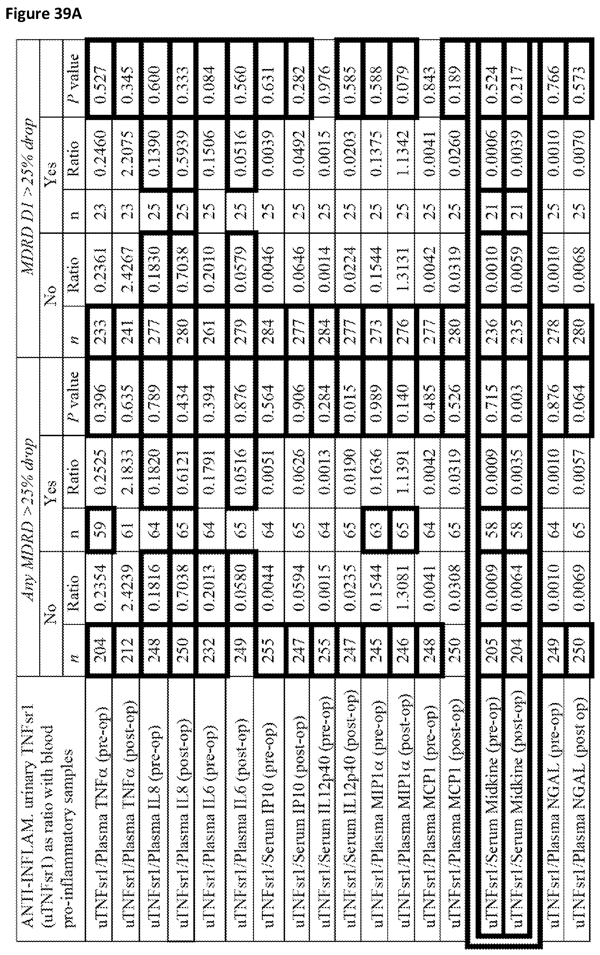

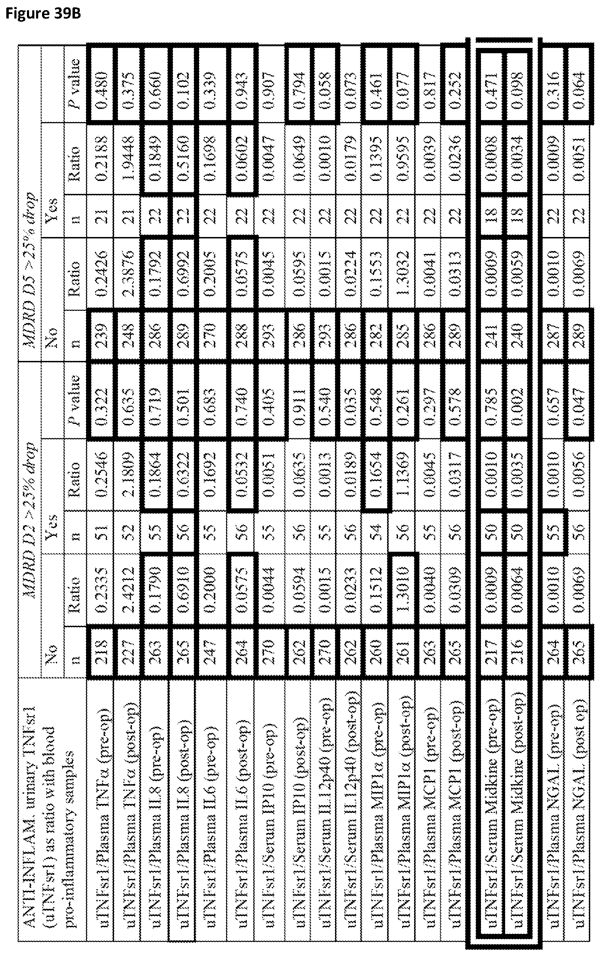

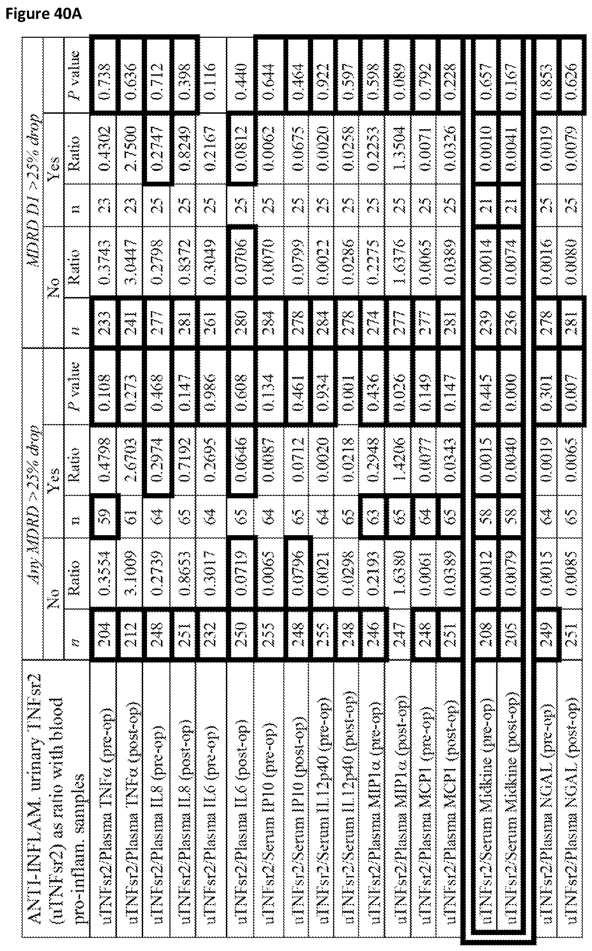

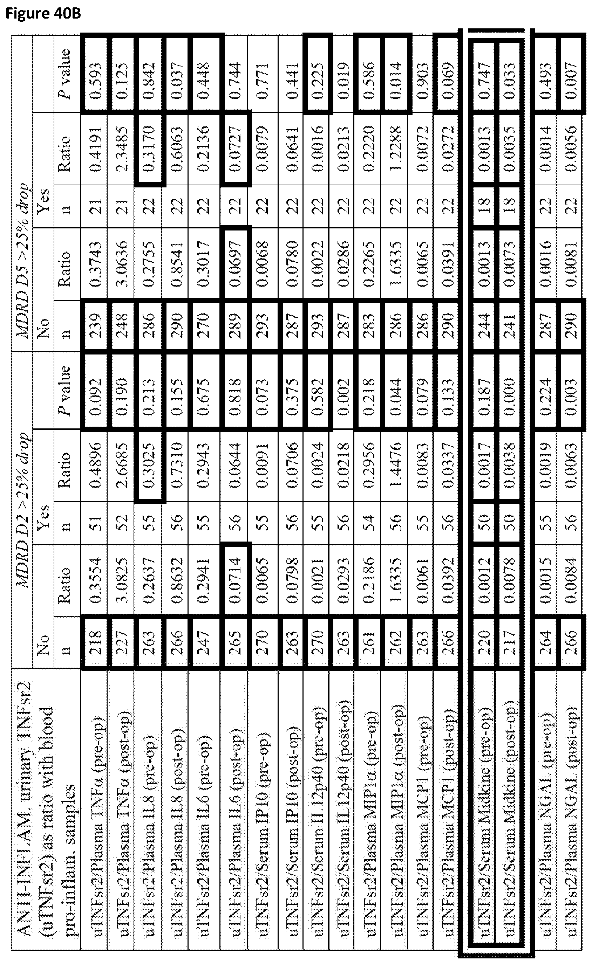

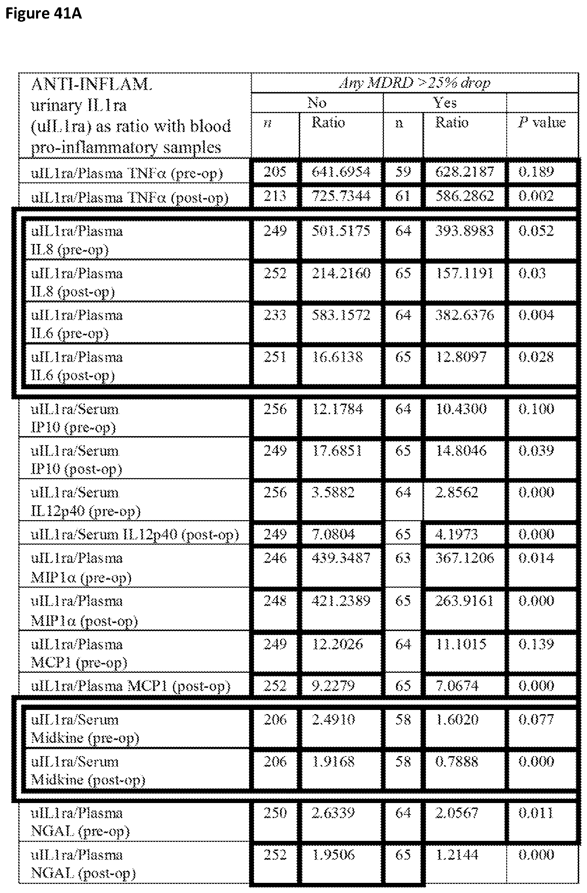

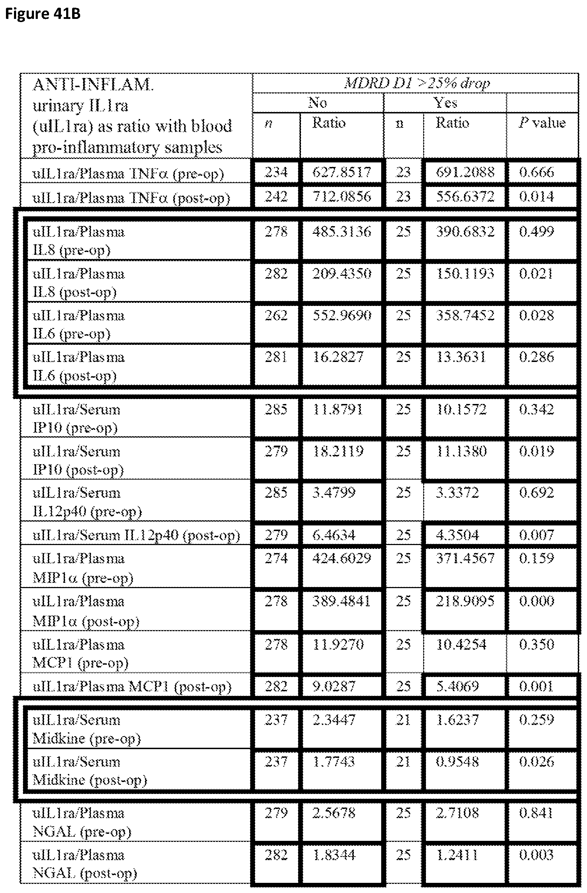

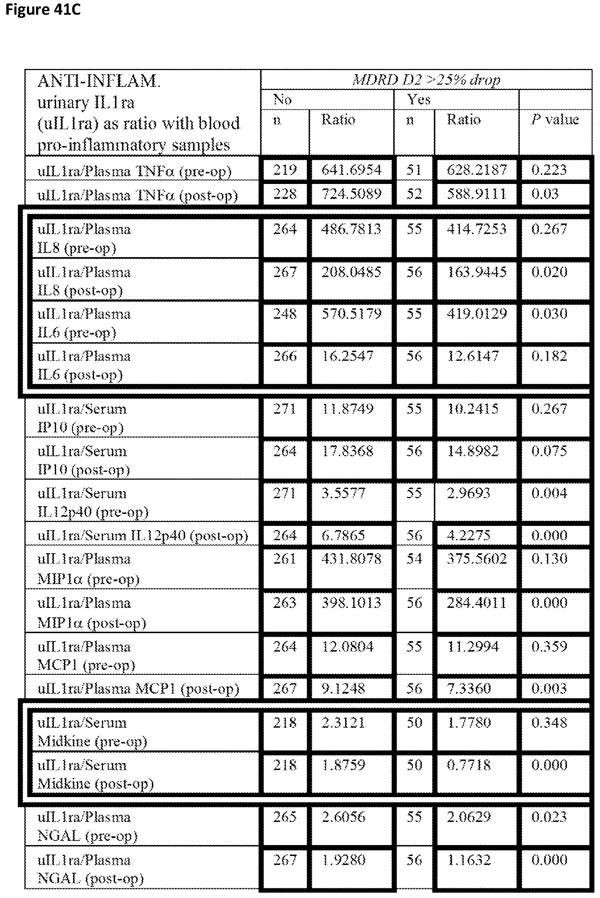

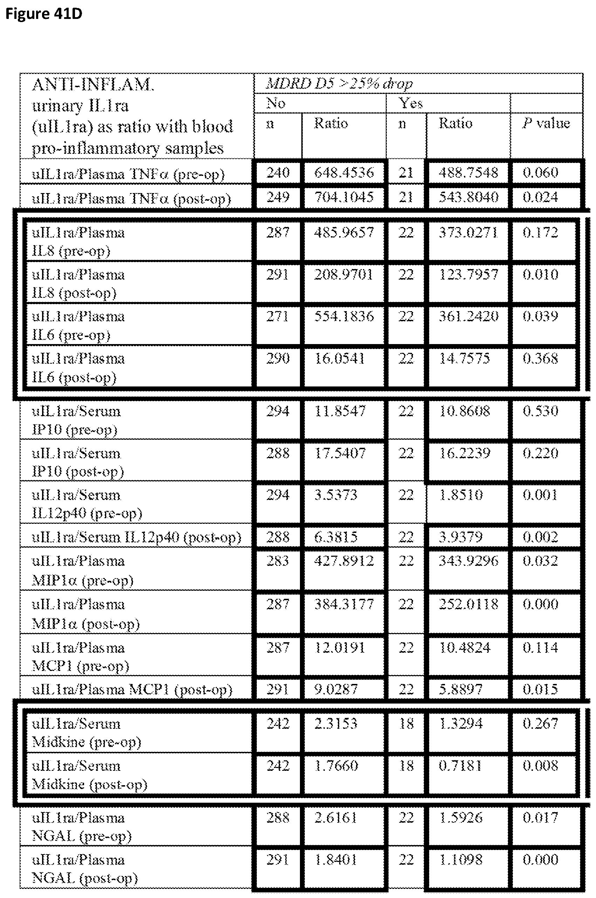

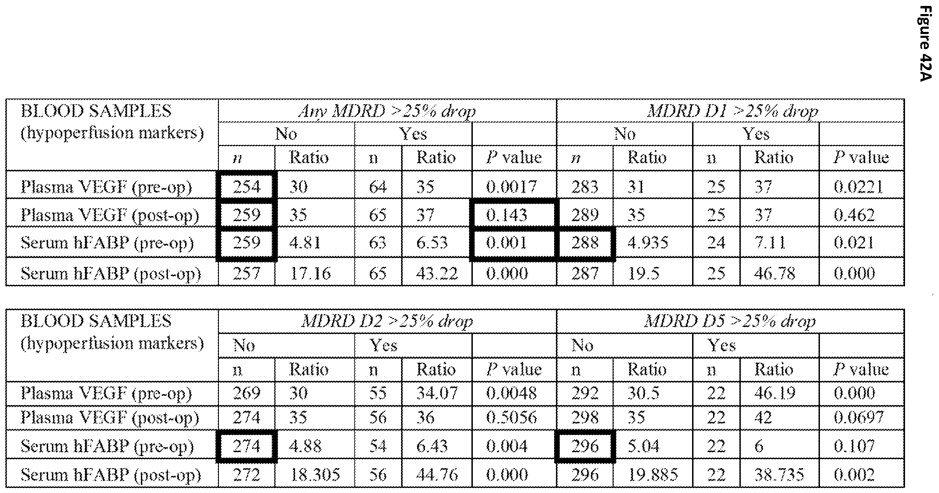

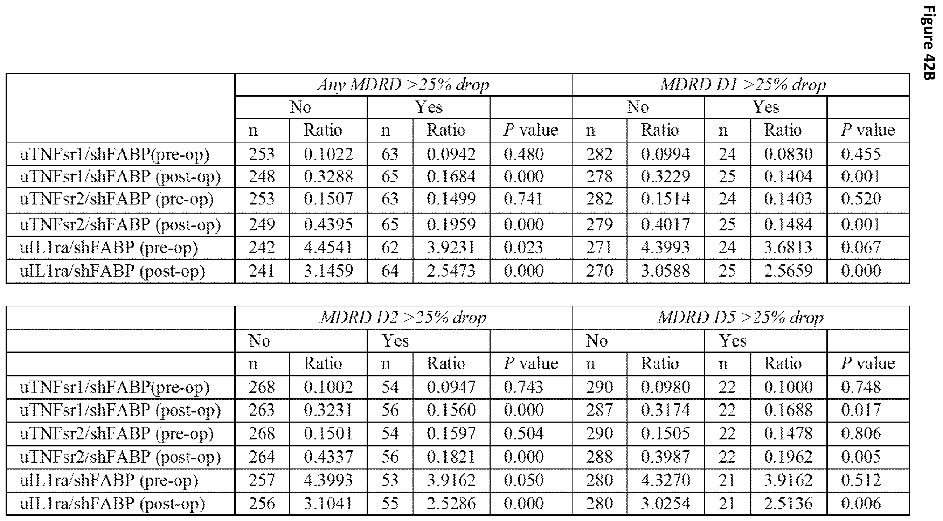

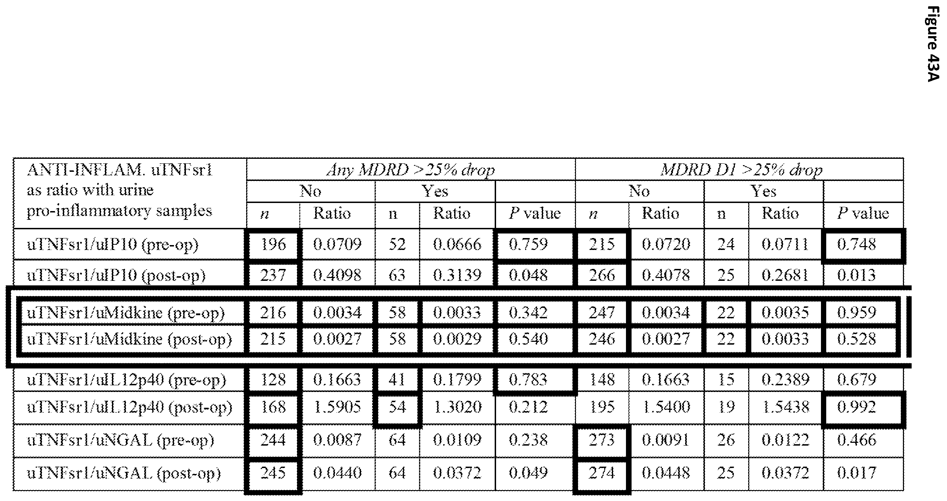

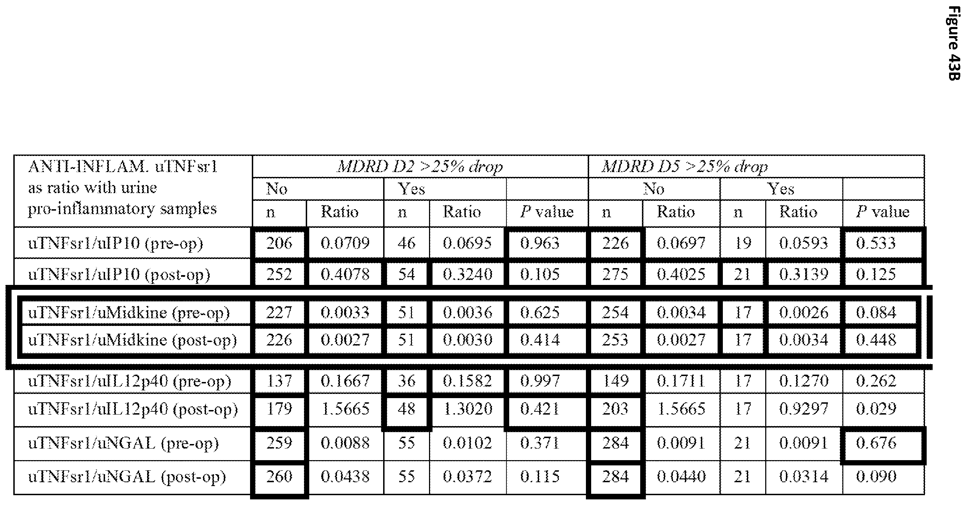

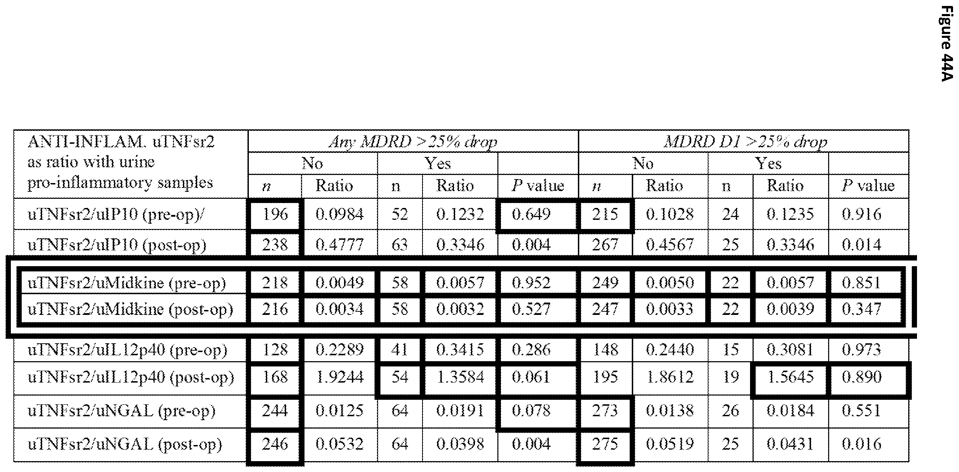

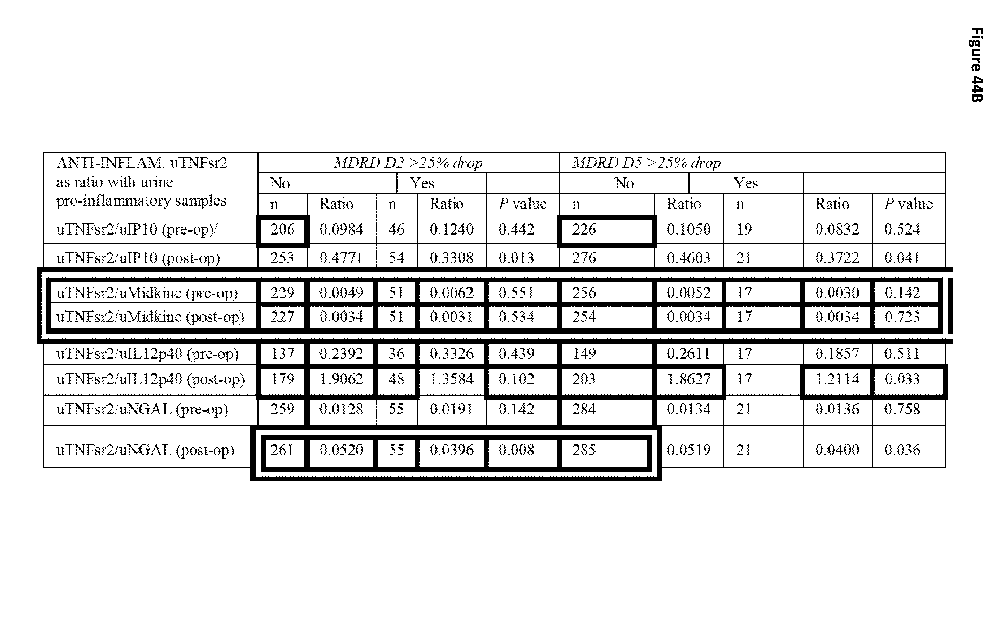

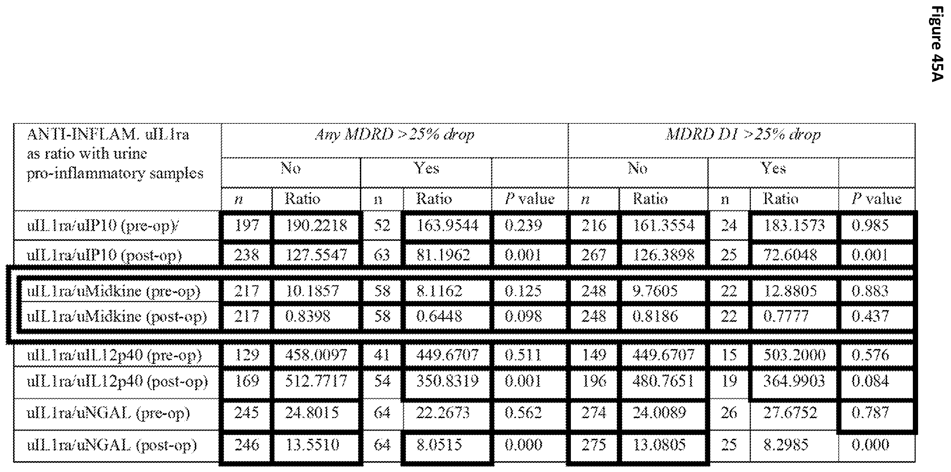

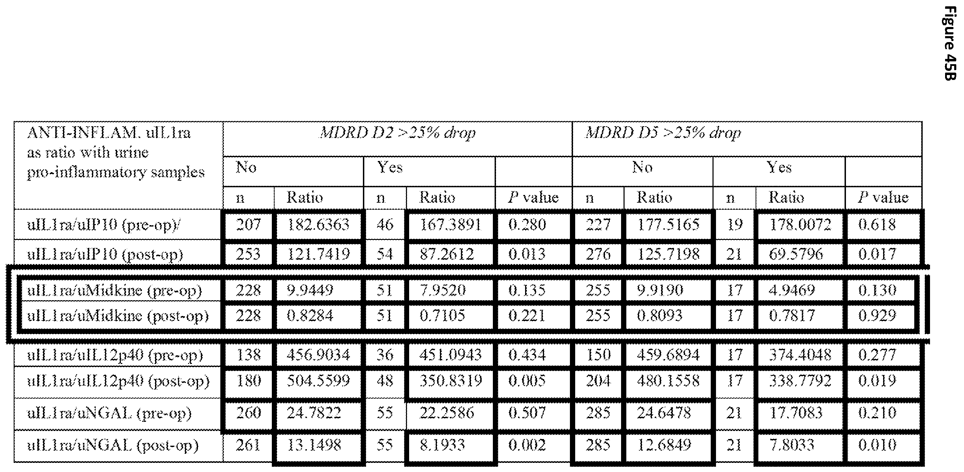

Based on the results shown in Figures 30 to 45 (wherein no shading indicates no differences in the Number of subjects (n) between SPSS and PRISM results / no differences or small differences in Ratios and *p*-values between SPSS and PRISM results and shading indicates differences in the Number of subjects (n) between SPSS and PRISM results /Greater differences in Ratios and p-values between SPSS and PRISM results, particular ratios were found to be predictive.

It was determined that the ratio of postoperative urinary TNFsr2/serum HFABP fell in AKI patients, suggesting that organ hypoperfusion as indicated by increased serum HFABP was particularly injurious to those patients who did not develop an adequate anti-inflammatory urinary TNFsr2 response. Without wishing to be bound by theory it is considered that an inadequate protective urinary anti-inflammatory response becomes significant in those patients who endure the double insults of proinflammation (as evidenced by urinary proinflammatory mediators) as well as hypoperfusion (as evidenced by increased serum HFABP). It is further considered that inadequate protective urinary anti-inflammatory response becomes significant in those patients who endure the double insults of proinflammation (as evidenced by blood and urinary proinflammatory mediators)as well as hypoperfusion as evidenced by increased serum HFABP and VEGF.

Preoperative VEGF has been determined to be higher in the AKI group for all endpoints including D5 renal dysfunction - (P < 0.001). VEGF is considered to be a direct hypoperfusion/hypoxia marker.

Without wishing to be bound by theory, it is considered that post operatively, after cardiac surgery, when the heart is fixed, overall perfusion and oxygenation is typically improved. HFABP post op does not typically indicate overall oxygenation and perfusion post op. Post op HFABP may be considered to be more indicative of the magnitude of the intraoperative insult and in turn be predictive of AKI.

Post op hypoperfusion/hypoxia is typically less of a problem in the majority of straightforward low risk cases because following cardiac surgery the heart is revascularised and the subject is typically mechanically ventilated with oxygen supplementation giving them a supra physiological pO2. VEGF as a marker of hypoperfusion and hypoxia at the moment of measurement, may those be indicative of artificially supplemented oxygenation. However, a rise in VEGF post op may be particularly significant.

Pre op VEGF thus is considered predictive of vulnerability to perioperative hypoperfusion on CPB with heightened AKI risk stretching as far as D5.

In fracture subjects it was determined that peri-operative increases were observed in almost all blood pro- and anti-inflammatory mediators measured, with some pro-inflammatory mediators (pre and post op) and some anti-inflammatory mediators showing significantly greater increases in the renal dysfunction group compared with the normal renal function subjects.

It is considered results determined in relation to cardiac surgery with respect to pre and post operative blood and urinary cytokines and anti/pro inflammatory cytokine ratios are similarly observed in post trauma orthopaedic surgery patients with the difference that base line post trauma pre operative samples will have undergone some increase in response to the fracture trauma.

For fracture subjects, it is considered the ratio of
urinary TNFsr1/HFABP (serum)
TNFsr1 (UA)/HFABP (SA) (Do AKI) (was lower in AKI)
TNFsr1 (UB)/HFABP (SB) (D0 ,D1, D5) (was lower in AKI)
urinary TNFsr2/HFABP(serum)
TNFsr2(UA)/HFABP (SA) (Do AKI) (was lower in AKI)
TNFsr2 (UB)/HFABP(SB) (D0, D1,D2,D5) (was lower in AKI)

UA refers to 'pre' urine samples and UB refers to 'post' urine samples. The same applies for serum.

For the fracture patients - D0 = before the operation (this can still be 3-4 days after the initial trauma)
D1 = 24 hours after the operation
D5 = 5 days after the operation
   and
TNFsr2UB/TNFSR2 (SB) (D5AKI) (was lower in AKI)
are particularly significant.

This has also been considered in cardiac patients wherein the ratio of postoperative urinary TNFsr2/serum HFABP fell in AKI patients, suggesting that organ hypoperfusion as indicated by increased serum HFABP was particularly injurious to those patients who did not develop an adequate anti-inflammatory urinary TNFsr2 response.

Post-operative ratios of urinary anti-inflammatory mediators/blood pro-inflammatory mediators were generally lower in renal dysfunction patients than the normal renal patients.

Further it is considered increased TNFα in blood correlates weakly but significantly with TNFsr2 in urine in non-AKI patients. This may suggest that in AKI patients the post operative compensatory rise in urinary TNFsr2 is compromised: and there may be a tendency to this deficiency pre-operatively even when renal function is normal

**Table 8**

| **Plasma TNFα vs urinary TNFsr2 - cardiac** | | | | | |
|---|---|---|---|---|---|
| **TNFSR2 UB/HFABP SB** | | | | | |
| | **AKI** | **AKI** | | **Normal** | **Normal** |
| | N | Median | N | Median | P Value |
| D1 | 25 | 0.1484 | 286 | 0.3978 | 0.0016 |
| D2 | 56 | 0.1821 | 271 | 0.4061 | <0.001 |
| D5 | 22 | 0.1962 | 295 | 0.3949 | 0.0102 |
| Any time | 65 | 0.1959 | 247 | 0.4395 | <0.0001 |

| **Timepoint** | | **P-Value** | **r-Value** | **n (XY pairs)** |
|---|---|---|---|---|
| **A** | **AKI Anytime 75%** | 0.6016 | 0.0665 | 64 |
| | **Non-AKI** | 0.0045 | 0.1834 | 239 |
| **B** | **AKI Anytime 75%** | 0.6387 | 0.05933 | 65 |
| | **Non-AKI** | <0.0001 | 0.2553 | 242 |

### Correlations between plasma TNFα and urinary TNFsr2

**Table 10**

| | **TNFSR2 UA/HFABP SA** | | | | |
|---|---|---|---|---|---|
| | **AKI** | | | **Non AKI** | |
| | N | Mean | N | Mean | P Value |
| Any Time AKI | 63 | 0.1441 | 250 | 0.1384 | 0.5313 |
| Day 1 AKI | 24 | 0.1403 | 288 | 0.1482 | 0.6467 |
| Day 2 AKI | 54 | 0.1597 | 274 | 0.1395 | 0.3645 |
| Day 5 AKI | 22 | 0.1478 | 296 | 0.1442 | 0.9358 |

**Table 11**

| TNFSR2 UB / HFABP SB | | | | | |
|---|---|---|---|---|---|
| | SB AKI N | AKI Median | Normal N | Normal Median | P Value |
| D1 | 25 | 0.1484 | 286 | 0.3978 | 0.0016 |
| D2 | 56 | 0.1821 | 271 | 0.4061 | <0.001 |
| D5 | 22 | 0.1962 | 295 | 0.3949 | 0.0102 |
| Anytime | 65 | 0.1959 | 247 | 0.4395 | <0.0001 |

### Table 12

In the cardiac study it was determined that there were peri-operative increases in almost all blood pro- and anti-inflammatory mediators measured, with both pro-inflammatory mediators (pre and post op TNFalpha, IP10,IL12p40, MIP1alpha, MCP1 and NGAL; post op IL8 and Midkine; pre op IL6) and anti-inflammatory mediators (pre and post op TNFsr1, TNFsr2 and IL1ra; post op IL10 ) showing significantly greater increases in the anytime renal dysfunction group compared with the normal renal function patients. This study shows that in comparison with those patients who retained normal renal function, those who developed renal dysfunction post operatively had a higher baseline preoperative blood concentration of a range of pro-inflammatory markers (eg TNFa, IP10, IL12p40, MIP1alpha, MCP1 , NGAL and IL6) as well as a greater postoperative proinflammatory response in blood (eg TNFa, IP10, IL12p40, MIP1alpha, MCP1 , NGAL, IL8, and Midkine).

Determination of ratios of blood anti-inflammatory mediators/blood pro-inflammatory mediators consistently showed higher anti-inflammatory/pro-inflammatory ratios in the renal dysfunction group(these differences being significant in pre and post op sTNFsr1/plasma TNF, sTNFsr1/plasmaMCP1, sTNFsr2/plasma TNFalpha, sTNFsr2/plasma IL8, sTNFsr2/sIP10, sTNFsr2/plasmaMCP1; pre-op sTNFsr1/plasma IL8, sTNFsr1/sIP10, sTNFsr1/plasma MIP1alpha, sTNFsr1/plasma NGAL, sTNFsr2/plasma MIP1alpha, sTNFsr2/sMidkine, sTNFsr2/plasmaNGAL; post op sTNFsr1/plasma IL6, sTNFsr2/plasma IL6, slL1ra/plasma TNFalpha, slL1ra/plasma IL8, slL1ra/plasma IL6, sIL1ra/sIP10, slL1ra/plasma MCP1, sIL1ra/plasma NGAL).

In cardiac and fracture studies, in contrast to blood, in the urine, the anti-/pro-inflammatory ratios were consistently lower in the renal dysfunction patients than the normal renal function patients. In the cardiac study, these differences were significant with respect to any time renal dysfunction in the following ratios: post-operative uTNFsr1/uIP10; post-op uTNFsr1/uNGAL; post-operative uTNFsr2/uIP10; post-operative uTNFsr2/uNGAL; post-operative uIL1ra/uIP10; post-operative ulL1ra/ulL12p40; post-operative uIL1ra/uNGAL. These differences were also significant with respect to D5 renal dysfunction patients in the following ratios: post-operative uTNFsr2/uIL12p40; post-operative uIL1ra/uIP10; post-operative uIL1ra/uIL12p40; post-operative uIL1ra/uNGAL.

Post-operative ratios of urinary anti-inflammatory mediators/blood pro-inflammatory mediators were generally lower in the renal dysfunction patients than the normal renal function patients. Differences considered to be significant with respect to any time renal dysfunction were the following post-operative ratios: uTNFsr1/sIL12p40, uTNFsr1/sMidkine, uTNFsr2/sIL12p40, uTNFsr2/pMIP1alpha, uTNFsr2/sMidkine, uTNFsr2/pNGAL, ulL1ra/pTNFalpha, uIL1ra/pIL8, ulL1ra/plL6, uIL1ra/sIP10, ulL1ra/sIL12p40, ulL1ra/pMIP1alpha, ulL1ra/pMCP1, ulL1ra/sMidkine, uIL1ra/pNGAL.

Although no pre-operative uTNFSR1/blood pro-inflammatory or uTNFsr2/ blood pro-inflammatory ratios were different between normal renal function and renal dysfunction groups, some pre-operative ulL1ra/blood pro-inflammatory ratios were also significantly lower in those who later developed renal dysfunction namely preoperative ulL1ra/plL6, uIL1ra/sIL12p40, ulL1ra/pMIP1alpha, ulL1ra/pNGAL. Of particular interest to clinicians, these differences were also significant with respect to D5 renal dysfunction patients in the following post operative ratios: post op uTNFsr2/plL8, uTNFsr2/slL12p40, uTNFsr2/pMIP1alpha, uTNFsr2/pNGAL, ulL1ra/pTNFalpha, ulL1ra/pIL8, ulL1ra/sIL12p40, uIL1ra/pMIP1alpha, uIL1ra/pMCP1, ulL1ra/sMidkine, ulL1ra/pNGAL and of note with respect to the pre-operative ratio of pre-operative ulL1ra/pIL6, ulL1ra/sIL12p40, ulL1ra/pMIP1alpha, uIL1ra/pNGAL.

If urinary anti-inflammatory / blood pro-inflammatory ratios (in contrast to blood anti-inflammatory/blood pro-inflammatory ratios), are lower in the renal dysfunction groups than in the normal renal function subjects, it may be inferred that filtered blood pro-inflammatory mediators are less well counterbalanced by a compensatory intrarenal anti-inflammatory response in the renal dysfunction patients than the normal renal function patients. This is further corroborated by the demonstration of negative correlations between blood pro- and urinary anti-inflammatory mediators in the renal dysfunction groups.

### Ratio of Urinary TNFsr2/serum HFABP

Finally, in cardiac patients, the ratio of postoperative urinary TNFsr2/serum HFABP fell in AKI patients, suggesting that organ hypoperfusion as indicated by increased serum HFABP was particularly injurious to those patients who did not develop an adequate anti-inflammatory urinary TNFsr2 response.

Reference to cited material or information contained in the text should not be understood as a concession that the material or information was part of the common general knowledge or was known in any country.

Although the invention has been particularly shown and described with reference to particular examples, it will be understood by those skilled in the art that various changes in the form and details may be made therein without departing from the scope of the present invention, which is defined by the appended claims.

## Claims

1. A method to determine a predisposition of a subject to developing acute kidney injury (AKI), the method comprising the step of:
determining the level of at least one marker selected from Midkine (MK) or heart-type Fatty Acid Binding Protein (H-FABP) present in a blood or urine sample taken from the subject within 48 hours following cardiac surgery or a fracture trauma;
wherein when the level of the Midkine or H-FABP is higher than a normal level of Midkine or H-FABP in a blood or urine sample from a control it is indicative the subject has a greater than normal predisposition for developing AKI following cardiac surgery or a fracture trauma;
and further comprising, determining the level of at least a second marker selected from at least one of Soluble Tumour Necrosis Factor Receptor I (TNFRI) and Soluble Tumour Necrosis Factor Receptor II (TNFRII) wherein when the detected level of TNFRI and/ or TNFRII is higher than a normal level of TNFRI and/or TNFRII respectively in a control it is indicative the subject has a greater than normal predisposition for developing AKI following cardiac surgery or fracture trauma; and,
further wherein the determining step comprises determining the level of at least three markers selected from: TNFRI, H-FABP and Midkine; or TNFRI, TNFRII and Midkine.

2. A method to determine a predisposition of a subject to developing AKI, the method comprising the step of:
determining the level of at least one marker selected from H-FABP or Midkine (MK) present in a blood or urine sample taken from the subject prior to cardiac surgery or a fracture trauma;
wherein when the level of the H-FABP or Midkine marker is higher than a normal level of H-FABP or Midkine in a blood or urine sample from a control it is indicative the subject has a greater than normal predisposition for developing AKI following cardiac surgery or a fracture trauma;
and further comprises, determining the level of at least a second marker selected from at least one of TNFRI And TNFRII wherein when the detected level of TNFRI and/ or TNFRII is higher than a normal level of TNFRI and/or TNFRII respectively in a control it is indicative the subject has a greater than normal predisposition for developing AKI following cardiac surgery or fracture trauma; and,
further wherein the determining step comprises determining the level of at least three markers selected from: TNFRI, H-FABP and Midkine; or TNFRI, TNFRII and Midkine.

3. The method of claim 1 or 2 wherein the step of determining the level of at least one marker selected from H-FABP or Midkine is undertaken on serum from the subject.

4. The method of any preceding claims wherein the method further comprises detecting at least one marker selected from IL-1a, IL-5, IL-6, IL-8, IL-10, IL-15, MIP1-alpha, VEGF, INF-gamma, TNF-alpha, MCP, NGAL, IL12P40, IP10 or IL1Ra.

5. The method of claim 4 wherein at least one of IL-1a, IL-5, IL-6, IL-8, IL-10, IL-15, MIP1-alpha, VEGF, INF-gamma, TNF-alpha, MCP and NGAL is detected from plasma from the subject.

6. The method of claim 4 wherein IL12P40 is detected from serum or urine of a subject.

7. The method of claim 4 wherein IP10 or IL1Ra are detected in urine.

8. The method of any of claims 2 to 7 wherein the sample is obtained from a subject within 24 hours of a proposed surgery.

9. The method of any of claims 1 and 3 to 7 wherein the sample is obtained from a subject within 24 hours post surgery.

10. The method of any preceding claims wherein the sample is plasma or serum or urine.

11. The method of any of claims 1, 3 to 7 and 9 to 10 wherein a ratio of urinary TNFRI to serum H-FABP post-surgery is determined, and wherein the ratio is lower in AKI subjects than non AKI subjects.

12. The method of any of claims 1, 3 to 7 and 9 to 10 wherein the ratio of urinary TNFRII to serum H-FABP post-surgery is determined, and wherein the ratio is lower in AKI subjects than non AKI subjects.

13. The method of any of claims 1, 3 to 7 and 9 to 10 wherein the method comprises determining AKI risk on post-operative ratios selected from urinary TNFRI to seum Midkine, urinary TNFRII to serum Midkine or urinary IL1ra to serum Midkine wherein the urinary anti-inflammatory / blood pro-inflammatory ratios are lower in those who develop renal dysfunction.

14. The method of any of claims 2, 3 to 8 and 10 wherein the method comprises determining pre-operative urinary TNFRI / blood pro-inflammatory or urinary TNFRII / blood pro-inflammatory ratios wherein urinary anti-inflammatory /blood pro-inflammatory ratios were lower in those who later developed renal dysfunction.

## Patentansprüche

1. Ein Verfahren zur Bestimmung einer Prädisposition eines Individuums, eine akute Nierenverletzung (AKI) zu entwickeln, wobei das Verfahren den folgenden Schritt beinhaltet:
Bestimmen des Spiegels von mindestens einem Marker, ausgewählt aus Midkin (MK) oder einem fettsäurebindenden Protein des Herztyps (H-FABP), der in einer Blut- oder Urinprobe vorliegt, die dem Individuum innerhalb von 48 Stunden nach einer Herzoperation oder einem Frakturtrauma entnommen wurde;
wobei, wenn der Spiegel von Midkin oder H-FABP höher als ein normaler Spiegel von Midkin oder H-FABP in einer Blut- oder Urinprobe von einer Kontrolle ist, dies darauf hindeutet, dass das Individuum eine größere als normale Prädisposition hat, AKI nach einer Herzoperation oder einem Frakturtrauma zu entwickeln;
und ferner beinhaltend das Bestimmen des Spiegels von mindestens einem zweiten Marker, ausgewählt aus mindestens einem von löslichem Tumornekrosefaktor-Rezeptor I (TNFRI) und löslichem Tumornekrosefaktor-Rezeptor II (TNFRII), wobei, wenn der detektierte Spiegel von TNFRI und/oder TNFRII höher als ein normaler Spiegel von TNFRI bzw. TNFRII in einer Kontrolle ist, dies darauf hindeutet, dass das Individuum eine größere als normale Prädisposition hat, AKI nach einer Herzoperation oder einem Frakturtrauma zu entwickeln; und
ferner wobei der Bestimmungsschritt das Bestimmen des Spiegels von mindestens drei Markern beinhaltet, die aus Folgendem ausgewählt sind: TNFRI, H-FABP und Midkin;
oder TNFRI, TNFRII und Midkin.

2. Ein Verfahren zur Bestimmung einer Prädisposition eines Individuums, AKI zu entwickeln, wobei das Verfahren den folgenden Schritt beinhaltet:
Bestimmen des Spiegels von mindestens einem Marker, ausgewählt aus H-FABP oder Midkin (MK), der in einer Blut- oder Urinprobe vorliegt, die dem Individuum vor einer Herzoperation oder einem Frakturtrauma entnommen wurde;
wobei, wenn der Spiegel von dem H-FABP- oder Midkin-Marker höher als ein normaler Spiegel von H-FABP oder Midkin in einer Blut- oder Urinprobe von einer Kontrolle ist, dies darauf hindeutet, dass das Individuum eine größere als normale Prädisposition hat, AKI nach einer Herzoperation oder einem Frakturtrauma zu entwickeln;
und ferner beinhaltend das Bestimmen des Spiegels von mindestens einem zweiten Marker, ausgewählt aus mindestens einem von TNFRI und TNFRII, wobei, wenn der detektierte Spiegel von TNFRI und/oder TNFRII höher als ein normaler Spiegel von TNFRI bzw. TNFRII in einer Kontrolle ist, dies darauf hindeutet, dass das Individuum eine größere als normale Prädisposition hat, AKI nach einer Herzoperation oder einem Frakturtrauma zu entwickeln; und
ferner wobei der Bestimmungsschritt das Bestimmen des Spiegels von mindestens drei Markern beinhaltet, die aus Folgendem ausgewählt sind: TNFRI, H-FABP und Midkin;
oder TNFRI, TNFRII und Midkin.

3. Verfahren gemäß Anspruch 1 oder 2, wobei der Schritt des Bestimmens des Spiegels von mindestens einem Marker, ausgewählt aus H-FABP oder Midkin, an Serum von dem Individuum vorgenommen wird.

4. Verfahren gemäß einem der vorgehenden Ansprüche, wobei das Verfahren ferner das Detektieren von mindestens einem Marker, ausgewählt aus IL-1a, IL-5, IL-6, IL-8, IL-10, IL-15, MIP1-alpha, VEGF, INF-gamma, TNF-alpha, MCP, NGAL, IL12P40, IP10 oder IL1Ra, beinhaltet.

5. Verfahren gemäß Anspruch 4, wobei mindestens eines von IL-1a, IL-5, IL-6, IL-8, IL-10, IL-15, MIP1-alpha, VEGF, INF-gamma, TNF-alpha, MCP und NGAL aus Plasma von dem Individuum detektiert wird.

6. Verfahren gemäß Anspruch 4, wobei IL12P40 aus Serum oder Urin eines Individuums detektiert wird.

7. Verfahren gemäß Anspruch 4, wobei IP10 oder IL1Ra in Urin detektiert werden.

8. Verfahren gemäß einem der Ansprüche 2 bis 7, wobei die Probe von einem Individuum innerhalb von 24 Stunden von einer vorgeschlagenen Operation erhalten wird.

9. Verfahren gemäß einem der Ansprüche 1 und 3 bis 7, wobei die Probe von einem Individuum innerhalb von 24 Stunden nach einer Operation erhalten wird.

10. Verfahren gemäß einem der vorstehenden Ansprüche, wobei die Probe Plasma oder Serum oder Urin ist.

11. Verfahren gemäß einem der Ansprüche 1, 3 bis 7 und 9 bis 10, wobei ein Verhältnis von Urin-TNFRI zu Serum-H-FABP nach einer Operation bestimmt wird und wobei das Verhältnis bei AKI-Individuen niedriger ist als bei Nicht-AKI-Individuen.

12. Verfahren gemäß einem der Ansprüche 1, 3 bis 7 und 9 bis 10, wobei das Verhältnis von Urin-TNFRII zu Serum-H-FABP nach einer Operation bestimmt wird und wobei das Verhältnis bei AKI-Individuen niedriger ist als bei Nicht-AKI-Individuen.

13. Verfahren gemäß einem der Ansprüche 1, 3 bis 7 und 9 bis 10, wobei das Verfahren das Bestimmen des AKI-Risikos bei postoperativen Verhältnissen beinhaltet, ausgewählt aus Urin-TNFRI zu Serum-Midkin, Urin-TNFRII zu Serum-Midkin oder Urin-IL1ra zu Serum-Midkin, wobei die entzündungshemmenden/entzündungsfördernden Urin-/Blut-Verhältnisse bei jenen, die eine Nierenfunktionsstörung entwickeln, niedriger sind.

14. Verfahren gemäß einem der Ansprüche 2, 3 bis 8 und 10, wobei das Verfahren das Bestimmen von präoperativen Urin-TNFRI-/entzündungsfördernden Blut- oder Urin-TNFRII-/entzündungsfördernden Blut-Verhältnissen beinhaltet, wobei die entzündungshemmenden/entzündungsfördernden Urin-/Blut-Verhältnisse bei jenen, die später eine Nierenfunktionsstörung entwickelten, niedriger waren.

## Revendications

1. Un procédé pour déterminer une prédisposition d'un sujet à développer une insuffisance rénale aiguë (IRA), le procédé comprenant l'étape :
de détermination du taux d'au moins un marqueur sélectionné parmi Midkine (MK) ou une protéine de liaison aux acides gras de type cardiaque (H-FABP) présent dans un échantillon de sang ou d'urine prélevé chez le sujet dans les 48 heures suivant une chirurgie cardiaque ou un traumatisme de fracture ;
dans lequel lorsque le taux de la Midkine ou de la H-FABP est plus élevé qu'un taux normal de Midkine ou d'H-FABP dans un échantillon de sang ou d'urine provenant d'un témoin, il est indicatif du fait que le sujet a une prédisposition supérieure à la normale à développer une IRA à la suite d'une chirurgie cardiaque ou d'un traumatisme de fracture ;
et comprenant en outre, la détermination du taux d'au moins un deuxième marqueur sélectionné parmi au moins l'un du récepteur I du facteur de nécrose tumorale soluble (TNFRI) et du récepteur II du facteur de nécrose tumorale soluble (TNFRII), dans lequel lorsque le taux détecté de TNFRI et/ou de TNFRII est plus élevé qu'un taux normal de TNFRI et/ou de TNFRII respectivement dans un témoin, il est indicatif du fait que le sujet a une prédisposition supérieure à la normale à développer une IRA à la suite d'une chirurgie cardiaque ou d'un traumatisme de fracture ; et,
en outre dans lequel l'étape de détermination comprend la détermination du taux d'au moins trois marqueurs sélectionnés parmi : TNFRI, H-FABP et Midkine ; ou TNFRI, TNFRII et Midkine.

2. Un procédé pour déterminer une prédisposition d'un sujet à développer une IRA, le procédé comprenant l'étape :
de détermination du taux d'au moins un marqueur sélectionné parmi H-FABP ou Midkine (MK) présent dans un échantillon de sang ou d'urine prélevé chez le sujet avant une chirurgie cardiaque ou un traumatisme de fracture ;
dans lequel lorsque le taux du marqueur H-FABP ou Midkine est plus élevé qu'un taux normal de H-FABP ou de Midkine dans un échantillon de sang ou d'urine provenant d'un témoin, il est indicatif du fait que le sujet a une prédisposition supérieure à la normale à développer une IRA à la suite d'une chirurgie cardiaque ou d'un traumatisme de fracture ;
et comprenant en outre, la détermination du taux d'au moins un deuxième marqueur sélectionné parmi au moins l'un de TNFRI et TNFRII, dans lequel lorsque le taux détecté de TNFRI et/ou de TNFRII est plus élevé qu'un taux normal de TNFRI et/ou de TNFRII respectivement dans un témoin, il est indicatif du fait que le sujet a une prédisposition supérieure à la normale à développer une IRA à la suite d'une chirurgie cardiaque ou d'un traumatisme de fracture ; et,
en outre dans lequel l'étape de détermination comprend la détermination du taux d'au moins trois marqueurs sélectionnés parmi : TNFRI, H-FABP et Midkine ; ou TNFRI, TNFRII et Midkine.

3. Le procédé de la revendication 1 ou de la revendication 2 dans lequel l'étape de détermination du taux d'au moins un marqueur sélectionné parmi H-FABP ou Midkine est entreprise sur du sérum provenant du sujet.

4. Le procédé de n'importe quelles revendications précédentes, le procédé comprenant en outre la détection d'au moins un marqueur sélectionné parmi IL-1a, IL-5, IL-6, IL-8, IL-10, IL-15, MIP1-alpha, VEGF, INF-gamma, TNF-alpha, MCP, NGAL, IL12P40, IP10 ou IL1Ra.

5. Le procédé de la revendication 4 dans lequel au moins un élément parmi IL-1a, IL-5, IL-6, IL-8, IL-10, IL-15, MIP1-alpha, VEGF, INF-gamma, TNF-alpha, MCP et NGAL est détecté à partir de plasma provenant du sujet.

6. Le procédé de la revendication 4 dans lequel IL12P40 est détecté à partir de sérum ou d'urine d'un sujet.

7. Le procédé de la revendication 4 dans lequel IP10 ou IL1Ra sont détectés dans l'urine.

8. Le procédé de n'importe lesquelles des revendications 2 à 7 dans lequel l'échantillon est obtenu à partir d'un sujet dans les 24 heures par rapport à une chirurgie proposée.

9. Le procédé de n'importe lesquelles des revendications 1 et 3 à 7 dans lequel l'échantillon est obtenu à partir d'un sujet dans les 24 heures après une chirurgie.

10. Le procédé de n'importe quelles revendications précédentes dans lequel l'échantillon est du plasma ou du sérum ou de l'urine.

11. Le procédé de n'importe lesquelles des revendications 1, 3 à 7 et 9 à 10 dans lequel un rapport de TNFRI urinaire sur H-FABP sérique après une chirurgie est déterminé, et dans lequel le rapport est plus faible chez des sujets IRA que chez des sujets non IRA.

12. Le procédé de n'importe lesquelles des revendications 1, 3 à 7 et 9 à 10 dans lequel le rapport de TNFRII urinaire sur H-FABP sérique après une chirurgie est déterminé, et dans lequel le rapport est plus faible chez des sujets IRA que chez des sujets non IRA.

13. Le procédé de n'importe lesquelles des revendications 1, 3 à 7 et 9 à 10, le procédé comprenant la détermination d'un risque d'IRA sur des rapports post-opératoires sélectionnés parmi TNFRI urinaire sur Midkine sérique, TNFRII urinaire sur Midkine sérique ou IL1ra urinaire sur Midkine sérique, dans lequel les rapports anti-inflammatoires urinaires/pro-inflammatoires sanguins sont plus faibles chez les personnes qui développent une dysfonction rénale.

14. Le procédé de n'importe lesquelles des revendications 2, 3 à 8 et 10, le procédé comprenant la détermination de rapports pré-opératoires TNFRI urinaire/pro-inflammatoires sanguins ou TNFRII urinaire/pro-inflammatoires sanguins, dans lequel les rapports anti-inflammatoires urinaires/pro-inflammatoires sanguins étaient plus faibles chez les personnes qui développèrent plus tard une dysfonction rénale.
